# EUROPEAN PATENT APPLICATION

(11) **EP 1 739 081 A1**
(43) Date of publication of application: **03.01.2007**
(21) Application number: 05734702.3
(22) Date of filing: 20.04.2005
(51) Int. Cl.: C07D 239/42, A61K 31/421, A61K 31/426, A61K 31/505, A61K 31/506, A61K 31/517, A61K 31/53, A61P 1/04, A61P 1/16, A61P 1/18, A61P 3/06, A61P 3/10, A61P 5/16, A61P 7/06, A61P 9/00, A61P 9/10, A61P 11/00, A61P 11/06, A61P 13/08, A61P 13/12, A61P 17/06

(54) **HYDRAZINO-SUBSTITUTED HETEROCYCLIC NITRILE COMPOUNDS AND USE THEREOF**

(30) Priority: 21.04.2004 JP 2004125992; 27.01.2005 JP 2005019690
(71) Applicant: ONO PHARMACEUTICAL CO., LTD., Osaka-shi 541-8526 (JP)
(72) Inventor: OHMOTO, Kazuyuki, c/o Ono Pharmaceutical Co., Ltd., Mishima-gun, Osaka 618-8585 (JP); HATAYAMA, Akira, c/o Ono Pharmaceutical Co., Ltd., Mishima-gun, Osaka 618-8585 (JP); HISAICHI, Katsuya, Ono Pharmaceutical Co., Ltd., Mishima-gun, Osaka 618-8585 (JP); TANAKA, Makoto, c/o Ono Pharmaceutical Co., Ltd., Mishima-gun, Osaka 618-8585 (JP); YAMADA, Hiroyuki, c/o Ono Pharmaceutical Co., Ltd., Mishima-gun, Osaka 618-8585 (JP)
(74) Representative: Henkel, Feiler & Hänzel
(86) International application number: PCT/JP2005/007947
(87) International publication number: WO 2005/103012

(57) **Abstract**

A compound having an inhibitory activity against cysteine protease, represented by formula (I): 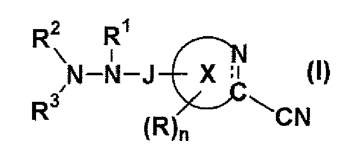 (wherein ring X is a heterocyclic group which may contain 1 to 4 hetero atoms selected from an nitrogen atom(s), a oxygen atom(s) and a sulfur atom(s) which may be oxidized, in addition to said nitrogen atom in the ring; J is a bond or a spacer having from 1 to 8 atoms; R, R¹, R² and R³ is each independently hydrogen atom or a substituent; n is 0 or an integer of 1 to 10), a salt thereof, a solvate thereof, or an N-oxide thereof, or a prodrug thereof; and an agent for prevention and/or treatment for a cysteine protease-related disease, such as osteoporosis, comprising it as an active ingredient are provided.

## Description

### TECHNICAL FIELD

The present invention relates to hydrazino-substituted heterocyclic nitrile compounds which are useful for the treatment of bone diseases such as osteoporosis, a method for the preparation thereof and use thereof.

### BACKGROUND OF ART

Cysteine protease is a generic name of proteases which have a cysteine residue in the activity center and catalyze protein degradation thereat. In animal cells, many cysteine proteases are known; for example, cathepsin family, calpain, caspase, *etc.* Cysteine protease exists in various kinds of cells extensively and plays a basic and essential role in the homeostasis, such as conversion of precursor protein into its active form (processing) and degradation of proteins which have become out of use, *etc.* Until now, its physiological effects are being vigorously studied, and as the studies progress and characters of the enzymes are revealed, cysteine proteases came to be taken as a cause of really various kinds of diseases.

It is revealed that cathepsin S (see J. Immunol., 161, 2731 (1998)), cathepsin L (see J Exp. Med., 183, 1331 (1996)) and cathepsin F *(*J Exp. Med., 191, 1177 (2000)) play a role in processing of major histocompatibility complex class-II in antigen presenting cells which play an important role in the early stage of immune responses. In an experimental inflammatory response model induced by antigens, a specific inhibitor of cathepsin S showed an inhibitory effect (see J. Clin. Invest., 101, 2351 (1998)). It is also reported that in a leishmania-infected immune response model a cathepsin B inhibitor controlled an immune response and by means of this effect it inhibited the proliferation of protozoans (see J. Immunol., 161, 2120 (1998)). In vitro, a result is given that a calpain inhibitor and a cysteine protease inhibitor E-64 inhibited apoptosis which is induced by stimuli on T cell receptors (see J. Exp. Med., 178, 1693 (1993)). And cathepsin W, which is expressed in CD8 T cells and NK cells specifically, is known to increase its expression by stimuli of IL-2 by 7 times and so it is conceived that it is concerned with immune responses (see J. Immunol., 167, 2172 (2001)). It is also reported that in leukemia patients, gene expression of cathepsin C and cathepsin W increase and cytotoxic T cells are activated (see Int. J. Oncol., 22, 33 (2003)). Therefore, it is conceivable that cysteine proteases are much concerned with the progress of immune responses.

It is speculated that caspase or a similar cysteine protease thereto occupies an important position in the mechanism of cell death including apoptosis. Therefore it is expected for a cysteine protease inhibitor to be used as an agent for the prophylaxis and/or treatment of those diseases concerning apoptosis, such as infectious diseases, deterioration or sthenia of immune function and brain function, or tumors *etc.* Diseases concerning apoptosis include, acquired immune deficiency syndrome (AIDS), AIDS-related complex (ARC), adult T cell leukemia, hairy cell leukemia, spondylopathy, respiratory apparatus disorder, arthritis, virus-related diseases (HIV, HTLV-1 related diseases (uveitis, *etc.)* and hepatitis C, *etc.),* cancer, collagenosis (systemic lupus erythematosus, rheumatoid arthritis, *etc.*), autoimmune diseases (inflammatory bowel diseases, Sjoegren syndrome, primary biliary cirrhosis, spontaneous thrombocytopenic purpura, autoimmune hemolytic anemia, myasthenia gravis, insulin dependent (type-I) diabetes, *etc.),* diseases accompanied by thrombocytopenia (osteomyelodysplasia syndrome, periodic thrombocytopenia, aplastic anemia, spontaneous thrombocytopenia, disseminated intravascular coagulation (DIC), *etc.),* hepatic diseases such as viral hepatitis (C, A, B, F, *etc.)* or hepatitis medicamentosa and cirrhosis, dementia (Alzheimer's disease, Alzheimer's senile dementia, *etc.),* cerebrovascular injury, nerve degeneration diseases, adult acute respiratory distress syndrome, infectious diseases, prostate hypertrophy, hysteromyoma, bronchial asthma, arteriosclerosis, all kinds of lusus naturae, nephropathy, senile cataract, chronic fatigue syndrome, myodystrophy, peripheral neuropathy, *etc.*

Moreover, caspase-1 is concerned with various inflammatory diseases and those diseases caused by immune disorders, by means of interleukin-1β (IL-1β) production. A lot of diseases are shown to be involved with caspase-1; for example, inflammatory bowel diseases such as ulcerative colitis, inflammatory diseases and autoimmune disease (insulin-dependent (type-I) diabetes, autoimmune thyroid diseases, infectious diseases, rejection of an organ transplant, graft versus host diseases, psoriasis, periodontitis (above, see N. Eng. J Med., 328, 106 (1993)), pancreatitis (see J. Interferon Cytokine Res., 17, 113 (1997)), hepatitis (see J. Leuko. Biol., 58, 90 (1995)), glomerulonephritis (see Kidney Int., 47, 1303 (1995)), endocarditis (see Infect. Immun., 64, 1638 (1996)), myocarditis (see Br. Hearat J., 72, 561 (1995)), systemic lupus erythematosus (see Br. J. Rheumatol., 34, 107 (1995)), Hashimoto's diseases (see Autoimmunity, 16, 141 (1993)), *etc.), etc.* Experimentally, it is reported that in liver injury model induced by lipopolysaccharide and D-galactosamine, a caspase-1 inhibitor improved the symptoms, and it is expected that a caspase inhibitor shows an effect in sepsis, ischemic reperfusion and hepatitis gravis (see Am. J_ Respir. Crit. Care Med., 159, 1308 (1999)).

It is also shown that cysteine protease is concerned with rheumatoid arthritis. IL-1β is shown to be concerned with this disease (see Arthritis Rheum., 39, 1092 (1996)), and in addition, as autoantibody toward calpastatin (endogenous calpain inhibitor) was found in the serum of the patients (see Proc. Natl. Acad. Sci. USA, 92, 7267 (1995)), it is thought that increase of calpain activity leads to the cause of diseases. Also, it is reported that cathepsin B and cathepsin C activity is increased in leukocyte of patients suffering from rheumatoid arthritis (see Biol. Chem-, 383, 865 (2002)). It is reported that in experimental arthritis model the production of inflammatory cytokine is suppressed and affection of arthritis completely in cathepsin C knock-out mice, so it is expected that cathepsin C inhibition leads to treatment of rheumatoid arthritis (see J. Clin. Invest., 109, 357 (2002)).

It is also known that cysteine protease causes a disease symptom by decomposing various proteins which compose the organism.

It is reported that cathepsin B plays a role in decomposing muscular protein in the chronic phase of sepsis (see J. Clin. Invest., 97, 1610 (1996)), and in decomposing muscular protein in myodystrophy model (see Biochem. J., 288, 643 (1992)). At the same time it is reported that calpain decomposes the myocyte cell proteins of myodystrophy patients (see J. BioL Chem., 270, 10909 (1995)).

In ischemic reperfusion model, a result is given that calpain causes degeneration of brain tissues by means of degradation of protein kinase C-β (see J. Neurochem., 72, 2556 (1999)) and that a cathepsin B inhibitor inhibits nerve injury (see Eur. J. Neurosci., 10, 1723 (1998)).

In the brain ischemic model, it is known that the degradation of spectrin by calpain causes a damage and its function disorder in the neurocyte (see Brain Res., 790, 1 (1998)) and it is reported that an IL-1β receptor antagonist relieved the symptoms (see Brain Res. Bull., 29, 243 (1992)).

In myocardial ischemic model it is confirmed that cathepsin B activity increases in the lesion (see Biochem- Med. Metab. Biol., 45, 6 (1991)).

In the experiment utilizing ischemic liver injury model, it proved that necrosis and apoptosis of hepatocyte were induced by means of protein-decomposing activity of calpain (see Gastroenterology, 116, 168 (1999)).

Otherwise, it is known that calpain causes cornea turbid by means of degradation of crystalline (see Biol. Chem., 268, 137 (1993)) and that in the lesion of contracted gut mucosa model it was confirmed that the activity of cathepsin B, H and L increased (see J. Parenter. Enteral. Nutr., 19, 187 (1995)) and it is shown that cysteine protease is a cause of the diseases resulting from these protein degradation.

It has been revealed that cysteine protease is concerned with systemic disorders of organs and tissues by shock.

It is shown that IL-1β is concerned with septic shock and systemic inflammatory response syndrome (see Igakuno Ayumi, 169, 850 (1994)) and besides, it is reported that in endotoxin shock model induced by lipopolysaccharide, a calpain inhibitor prevented circulatory system disorder, disorders of liver and pancreas and acidosis by means of inhibitory effect of activation of nuclear factor κB (see Br. J. Pharmacol., 121, 695 (1997)).

Since it is reported that calpain is concerned with platelet coagulation process and a calpain inhibitor prevented platelet coagulation (see Am. J Physiol., 259, C862 (1990)), it is conceivable that a cysteine protease inhibitor is useful for the disorder of blood coagulation. From the fact that calpain activity increased in the serum of the patients of purpura (thrombocytopenia) resulting from marrow transplantation, so it is conceivable that calpain is concerned with the actual disease symptoms (see Bone Marrow Transplant., 24, 641 (1999)).

Caspase-1 inhibitor suppressed apoptosis of blood vessel endothelial cells, which is seen in the early phase of purpura (thrombocytopenia) and is thought to be important for the progression of the pathology afterwards (see Am. J. Hematol., 59, 279 (1998)), so it is expected that a cysteine protease inhibitor makes effect on purpura and hemolytic uremic syndrome.

The effect of cysteine protease and its inhibitor is being investigated in the area of cancer and metastasis of cancer.

Since the proliferations of pancreas cancer cells (see Cancer Res., 59, 4551 (1999)) and acute myeloid leukemia cells (see Clin. Lab. Haematol., 21, 173 (1999)) were inhibited by a caspase-1 inhibitor or its receptor antagonist, it is expected that caspase-1 activity is essential for the process of proliferation of tumor cells, and that an inhibitor thereof is effective for these cancers. Also, from the facts that cathepsin B activity increased in colon cancer metastasis model (see Clin. Exp. Metastasis, 16, 159 (1998)), that cathepsin L activity increased in urine of bladder cancer patients (see Urology, 59, 308 (2002)), that cathepsin Z expression was recognized in tumor cells (see J. Biol. Chem., 273, 16816 (1998)), that cathepsin K protein expression recognized in human breast cancer cells proved the relationship of cathepsin K and bone metastasis (see Cancer Res., 57, 5386 (1997)), and that a calpain inhibitor suppressed migration of the cells, which implies that calpain inhibition might be able to inhibit metastasis of cancer (see J. Biochem., 272, 32719 (1997)), a cysteine protease inhibitor is expected to exhibit an inhibitory effect on the metastasis of various malignant tumors.

As to AIDS (see AIDS, 10, 1349 (1996)) and AIDS-related complex (ARC) (see Arch. Immunol. Ther. Exp. (Warsz), 41, 147 (1993)), it is implied that IL-1 is concerned with the progress of symptoms, and so it is conceivable that cysteine protease inhibition leads to an effective therapy of AIDS and its complication.

Some parasites have cysteine protease activity in their bodies. Cysteine protease in the phagosome of malaria protozoan is an essential enzyme for supplying nutrition of the parasites. Its inhibitor show an inhibitory effect of the proliferation of the protozoan (see Blood, 87, 4448 (1996)). Cystein protease inhibitor is thought of as drug for treatment of malaria.

In Alzheimer-type dementia, it is said that adhesion of non-physiological protein called amyloid to brain is deeply involved with nervous function disorders. Cysteine protease has an activity of generating amyloid by decomposing its precursor protein. Clinically, it is shown that cathepsin B possesses a processing activity of amyloid proteins in the brains of Alzheimer-type dementia patients (see Biochem. Biophys. Res. Commun., 177, 377 (1991)). And expressions of cathepsin B protein (see Virchows Arch. A. Pathol. Anat. Histpathol., 423, 185 (1993)), cathepsin S protein (see Am. J Pathol., 146, 848 (1995)) and calpain protein (see Proc. Natl. Acad Sci. USA, 90, 2628 (1993)) and increase of caspase-1 activity (see J. Neuropathol. Exp. Neurol., 58, 582 (1999)) were confirmed in the brain lesions. And it is implied that cysteine protease is concerned with the disease symptoms, by the fact that calpain is concerned with the formation of paired helical filaments which accumulate in Alzheimer dementia patients and production of protein kinase C which stabilizes the protein (see J. Neurochem., 66, 1539 (1996)) and by the knowledge that caspase is concerned with neurocyte death by β amyloid protein adhesion (see Exp. Cell Res., 234, 507 (1997)).

As to Huntington's chorea, cathepsin H activity increased in the patient's brain (see J. Neurol. Sci., 131, 65 (1995)), and the ratio of activated form of calpain (see J. Neurosci., 48, 181 (1997)) increased. In Parkinson's disease, the increase of expression of m-calpain was recognized in the mesencephalon in the patients (see Neuroscience, 73, 979 (1996)) and IL-1β protein was expressed in brain (see Neurosci. Let., 202, 17 (1995)). Therefore, it is speculated that cysteine protease is concerned with the genesis and progress of these diseases.

Otherwise, in the central nervous system, spectrin degradation by calpain is found in the process of injury on neurocyte observed in the traumatic brain injury model (see J. Neuropathol. Exp. Neurol., 58, 365 (1999)).

In spinal cord injured model it was recognized that in glia cells calpain messenger RNA increased and its activity increased in the lesion and the possibility was shown that calpain had much to do with the degeneration of myelin and actin (see Brain Res., 816, 375 (1999)). And IL-1β was shown to be concerned with the genesis of multiple sclerosis (see Immunol. Today, 14, 260 (1993)). Therefore, it is conceivable that a cysteine protease inhibitor is hopeful as an agent for the treatment of these nerve-injured diseases.

Normally, cathepsin S and cathepsin K do not exist in human arterial walls, but it was confirmed that they expressed in arteriosclerosis lesion and they had an decomposing activity of alveolus elastica (see J. Clin. Invest., 102, 576 (1998)) and a calpain inhibitor and antisense of m-calpain inhibited the proliferation of human blood vessel smooth muscle cells and it is shown that m-calpain is concerned with the proliferation of smooth muscle (see Arteioscler. Thromb. Vssc. Biol., 18, 493 (1998)), so it is conceivable that a cysteine protease inhibitor is hopeful for the treatment of blood vessel lesion such as arteriosclerosis, restenosis after percutaneous transluminal coronary angioplasty (PTCA), *etc.* And it is also reported that LDL induces cathepsin H expression in human monocyte and cathepsin H is concerned with LDL transformation and it is implied that LDL is concerned with circulatory disorder (arteriosclerosis) (see Arterioscler. Thromb. Vasc. Biol., 27 (2003)).

It is reported that in liver, cathepsin B is activated in the process of injuring hepatocyte by bile acid (see J Clin. Invest., 103, 137 (1999)) and so it is expected that a cysteine protease inhibitor is useful for cholestatic cirrhosis.

It is reported that in spleen, cathepsin Y is concerned with production of bradykinin potentiating peptide (BPP) which plays some role in converting kinin into bradykinin (see Immunopharmacology, 45, 207 (1999)). Therefore, it is expected that cathepsin Y inhibitor has anti-allergy effect.

In lungs and respiratory system, it is shown that cathepsin S is an enzyme that plays a role in elastin degradation by alveolus macrophages (see J. Biol. Chem., 269, 11530 (1994)), so it is probable that cysteine protease is a cause of pulmonary emphysema. In IL-13 transgenic mice in which COPD-like pathology is recognized, increase of cathepsin B, S, L, H and K expression is recognized and it is also reported that administration of a cysteine protease inhibitor suppresses lung inflammation and lung emphysema (see J. Clin. Invest., 106, 1081 (2000)). And it is also shown that lung injury (see J. Clin. Invest., 97, 963 (1996)), lung fibrosis (see Cytokine, 5, 57 (193)) and bronchial asthma (see J Immunol, 149, 3078 (1992)) are caused by way of production of IL-1β by caspase-1. It is also shown that blood cathepsin H concentration is increased in asthma patients, so antiasthma effect by cathepsin H inhibitor is expected (see Clin. Chim. Acta, 310, 113 (2001)). It is known that cathepsin H functions in the excision of surfactant protein C which is synthesized by type-2 pneumonia cells (see Am. J Respir. Cell Mol. Biol., 26, 659 (2002)).

It is pointed out that cysteine protease is also concerned with diseases concerning bones and cartilages. Cathepsin K is specifically recognized in osteoclast and it has a decomposing activity against bone matrix (see J Biol. Chem., 271, 12517 (1996)), so cathepsin K inhibitor is expected to show an effect in bone diseases where pathologic bone resorption is recognized, such as osteoporosis, bone fracture, arthritis, rheumatoid arthritis, osteoarthritis, hypercalcaemia, osteometastasis of cancer, periodontitis, bone Paget's disease, *etc.* Also, since IL-1β is shown to be concerned with bone resorption and cartilage degradation, and a caspase-1 inhibitor and IL-1β receptor antagonist inhibit the symptoms of bone resorption and arthritis, so it is expected that it is effective for arthritis (see Cytokine, 8, 377 (1996)) and osteoporosis (see J. Clin. Invest., 93, 1959 (1994)). And it is also reported that IL-1β is concerned with osteoarthritis (see Life Sci., 41, 1187 (1987)).

Cysteine protease is involved with production of various hormones. Since increase of messenger RNA of cathepsin S was recognized by stimuli of thytropin on thyroid epitheliocyte strains (see J. Biol. Chem., 267, 26038 (1992)), it is conceivable that a cysteine protease inhibitor is effective for hyperthyrodism.

Since quantity and activity of cathepsin B protein increased in the gingival sulcus liquid of periodontitis patients (see J. Clin. Periodontol., 25, 34 (1998)), it is pointed out that cysteine protease is concerned with periodontitis. Cathepsin G is concerned with abnormal connective tissue decomposition.

Therefore, those compounds which have an inhibitory activity against cysteine proteases, are useful as agents for the prophylaxis and/or treatment of inflammatory diseases (periodontitis, arthritis, inflammatory bowel diseases, infectious diseases, pancreatitis, hepatitis, glomerulonephritis, endocarditis, myocarditis, ulcerative colitis, *etc.),* diseases induced by apoptosis (graft versus host diseases, rejection in transplantation, acquired immunodeficiency syndrome (AIDS), AIDS-related complex (ARC), adult T cell leukemia, hairy cells leukemia, spondylopathy, disorders of respiratory apparatus, arthritis, HIV or HTLV-1 related diseases (uveitis *etc.),* virus related diseases (hepatitis C *etc.),* cancer, collagenosis (systemic lupus erythematosus, rheumatoid arthritis, *etc*.), ulcerative colitis, Sjoegren syndrome, primary biliary cirrhosis, spontaneous thrombocytopenic purpura, autoimmune hemolytic anemia, myasthenia gravis, autoimmune diseases (insulin dependent (type I) diabetes, *etc*.), diseases accompanied by thrombocytopenia (myelodysplastic syndrome, cyclic thrombocytopenia, aplastic anemia, spontaneous thrombocytopenia, disseminated intravascular coagulation (DIC), *etc.),* viral hepatitis (A, B, C, F, *etc.)* or hepatitis medicamentosa and cirrhosis, *etc*., dementia such as Alzheimer's disease, Alzheimer-type senile dementia, cerebrovascular injury, neurodegenerative disease, adult acute respiratory distress syndrome, infectious diseases, prostatomegaly, hysteromyoma, bronchial asthma, arteriosclerosis, hyperlipidemia, all kinds of lusus naturae, nephritis, senile cataract, chronic fatigue syndrome, myodystrophy, peripheral nerve injury, *etc*.), diseases induced by immune response disorder (graft versus host diseases, rejection in transplantation, allergic diseases (asthmatic bronchitis, atopic dermatitis, allergic rhinitis, hay fever, diseases by house dust, hypersensitive pneumonia, food allergy, *etc.),* psoriasis, rheumatoid arthritis, *etc.),* autoimmune diseases (insulin dependent (type I) diabetes, systemic lupus erythematosus, Hashimoto's diseases, multiple sclerosis, *etc.),* diseases induced by decomposition of proteins which compose a body (myodystrophy, cataract, periodontitis, hepatocyte injury by bile acid (cholestatic cirrhosis *etc.), etc.,* decomposition of alveolus elastica (emphysema etc.), ischemic diseases (brain ischemia, brain disorder by ischemic reperfusion, cardiac infarction, ischemic liver damage, *etc.), etc.),* shock (septic shock, systemic inflammatory responsive syndrome, endotoxin shock, acidosis, *etc*.), circulatory disorder (arteriosclerosis, restenosis after PTCA (percutaneous transluminal coronary angioplasty), *etc*.), disorder of blood coagulation system (thrombocytopenic purpura, hemolytic uremic syndrome, *etc*.), malignant tumor, acquired immune deficiency syndrome (AIDS, AIDS-related complex (ARC), *etc.),* parasitic diseases (malaria *etc*.), neurodegenerative disease (Alzheimer-type senile dementia, Huntington's chorea, Parkinson's disease, multiple sclerosis, traumatic encephalopathy, traumatic spondylopathy, *etc.*), lung disorder (fibroid lungs, *etc*.), bone diseases (osteoporosis, bone fracture, rheumatoid arthritis, arthritis, osteoarthritis, hypercalcaemia, osteometastasis of cancer, periodontitis, bone Paget's disease, *etc*.), endocrinesthenia (hyperthyroidism *etc.), etc.*

On the other hand, what is the most important for inhibitors in inhibiting the activity of proteases is, the special reaction site which interacts with the amino acid residues the activity center of proteases. The surrounding structure of the reaction sites are represented by ---P3P2P1-P1'P2'P3'---, centering peptide binding (P1-P1') of the reaction site, and at P1 site there exist amino acid residues which fit the substance specificity of proteases which the inhibitors aim. Some reaction sites against cysteine proteases are known, for example, in the specification of WO99/54317, the followings are described; P1 position against calpain I, II such as norvaline, phenylalanine, *etc.;* P1 position against calpain I such as arginine, lysine, tyrosine, valine, *etc*.; P1 position against papain such as homophenylalanine, arginine, *etc.;* P1 position against cathepsin B such as homophenylalanine, phenylalanine, tyrosine, *etc.;* P1 position against cathepsin S such as valine, norleucine, phenylalanine, *etc,;* P1 position against cathepsin L such as homophenylalanine, lysine, *etc.;* P1 position against cathepsin K such as arginine, homophenylalanine, leucine, *etc.;* P1 position against caspase such as aspartic acid, *etc.*

As a compound having an inhibitory activity against cathepsin K, known is a compound represented by formula (a): 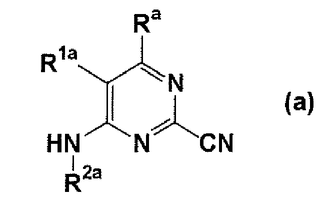 wherein R^{a} is hydrogen, R^{4a}, OR^{4a}or NR^{3a}R^{4a}; R^{1a} is CO-NR^{5a}R^{6a}, NH-COR^{5a}, -CH₂-NH-C(O)-R^{5a}, -CO-R^{5a}, -S(O)-R^{5a}, -S(O)₂-R^{5a}, -CH₂-CO-R^{5a}, -CH₂-NR^{5a}R^{6a}; R^{2a} is hydrogen or lower alkyl, aryl, aryl-lower alkyl, C3-10 cycloalkyl, C3-10 cycloalkyl-lower alkyl, a heterocyclic group or heterocyclic group-lower alkyl, which each may be substituted; and R^{2a} is halo, hydroxy, oxo, lower alkoxy, CN, NO₂, or amino substituted with mono- or di-lower alkyl which may be substituted (WO03/020278).

### DISCLOSURE OF THE INVENTION

A task of the present invention is to provide medicaments which are useful for the prevention and/or treatment of bone diseases such as osteoporosis.

Based on the above problems, the present inventors have energetically investigated to find such compounds having an inhibitory activity against cysteine protease, to find out that the hydrazino-substituted heterocyclic nitrile compound of formula (I) accomplishes the purpose.

The compound in the present invention has an inhibitory activity against cysteine protease, so it is useful for the treatment and/or prevention of bone metabolic diseases and the like.

That is, the present invention relates to the followings:
1. A compound represented by formula (I): 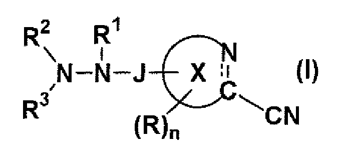 wherein ring X is a heterocyclic group which may contain 1 to 4 hetero atoms selected from a nitrogen atom(s), an oxygen atom(s) and a sulfur atom(s) which may be oxidized, in addition to the nitrogen atom in the ring; 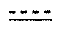 is a single bond or a double bond; J is a bond or a spacer having from 1 to 8 atoms of the principle chain; R is hydrogen atom or a substituent, and if R is plural, Rs are the same or different, and two Rs may form, together with the atom on the ring to which they bind, a cyclic group which may have a substituent(s); R¹, R² and R³ is each independently a hydrogen atom or a substituent; n is 0 or an integer of 1 to 10, and wherein R¹ and R² may form, together with the nitrogen atom to which they bind, a cyclic group which may have a substituent(s), and R² and R³ may form, together with the nitrogen atom to which they bind, a cyclic group which may have a substituent(s),
   a salt thereof, a solvate thereof, or an N-oxide thereof, or a prodrug thereof.
2. The compound according to the above-described 1, wherein the ring X is a 5- to 7-membered monocyclic heterocyclic group which may contain 1 to 3 hetero atoms selected from a nitrogen atom(s), an oxygen atom(s) and a sulfur atom(s) which may be oxidized, in addition to the nitrogen atom in the ring.
3. The compound according to the above-described 1, wherein the ring X is 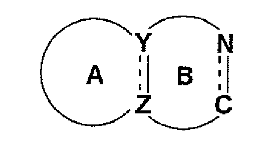 wherein ring A is a carbocyclic group or a heterocyclic group; ring B is a nitrogen-containing heterocyclic group; Y and Z is each independently a carbon atom or a nitrogen atom; and other symbols have the same meanings as in the above-described 1.
4. The compound according to the above-described 2, wherein the ring X is a ring selected from tetrahydropyrimidine, dihyrdopyridazine, pyridazine, dihydropyrimidine, dihydropyrazine, dihydrotriazine, dihydropyrazole, pyrrole, pyrimidine, pyrazine, imidazole, pyrazole, triazole, tetrazole, thiazole, oxazole, thiadiazole, oxadiazole, azepane, azepine, dihydroazepine, tetrahydroazepine, diazepane, diazepine, dihydrodiazepine and tetrahydrodiazepine.
5. The compound according to the above-described 1, wherein J is a bond.
6. The compound according to the above-described 1, wherein R¹ is C1-8 alkyl which may have a substituent(s).
7. The compound according to the above-described 1, wherein R² is C1-8 alkyl which may have a substituent(s), C1-8 alkylcarbonyl which may have a substituent(s), C1-8 alkylsulfonyl which may have a substituent(s), carbamoyl which may have a substituent(s), a carbocyclic group which may have a substituent(s), a heterocyclic group which may have a substituent(s), 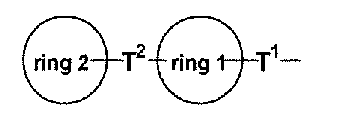 wherein T¹ and T² is each independently a bond or a spacer having from 1 to 3 atoms of the principle chain, and ring 1 and ring 2 is each independently a cyclic group which may have a substituent(s), or 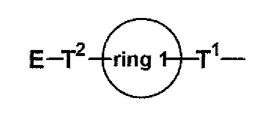 wherein E is a substituent containing a nitrogen atom having basicity, and other symbols have the same meanings as above, and
   R³ is hydrogen atom.
8. The compound according to the above-described 1, which is represented by formula (I-4): 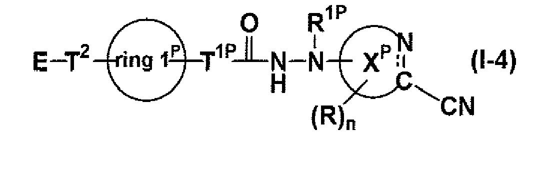 wherein ring X^{P} is a 5- to 7-membered monocyclic heterocyclic group which may contain 1 to 3 hetero atoms selected from a nitrogen atom(s), an oxygen atom(s) and a sulfur atom(s) which may be oxidized, in addition to the nitrogen atom in the ring; R^{1P} is C1-8 alkyl which may have a substituent(s); T^{1P} is a bond or a spacer having 1 or 2 atoms of the principle chain; ring1^{P} is a C5 to 7 monocyclic carbocyclic group which may have a substituent(s) or a 5- to 7-membered monocyclic heterocyclic group which may have a substituent(s); and other symbols have the same meanings as in the above-described 1 or 7.
9. The compound according to the above-described 1, which is
   N'-[2-cyano-6-(trifluoromethyl-4-pyrimidinyl]-4-[(4-methyl-1-piperazinyl)methyl]-N'-neopentylbenzohydrazide,
   N'-(2-cyano-5-methyl-4-pyrimidinyl)-4-[(4-methyl-1-piperazinyl)methyl]-N'-neopentylbenzohydrazide,
   N'-[2-cyano-6-(trifluoromethyl-4-pyrimidinyl]-2-(dimethylamino)-N'-neopentylacetohydrazide,
   N'-(5-bromo-2-cyano-4-pyrimidinyl)-4-[1-(2-methoxyethyl)-4-piperidinyl]-N'-neopentylbenzohydrazide,
   N'-[2-cyano-6-(trifluoromethyl-4-pyrimidinyl]-4-[(dimethylamino)methyl]-N'-neopentylbenzohydrazide,
   N'-[2-cyano-6-(trifluoromethyl-4-pyrimidinyl]-N'-neopentylnicotinohydrazide,
   N'-[2-cyano-6-(trifluoromethyl-4-pyrimidinyl]-4-(4-morpholinylmethyl)-N'-neopentylbenzohydrazide,
   N'-[2-cyano-6-(trifluoromethyl-4-pyrimidinyl]-4-[(dimethylamino)methyl]-3-fluora-N'-neopentylbenzohydrazide,
   N'-(2-cyanopyrazolo[1,5-a][1,3,5]triazin-4-yl)-4-[1-(2-methoxyethyl)-4-piperidinyl]-N'-neopentylbenzohydrazide,
   N'-[2-cyano-6-(trifluoromethyl-4-pyrimidinyl]-2-methyl-2-[4-(1-methyl-4-piperidinyl)phenyl]-N'-neopentylpropanohydrazide,
   N'-(2-cyano-6-methyl-4-pyrimidinyl)-4-[(dimethylamino)methyl]-N'-neopentylbenzohydrazide,
   N'-[2-cyano-6-(trifluoromethyl-4-pyrimidinyl]-2-{4-[(dimethylamino)methyl]phenyl}-2-methyl-N'-neopentylpropanohydrazide,
   N'-(2-cyano-6-methyl-4-pyrimidinyl)-4-[2-(dimethylamino)ethoxy]-N'-neopentylbenzohydrazide,
   N'-(2-cyano-6-methyl-4-pyrimidinyl)-2-{4-[(dimethylamino)methyl]phenyl}-2-methyl-N'-neopentylpropanohydrazide,
   N'-[2-cyano-6-(trifluoromethyl-4-pyrimidinyl]-N'-neopentyl-2-(1-pyrrolidinyl)acetohydrazide,
   N'-[2-cyano-6-(trifluoromethyl-4-pyrimidinyl]-N'-(2,2-dimethylpropyl)-6-(4-methyl-1-piperazinyl)nicotinohydrazide,
   N'-[2-cyano-6-(trifluoromethyl-4-pyrimidinyl]-N'-(2,2-dimethylpropyl)-4-{[(2-methoxyethyl)(methyl)amino]methyl} benzohydrazide,
   N'-(2-cyano-6,7-dihydro-5H-cyclopenta[d]pyrimidin-4-yl)-4-[(dimethylamino)methyl]-N'-(2,2-dimethylpropyl)benzohydrazide,
   N'-[2-cyano-6-(trifluoromethyl-4-pyrimidinyl]-3-(dimethylamino)-N'-(2,2-dimethylpropyl)propanohydrazide,
   N'-(2-cyano-6,7-dihydro-5H-cyclopenta[d]pyrimidin-4-yl)-2-{4-[(dimethylamino)methyl]phenyl}-N'-(2,2-dimethylpropyl)-2-methylpropanohydrazide,
   N'-(2-cyano-6-methyl-4-pyrimidinyl)-N'-(2,2-dimethylpropyl)-2-methyl-2-{4-[(4-methyl-1-pyrimidinyl)methyl]phenyl}propanohydrazide,
   N'-[2-cyano-6-(trifluoromethyl-4-pyrimidinyl]-4'-[(dimethylamino)methyl]-N'-(2,2-dimethylpropyl)-4-biphenylcarbohydrazide,
   N'-(2-cyano-6-methyl-4-pyrimidinyl)-4-[(dimethylamino)methyl]-N'-(2,2-dimethylpropyl)-3-methoxybenzohydrazide,
   N'-[2-cyano-6-(trifluoromethyl-4-pyrimidinyl]-N'-(2,2-dimethylpropyl)-2-(methylamino)acetohydrazide, or
   *tert*-butyl 2-(2-cyano-5,7-dihydrofuro[3,4-d]pyrimidin-4-yl)-2-(2,2-dimethylpropyl)hydrazinecarboxylate.
10. A pharmaceutical composition comprising the compound of formula (I) according to the above-described 1, a salt thereof, a solvate thereof, or an N-oxide thereof, or a prodrug thereof as an active ingredient.
11. The pharmaceutical composition according to the above-described 10, which is an agent for prevention and/or treatment for a cysteine protease-related disease.
12. The pharmaceutical composition according to the above-described 11, wherein a cysteine protease-related disease is a cathepsin K-related disease.
13. The pharmaceutical composition according to the above-described 12, wherein a cathepsin K-related disease is a bone disease.
14. The pharmaceutical composition according to the above-described 13, wherein a bone disease is at least one selected from osteoporosis, bone fracture, arthritis, rheumatoid arthritis, osteoarthritis, hypercalcaemia, osteometastasis of cancer, osteosarcoma, periodontitis, and bone Paget's disease.
15. The pharmaceutical composition according to the above-described 10, which is a bone resorption inhibitor.
16. A drug comprising the compound of formula (I) according to the above-described 1, a salt thereof, a solvate thereof, or an N-oxide thereof, or a prodrug thereof, combined with at least one selected from bisphosphonate formulations, vitamin D and its derivatives, vitamin K and its derivatives, calcitonin formulations, α-calcitonin gene-related peptide formulations, female hormone formulations, selective estrogen receptor modulators, ipriflavone formulations, calcium formulations, anabolic steroid formulations, parathyroid hormone formulations, PTHrP derivatives, caspase-1 inhibitors, farnesoid X receptor agonists, Bone Morphogenetic Protein formulations, anti-RANKL antibody, GSK inhibitor, metalloprotease inhibitors, prostaglandin derivatives, strontium formulations, anti TNF-α antibody, anti-IL-6 antibody, HMG-CoA reductase inhibitors, steroidal drugs, and antiinflammatory drugs.
17. A method for the prophylaxis and/or treatment of a cysteine protease-related disease in a mammal characterized by administering to a mammal an effective amount of the compound of formula (I) according to the above-described 1, a salt thereof, a solvate thereof, or an N-oxide thereof, or a prodrug thereof.
18. Use of the compound of formula (I) according to the above-described 1, a salt thereof, a solvate thereof, or an N-oxide thereof, or a prodrug thereof, for the manufacture of a pharmaceutical preparation for the prophylaxis and/or treatment of a cysteine protease-related disease.

Definition of the term in the present invention is described below.

The "heterocyclic group which may contain 1 to 4 hetero atoms selected from a nitrogen atom(s), an oxygen atom(s) and a sulfur atom(s) which may be oxidized, in addition to the nitrogen atom in the ring" represented by ring X is, for example, a monocyclic or polycyclic aromatic heterocyclic group which may be partially or fully saturated, contains one nitrogen atom and may further contain 1 to 4 hetero atoms selected from a nitrogen atom(s), an oxygen atom(s) and a sulfur atom(s) which may be oxidized (S, S(O), SO₂). Herein, the "monocyclic aromatic heterocyclic group" includes, for example, a 5- to 10-membered monocyclic aromatic heterocyclic group.

The "5- to 10-membered monocyclic aromatic heterocyclic group which may be partially or fully saturated, contains one nitrogen atom and may further contain 1 to 4 hetero atoms selected from a nitrogen atom(s), an oxygen atom(s) and a sulfur atom(s) which may be oxidized" represented by ring X includes, for example, pyrimidine, pyridine, pyrazine, triazine, pyridazine, imidazole, pyrrole, thiazole, oxazole, triazole, pyrazole, thiadiazole, oxadiazole, isothiazole, isoxazole, piperidine, tetrahydropyridine, dihyrdopyridine, tetrahydropyrimidine, dihydropyrimidine, hexahydropyrimidine, azepane, tetrahydroazepine, dihydroazepine, diazepane, tetrahydroazepine, dihyrdodiazepine, triazepane, tetrahydrotriazepine and dihyrdotriazepine rings, etc.

The "polycyclic heterocyclic group which may be partially or fully saturated, contains one nitrogen atom and may further contain 1 to 4 hetero atoms selected from a nitrogen atom(s), an oxygen atom(s) and a sulfur atom(s) which may be oxidized" represented by ring X includes, for example, 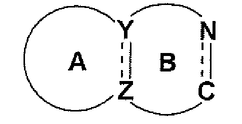 (wherein ring A is a carbocyclic group or a heterocyclic group; ring B is a nitrogen-containing heterocyclic group; Y and Z is each independently a carbon atom or a nitrogen atom; and 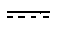 is a single bond or a double bond).

The "carbocyclic group" represented by ring A includes, for example, C3-15 mono-, polycyclic aromatic cyclic group, the carbocyclic group which is partially or fully saturated, a polycyclic carbocyclic group having a spiro bond, a polycyclic bridged carbocyclic group, *etc..* The C3-15 mono-, polycyclic aromatic cyclic group, the carbocyclic group which is partially or fully saturated includes, for example, cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indan, naphthalene, dihydronaphthalene, teterahydronaphthalene, perhydronaphthalene, heptalene, perhydroheptalene, biphenylene, as-indacene, s-indacene, acenaphthylene, acenaphthene, fluorene, phenalene, phenanthrene and anthracene rings, *etc.* The polycyclic carbocyclic group having a spiro bond and the polycyclic bridged carbocyclic group include, for example, spiro[4.4]nonane, spiro[4.5]decane, spiro[5.5]undecane, bicyclo[2.2.1]heptane, bicyclo[2.2.1]hept-2-ene, bicyclo[3.1.1]heptane, bicyclo[3.1.1]hept-2-ene, bicyclo[2.2.2]octane, bicyclo[2.2.2]oct-2-ene, bicyclo[3.3.0]octane, bicyclo[4.3.0]nonane, bicyclo[4.4.0]decane, adamantane and noradamantane rings, *etc.*

The "heterocyclic group" represented by ring A includes, for example, a 3-to 15- membered monocyclic or polycyclic aromatic heterocyclic group which may be partially or full saturated and contains 1 to 5 hetero atoms selected from an oxygen atom(s), a nitrogen atom(s) and a sulfur atom(s) which may be oxidized (S, SO, SO₂), a polycyclic heterocyclic group having a spiro bond, a polycyclic bridged heterocyclic group, *etc.* The 3- to 15- membered monocyclic or polycyclic aromatic heterocyclic group which may be partially or full saturated and contains 1 to 5 hetero atoms selected from an oxygen atom(s), a nitrogen atom(s) and a sulfur atom(s) which may be oxidized (S, SO, SO₂), the polycyclic heterocyclic group having a spiro bond, and the polycyclic bridged heterocyclic group include, for example, pyrrole, imidazole, triazole, tetrazole, pyrazole, pyridine, pyrazine, pyrimidine, pyridazine, triazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, pyrrolopyridine, benzoxazole, benzothiazole, thieno[3,2-c]pyridine, benzimidazole, chromene, benzoxepine, benzoxazepine, benzoxadiazepine, benzothiepine, benzothiazepine, benzothiadiazepine, benzazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine, pyridonaphthyridine, pyrazoloisoquinoline, pyrazolonaphthyridine, pyrimidoindole, aziridine, azetidine, pyrroline, pyrrolidine, imidazoline, imidazolidine, triazoline, triazolidine, tetrazoline, tetrazolidine, dihydropyrazole (pyrazoline), tetrahydropyrazole (pyrazolidine), dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrotriazine, tetrahydrotriazine, perhydrotriazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrathiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, octahydroquinoline, perhydroquinoline, dihydroisoquinoline, tetrahydroisoquinoline, octahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, tetrahydropyrrolopyridine, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrohenzothiazole, 4,5,6,7-tetrahydrothieno[3.2-c]pyridine, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, tetrahydronaphthyridine, tetrahydro-β-carboline, dihydroazepinoindole, hexahydroazepinoindole, tetrahydropyrazoloisoquinoline, tetrahydropyrazolonaphthyridine, dihydroazepinoindazole, hexahydroazepinoindazole, dihydropyrazolopyridoazepine, hexahydropyrazolopyridoazepine, tetrahydropyrimidoindole, dihydrothiazinoindole, tetrahydrothiazinoindole, dihydrooxazinoindole, tetrahydrooxazinoindole, 2,3-dihydro-IH-benzo[de]isoquinoline, dioxolane, dioxane, dithiolane, dithiane, benzodioxole, for example, 1,3-benzodioxole, *etc*., benzodioxane, chroman, benzodithiolane benzodithiane, azaspiro[4.4]nonane, oxaazaspiro[4.4]nonane, axaazaspiro[2.5]octane, dioxaspiro[4.4]nonane, azaspiro[4.5]decane, thiaspiro[4.5]decane, dithiaspiro[4.5]decane, dioxaspiro [4.5] decane, oxaazaspiro [4.5] decane, azaspiro[5.5]undecane, oxaspiro[5.5]undecane, dioxaspiro[5.5]undecane, 1,4-dioxa-8-azaspiro[4.5]undecane, 1,3,8-triazaspiro[4.5]decane, azabicyclo[2.2.1]heptane, oxabicyclo[2.2.1]heptane, azabicyclo[3.1.1]heptane, azabicyclo[3.2.1]octane, oxabicyclo [3.2.1]octane, azabicyclo[2.2.2]octane, diazabicyclo[2.2.2]octane, 2,5-diazabicyclo[2.2.1]heptane, 1,3,8-triazaspiro[4.5]decane, 2,7-diazaspiro[4.5]decane, 1,4,9-triazaspiro[5.5]undecane, 2,4,6-trioxaspiro[bicyclo[3.3.0]octane-3,1'-cyclohexane], 1,3-dioxolano[4,5-g]chromene, 2-oxabicyclo[2.2. 1]heptane, 2,3,4,9-tetrahydrospiro[β-carboline-1, 1'-cyclopentane] and 3,9-diazaspiro [5.5]undecane rings, *etc.*

The "nitrogen-containing heterocyclic group" represented by ring B includes, for example, a 3- to 15-membered monocyclic or polycyclic aromatic heterocyclic group which may be partially or fully saturated, contains one nitrogen atom and which may further contain 1 to 4 hetero atoms selected from a nitrogen atom(s), an oxygen atom(s) and a sulfur atom(s) which may be oxidized (S, S(O), SO₂), a polycyclic heterocyclic group having a spiro bond, a polycyclic bridged heterocyclic group, *etc.*

The "3- to 15-membered monocyclic or polycyclic aromatic heterocyclic group which may be partially or fully saturated, contains one nitrogen atom and which may further contain 1 to 4 hetero atoms selected from a nitrogen atom(s), an oxygen atom(s) and a sulfur atom(s) which may be oxidized (S, S(O), SO₂), polycyclic heterocyclic group having a spiro bond, and polycyclic bridged heterocyclic group" include, for example, pyrrole, imidazole, triazole, tetrazole, pyrazole, pyridine, pyrazine, pyrimidine, triazine, pyridazine, azepine, diazepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indazole, quinoline, isoquinoline, purine, phthalazine, pteridine, naphthyridine, quinoxaline, quinazoline, cinnoline, pyrrolopyridine, benzoxazole, benzothiazole, benzimidazole, benzoxazepine, benzoxadiazepine, benzothiazepine, benzothiadiazepine, benzazepine, benzodiazepine, β-carboline, phenanthridine, phenanthroline, pyridonaphthyridine, pyrazoloisoquinoline, pyrazolonaphthyridine, pyrimidoindole, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrotriazine, tetrahydrotriazine, perhydrotriazine, dihydroazepine, tetrahydroazepine, perhydroazepine, dihydrodiazepine, tetrahydrodiazepine, perhydrodiazepine, dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, indoline, isoindoline, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, tetrahydropyrrolopyridine, dihydrocinnoline, tetrahydrocinnoline, perhydrocinnoline, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, dihydrobenzoxazepine, tetrahydrobenzoxazepine, tetrahydronaphthyridine, tetrahydro-β-carboline, dihydroazepinoindole, hexahydroazepinoindole, tetrahydropyrazoloisoquinoline, tetrahydropyrazolonaphthyridine, dihydroazepinoindazole, hexahydroazepinoindazole, dihydropyrazolopyridoazepine, hexahydropyrazolopyridoazepine, tetrahydropyrimidoindole, dihydrothiazinoindole, tetrahydrothiazinoindole, azaspiro[4.4]nonane, oxaazaspiro[4.4]nonane, oxaazaspiro[2.5]octane, azaspiro[4.5]decane, oxaazaspiro[4.5]decane, azaspiro[5.5]undecane, 1,4-dioxa-8-azaspiro[4.5]undecane, 1,3, 8-triazaspiro [4.5] decane, azabicyclo[2.2.1]heptane, azabicyclo[3.1.1]heptane, azabicyclo[3.2.1]octane, oxabicyclo[3.2.1]octane, azabicyclo[2.2.2]octane, diazabicyclo[2.2.2]octane, 2,5-diazabicyclo[2.2.1]heptane, 1,3,8-triazaspiro[4.5]decane, 2,7-diazaspiro[4.5]decane, 1,4,9-triazaspiro[5.5]undecane, 2,3,4,9-tetrahydrospiro[β-carboline-1,1'-cyclopentane] and 3,9-diazaspiro[5.5]undecane rings, *etc.*

The substituent represented by R¹, R² and R³ includes, for example, (1) hydrocarbon group which may have a substituent(s), (2) carbocyclic group which may have a substituent(s), (3) heterocyclic group which may have a substituent(s), (4) hydroxy which may have a substituent(s), (5) mercapto which may have a substituent(s), (6) amino which may have a substituent(s), (7) carbamoyl which may have a substituent(s), (8) sulfamoyl which may have a substituent(s), (9) carboxy, (10) alkoxycarbonyl (e.g., C1-6 alkoxycarbonyl such as methoxycarbonyl, ethoxycarbonyl, *tert*-butoxycarbonyl, *etc.*)*,* (11) sulfo, (12) sulfino, (13) phosphono, (14) nitro, (15) cyano, (16) amidino, (17) imino, (18) dihydroxyboryl (-B(OH)₂), (19) halogen (fluoro, chloro, bromo, iodo), (20) alkylsulfinyl (e.g., C1-6 alkylsulfinyl such as methylsulfinyl, ethylsulfinyl, *etc.*), (21) aromatic ring-sulfinyl (e.g., C6-10 aromatic ring-sulfinyl such as phenylsulfnyl, etc.), (22) alkylsulfonyl (e.g., C1-6 alkylsulfonyl such as methylsulfonyl, ethylsulfonyl, *etc.*)*,* (23) aromatic ring-sulfonyl (e.g., C6-10 aromatic ring-sulfonyl such as phenylsulfonyl, *etc.*), (24) (C1-6 alkoxyimino)methyl (e.g., (methoxyimino)methyl, *etc.),* (25) acyl, (26) formyl, (27) alkyl substituted by hydroxy which may have a substituent(s), (28) alkyl substituted by mercapto which may have a substituent(s), (29) alkyl substituted by amino which may have a substituent(s), (30) (alkyl which may have a substituent(s))oxycarbonyl, (31) HO-(CO-amino acid residue-NH)_{q}-CO-T⁰- which may have a substituent(s), (32) H-(NH-amino acid residue-CO)_{q}-O-T⁰- which may have a substituent(s), *etc*.

R¹, R² and R³ are each independently the same or different.

The substituent represented by R includes, for example, (1) hydrocarbon group which may have a substituent(s), (2) carbocyclic group which may have a substituent(s), (3) heterocyclic group which may have a substituent(s), (4) hydroxy which may have a substituent(s), (5) mercapto which may have a substituent(s), (6) amino which may have a substituent(s), (7) carbamoyl which may have a substituent(s), (8) sulfamoyl which may have a substituent(s), (9) carboxy, (10) alkoxycarbonyl (e.g., C1-6 alkoxycarbonyl such as methoxycarbonyl, ethoxycarbonyl, *tert*-butoxycarbonyl, *etc.),* (11) sulfo, (12) sulfino, (13) phosphono, (14) nitro, (15) cyano, (16) amidino, (17) imino, (18) dihydroxyboryl (-B(OH)₂), (19) halogen (fluoro, chloro, bromo, iodo), (20) alkylsulfinyl (e.g., C1-6 alkylsulfinyl such as methylsulfinyl, ethylsulfinyl, *etc.), (21)* aromatic ring-sulfinyl (e.g., C6-10 aromatic ring-sulfinyl such as phenylsulfinyl, *etc.),* (22) alkylsulfonyl (e.g., C1-6 alkylsulfonyl such as methylsulfonyl, ethylsulfonyl, *etc.),* (23) aromatic ring-sulfonyl (e.g., C6-10 aromatic ring-sulfonyl such as phenylsulfonyl, *etc.),* (24) (C1-6 alkoxyimino)methyl (e.g., (methoxyimino)methyl, *etc.),* (25) acyl, (26) formyl, (27) alkyl substituted by hydroxy which may have a substituent(s), (28) alkyl substituted by mercapto which may have a substituent(s), (29) alkyl substituted by amino which may have a substituent(s), (30) (alkyl which may have a substituent(s))oxycarbonyl, (31) HO-(CO-amino acid residue-NH)q-CO-T°- which may have a substituent(s), (32) H-(NH-amino acid residue-CO)_{q}-O-T⁰- which may have a substituent(s), (33) oxo, (34) thioxo, *etc.*

A hydrocarbon group in "(1) hydrocarbon group which may have a substituent(s)" includes, for example, alkyl, alkenyl, alkynyl, alkylidene, alkenylidene, *etc.*

The alkyl group includes, for example, straight chain or branched C1-8 alkyl such as methyl, ethyl, propyl, isopropyl, butyl, isobutyl, *tert*-butyl, pentyl, neopentyl, hexyl, heptyl, octyl, *etc.*

The alkenyl group includes, for example, straight chain or branched C2-8 alkenyl such as ethenyl, propenyl, butenyl, pentenyl, hexenyl, heptenyl, octenyl, *etc*.

The alkynyl group includes, for example, straight chain or branched C2-8 alkynyl such as ethynyl, propynyl, butynyl, pentynyl, hexynyl heptynyl, octynyl, *etc*.

The alkylidene group includes, for example, straight chain or branched C1-8 alkylidene such as methylidene, ethylidene, propylidene, butylidene, pentylidene, hexylidene, heptylidene, octylidene, *etc*.

The alkenylidene includes, for example, straight chain or branched C2-8 alkenylidene such as ethenylidene, propenylidene, butenylidene, pentenylidene, hexenylide, heptenylidene, octenylidene, *etc.*

Herein, the substituent in "(1) hydrocarbon group which may have a substituent(s)" includes, for example, hydroxy, mercapto, amino, carboxy, nitro, cyano, mono- or di-C1-6 alkyl-substituted amino (e.g., methylamino, ethylamino, propylamino, dimethylamino, diethylamino, *etc.,* herein, the C1-6 alkyl may be substituted with C1-6 alkoxy such as methoxy, ethoxy, *etc*.), N-aromatic ring amino (e.g., N-phenylamino, *etc*.), N-aromatic ring-N-alkylamino (e.g., N-phenyl-N-methylamino, N-phenyl-N-ehtylamino, N-phenyl-N-propylamino, N-phenyl-N-butylamino, N-phenyl-N-pentylamino, N-phenyl-N-hexylamino, *etc*.), acylamino (e.g., acetylamino, *etc*.), N-acyl-N-alkylamino (e.g., N-acetyl-N-methylamino, *etc*.), N-alkyloxycarbonyl-N-alkylamino (e.g., N-tert-butoxycrbonyl-N-methylamino, *etc.), etc.),* C1-6 alkoxy (e.g., methoxy, ethoxy, propoxy, isopropoxy, hexyloxy, *etc*.), C3-7 cycloalkyl-C1-6 alkoxy (e.g., cyclohexylmethyloxy, cyclopentylethyloxy, *etc.),* C3-7 cycloalkyloxy (e.g., cyclohexyloxy, *etc.),* C7-15 aralkyloxy (e.g., benzyloxy, phenethyloxy, phenylpropyloxy, naphthylmethyloxy, naphthylethyloxy, *etc*.), phenoxy, C1-6 alkoxycarbonyl (e.g., methoxycarbonyl, ethoxycarbonyl, *tert*-butoxycarbonyl, *etc.),* C1-6 alkylcarbonyloxy (e.g., acetoxy, ethylcarbonyloxy, *etc.),* C1-6 alkylthio (e.g., methylthio, ethylthio, propylthio, butylthio, *etc.),* halogen (fluoro, chloro, bromo, iodo), alkylsulfonyl (e.g., C1-4 alkylsulfonyl such as methylsulfonyl, ethylsulfonyl, *etc.),* aromatic ring-sulfonyl (e.g., C6-10 aromatic ring-sulfonyl such as phenylsulfonyl, *etc.*), carbamoyl which may have a substituent(s) (e.g., non-substituted carbamoyl, N-mono-(C1-8 alkyl)carbamoyl (N-methylcarbamoyl, N-ethylcarbamoyl, N-propylcarbamoyl, N-isopropylcarbamoyl, N-butylcarbamoyl, *etc*.), N,N-di(C1-8 alkyl)carbamoyl (e.g., N,N-dimethylcarbamoyl, N,N-diethylcarbamoyl, N,N-dipropylcarbamoyl, N,N-dibutylcarbamoyl, *etc*.), piperidin-1-ylcarbonyl, *etc.),* acyl, carbocyclic group which may have a substituent(s), heterocyclic group which may have a substituent(s), *etc.* The 1 to 4 substituent(s) may exist wherever possible. Alkyl in the N-acyl-N-alkylamino and N-alkyloxycarbonyl-N-alkylamino includes, for example, straight chain or branched C1-6 alkyl such as methyl, ethyl, propyl, isopropyl, butyl, isobutyl, *sec-*butyl, *tert*-butyl, pentyl, hexyl, *etc.* Acyl, acyl in the acylamino and N-acyl-N-alkylamino includes, for example, C1-8 alkylcarbonyl such as formyl, acetyl, propanoyl, butanoyl, pentanoyl, hexanoyl, heptanoy, octanoyl, pivaloyl, *etc.,* C6-10 aromatic ring-carbonyl such as benzoyl, phenylmethylcarbonyl, 2-phenylethylcarbonyl, *etc..* Carbocyclic group which may have a substituent(s), heterocyclic group which may have a substituent(s) have the same meanings as the below-described "(2) carbocyclic group which may have a substituent(s)", and "(3) heterocyclic group which may have a substituent(s)", respectively.

The carbocyclic group in the "(2) carbocyclic group which may have a substituent(s)" has the same meaning as the above-described carbocyclic group represented by ring A. Herein, substituent in the "(2) carbocyclic group which may have a substituent(s)" includes, for example, straight chain or branched C1-6 alkyl (e.g., methyl, ethyl, propyl, isopropyl, butyl, isobutyl, *sec*-butyl, *tert*-butyl, pentyl, isopentyl, neopentyl, hexyl, isohexyl, *etc.),* straight chain or branched C2-6 alkenyl (e.g., ethenyl, propenyl, butenyl, pentenyl, hexenyl, *etc.),* or straight chain or branched C2-6 alkynyl (e.g., ethynyl, propynyl, butynyl, pentynyl, hexynyl, *etc*.) which may be substituted by hydroxy, C1-6 alkoxy (e.g., methoxy, ethoxy, propoxy, isopropoxy, hexyloxy, *etc*.), carbocyclic group (which has the same meaning as the above-described carbocyclic group represented by ring A.), or heterocyclic group (which has the same meaning as the above-described heterocyclic group represented by ring A), hydroxy, straight chain or branched C1-6 alkoxy (e.g., methoxy, ethoxy, propoxy, isopropoxy, butoxy, isobutyloxy, *tert-*butoxy, pentyloxy, isopentyloxy, neopentyloxy, hexyloxy, isohexyloxy, *etc.*)*,* mercapto, straight chain or branched C1-6 alkylthio (e.g., methylthio, ethylthio, propylthio, isopropylthio, butylthio, isobutylthio, *tert*-butylthio, pentylthio, isopentylthio, neopentylthio, hexylthio, isohexylthio, *etc*.), amino, mono- or di-C1-6 alkylamino (e.g., methylamino, ethylamino, propylamino, isopropylamino, butylamino, isobutylamino, *tert*-butylamino, pentylamino, isopentylamino, neopentylamino, hexylamino, dimethylamino, diethylamino, dipropylamino, N-methyl-N-ethylamino, *etc.),* halogen (fluoro, chloro, bromo, iodo), cyano, nitro, carboxy, straight chain or branched C1-6 alkoxycarbonyl (e.g., methoxycarbonyl, ethoxycarbonyl, *tert*-butoxycarbonyl, isopropyloxycarbonyl, butoxycarbonyl, isobutyloxycarbonyl, *tert*-butoxycarbonyl, *etc.),* trihalomethyl (e.g., trifluoromethyl, trichloromethyl, *etc.),* trihalomethoxy (e.g., trifluoromethoxy, trichloromethyloxy, *etc.),* trihalomethylthio (trifluoromethylthio, trichloromethylthio, *etc.),* dihalomethylthio (difluoromethylthio, dichloromethylthio, *etc*.), oxo, carbocyclic group (which has the same meaning as the above-described carbocyclic group represented by ring A.), heterocyclic group (which has the same meaning as the above-described heterocyclic group represented by ring A), *etc.* And the 1 to 4 substituent(s) may exist wherever possible.

Heterocyclic group in the "(3) heterocyclic group which may have a substituent(s)" has the same meaning as the above-described heterocyclic group represented by ring A. Herein, substituent in the heterocyclic group has the same meaning as the above-described substituent(s) in the "(2) carbocyclic group which may have a substituent(s)".

Substituent in the "(4) hydroxy which may have a substituent(s)", "(5) mercapto which may have a substituent(s)", "(6) amino which may have a substituent(s)", "(31) HO-(CO-amino acid residue-NH)_{q}-CO-T⁰- which may have a substituent(s)", and "(32) H-(NH-amino acid residue-CO)_{q} O-T⁰- which may have a substituent(s)" includes, for example, hydrocarbon group which may have a substituent(s) (which has the same meaning as the above-described "(1) hydrocarbon group which may have a substituent(s)"), carbocyclic group which may have a substituent(s) (which has the same meaning as the above-described "(2) carbocyclic group which may have a substituent(s)"), heterocyclic group which may have a substituent(s) (which has the same meaning as the above-described "(3) heterocyclic group which may have a substituent(s)"), alkylsulfonyl (e.g., C1-4 alkylsulfonyl such as methylsulfonyl, ethylsulfonyl, *etc*.), aromatic ring-sulfonyl (e.g., C6-10 aromatic ring-sulfonyl such as phenylsulfonyl, *etc*.), acyl (which has the same meaning as the below-described "(25) acyl"), (alkyl which may have a substituent(s))oxycarbonyl (which has the same meaning as the below-described "(30) (alkyl which may have a substituent(s))oxycarbonyl"), *etc.*

"(7) Carbamoyl which may have a substituent(s)" includes, for example, non-substituted carbamoyl, N-mono-Cl-6 alkylcarbamoyl (e.g., N-methylcarbamoyl, N-ethylcarbamoyl, N-propylcarbamoyl, N-isopropylcarbamoyl, N-butylcarbamoyl, *etc.),* N,N-di(C1-6 alkyl)carbamoyl (e.g., N,N-dimethylcarbamoyl, N,N-diethylcarbamoyl, N,N-dipropylcarbamoyl, N,N-dibutylcarbamoyl, N-ethyl-N-methylcarbamoyl, *etc.*), piperidin-1-ylcarbonyl, morpholin-4-ylcarbonyl, *etc.*

"(8) Sulfamoyl which may have a substituent(s)" includes, for example, non-substituted sulfamoyl, N-mono-C1-6 alkylsulfamoyl (e.g., N-methylsulfamoyl, N-ethylsulfamoyl, N-propylsulfamoyl, N-isopropylsulfamoyl, N-butylsulfamoyl, *etc*.), N,N-di(C1-6 alkyl)sulfamoyl (e.g., N,N-dimethylsulfamoyl, N,N-diethylsulfamoyl, N,N-dipropylsulfamoyl, N,N-dibutylsulfamoyl, N-ethyl-N-methylsulfamoyl, *etc.), etc.*

"(25) Acyl" includes, for example, alkylcarbonyl which may have a substituent(s) (wherein alkyl which may have a substituent(s) has the same meaning as the above-described "alkyl which may have a substituent(s)" in the "(1) hydrocarbon group which may have a substituent(s)"), alkenylcarbonyl which may have a substituent(s) (wherein alkenyl which may have a substituent(s) has the same meaning as the above-described "alkenyl which may have a substituent(s)" in the "(1) hydrocarbon group which may have a substituent(s)"), alkynylcarbonyl which may have a substituent(s) (wherein alkynyl which may has a substituent(s) have the same meaning as the above-described "alkynyl which may have a substituent(s)" in the "(1) hydrocarbon group which may have a substituent(s)"), (carbocyclic group which may have a substituent(s))carbonyl (wherein carbocyclic group which may have a substituent(s) has the same meaning as the above-described "(2) carbocyclic group which may have a substituent(s)"), (heterocyclic group which may have a substituent(s))carbonyl (wherein heterocyclic group which may have a substituent(s) has the same meaning as the above-described "(3) heterocyclic group which may have a substituent(s)", alkylsulfonyl which may have a substituent(s) (wherein alkyl which may have a substituent(s) has the same meaning as the above-described "alkyl which may have a substituent(s)" in the "(1) hydrocarbon group which may have a substituent(s)"), alkenylsulfonyl which may have a substituent(s) (wherein alkenyl which may have a substituent(s) has the same meaning as the above-described "alkenyl which may have a substituent(s)" in the "(1) hydrocarbon group which may have a substituent(s)"), alkynylsulfonyl which may have a substituent(s) (wherein alkynyl which may has a substituent(s) have the same meaning as the above-described "alkynyl which may have a substituent(s)" in the "(1) hydrocarbon group which may have a substituent(s)"), (carbocyclic group which may have a substituent(s))sulfonyl (wherein carbocyclic group which may have a substituent(s) has the same meaning as the above-described "(2) carbocyclic group which may have a substituent(s)"), (heterocyclic group which may have a substituent(s))sulfonyl (wherein heterocyclic group which may have a substituent(s) has the same meaning as the above-described "(3) heterocyclic group which may have a substituent(s)", *etc*.

Hydroxy which may have a substituent(s) in the "(27) alkyl substituted by hydroxy which may have a substituent(s)" has the same meaning as the above-described "(4) hydroxy which may have a substituent(s)", mercapto which may have a substituent(s) in the "(28) alkyl substituted by mercapto which may have a substituent(s)" has the same meaning as the above-described "(5) mercapto which may have a substituent(s)", amino which may have a substituent(s) in the "(29) alkyl substituted by amino which may have a substituent(s)" has the same meaning as the above-described "(6) amino which may have a substituent(s)". Alkyl in the "(27) alkyl substituted by hydroxy which may have a substituent(s)", "(28) alkyl substituted by mercapto which may have a substituent(s)" and "(29) alkyl substituted by amino which may have a substituent(s) includes, for example, straight chain or branched C1-6 alkyl such as methyl, ethyl, propyl, isopropyl, butyl, isobutyl, sec-butyl, *tert*-butyl, pentyl, isopentyl, neopentyl, hexyl, isohexyl, *etc.*

Alkyl which may have a substituent(s) in the "(30) (alkyl which may have a substituent (s)) oxycarbonyl" has the same meaning as the above-described "alkyl which may have a substituent(s)" in the "(1) hydrocarbon group which may have a substituent(s)" .

"q" in the "(31) HO-(CO-amino acid residue-NH)_{q}-CO-T⁰- which may have a substituent(s)", and "(32) H-(NH-amino acid residue-CO)_{q}-O-T⁰- which may have a substituent(s)" is an integer of 1 to 3. "Amino acid" means natural amino acid or unusual amino acid, and includes, for example, glycine, alanine, valine, leucine, isoleucine, serine, threonine, cystein, methionine, proline, asparagine, glutamine, phenylalanine, tyrosine, tryotophan, aspartic acid, glutamic acid, lysine, arginine, histidine, β-alanine, cystathionine, cystine, homoserine, isoleucine, lanthionine, norleucine, norvaline, ornithine, sarcosine, thyronine, *etc.* T⁰ in the group has the same meaning as the below-described T¹, T² and T³.

Also, the substituent represented by R, R¹, R² and R³ includes the following group:

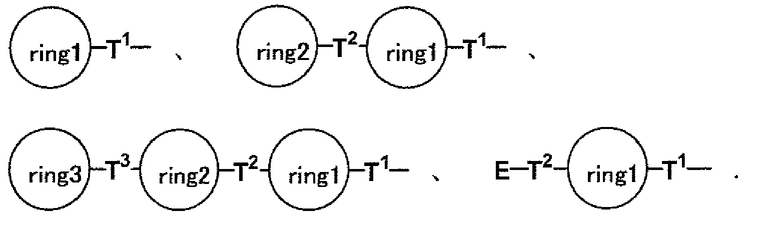

The nitrogen-containing substituent includes, for example, amino which may have a substituent(s) or a nitrogen-containing heterocyclic group which may have a substituent(s). The "amino which may have a substituent(s)" has the same meaning as the above-described "(6) amino which may have a substituent(s)". The "nitrogen-containing heterocyclic group" has the same meaning as the above-described "nitrogen-containing heterocyclic group" represented by ring B. The "nitrogen-containing heterocyclic group" may have 1 to 5 substituents. The substituent(s) in the nitrogen-containing heterocyclic group" has the same meaning as the above-described substituent(s) in the "(2) carbocyclic group which may have a substituent(s)". When the nitrogen-containing heterocyclic group has plural substituents, the substituents are the same or different.

T¹, T² and T³ is each independently a bond or a spacer having from 1 to 3 atoms of the principle chain, and ring 1, ring 2 and ring 3 is each independently a cyclic group which may have a substituent(s).

A spacer having from 1 to 3 atoms of the principle chain represented by T¹, T² and T³ means a space formed by 1 to 3 continued atoms of a main chain. In this case, the "number of atoms as a principle chain" should be counted such that atoms as a main chain become minimum. The "spacer having from 1 to 3 atoms of the principle chain" includes, for example, a bivalent group formed by 1 to 3 continued atoms of a main chain comprising 1 to 3 selected from -O-, -S-, -S(O)-, -SO₂-, -CO-, a nitrogen atom which may have a substituent(s), and a bivalent C1-3 aliphatic hydrocarbon group which may have a substituent(s), etc. "A nitrogen atom which may have a substituent(s)" includes, an -NH- in which hydrogen atom is replaced by hydrocarbon group which may have a substituent(s) (which has the same meaning as the above-described "(1) hydrocarbon group which may have a substituent(s)") besides -NH- and =N-.

"A divalent C1-3 aliphatic hydrocarbon group" in the "a divalent C1-3 aliphatic hydrocarbon group which may have a substituent(s)" includes, for example, C1-3 alkylene (e.g., -CH₂-, -(CH₂)₂-, -(CH₂)₃-, etc,), C2-3 alkenylene (e.g., -CH=CH-, - CH₂-CH=CH-, -CH=CH-CH₂-, *etc.),* C2-3 alkynylene (e.g., -C≡C-, -CH₂-C≡C-, -C≡C-CH₂-, *etc.).* "Substituent" in "a divalent C1-3 aliphatic hydrocarbon group which may have a substituent(s)" has the same meaning as the above-described " substituent" in "(1) hydrocarbon group which may have a substituent(s)". When the substituents is plural, these substituents may be taken together with carbon atom(s) which they bind to form carbocyclic group or heterocyclic group. The "carbocyclic group" and the "heterocyclic group" have the same meanings as the "carbocyclic group" and the "heterocyclic group", respectively, described above in ring A. The "carbocyclic group" is preferably cyclopropane, cyclobutane, cyclopentane, cyclohexane, *etc.,* and the "heterocyclic group" is preferably piperidine, etc.

The cyclic ring in the "cyclic group which may have a substituent(s)" represented by ring 1, ring 2 and ring 3 includes, for example, a carbocyclic group, a heterocyclic group, etc. This carbocyclic group and heterocyclic group have the same meanings as the above-described carbocyclic group and heterocyclic group represented by ring A.

Substituent in the "cyclic group which may have a substituent(s)" represented by ring 1, ring 2 and ring 3 includes, for example, hydrocarbon group which may have a substituent(s) (which has the same meaning as above-described "(1) hydrocarbon group which may have a substituent(s)"), carbocyclic group which may have a substituent(s) (which has the same meaning as the above-described "(2) carbocyclic group which may have a substituent(s)"), heterocyclic group which may have a substituent(s) (which has the same meaning as the above-described "(3) heterocyclic group which may have a substituent(s)"), hydroxy which may have a substituent(s) (which has the same meaning as the above-described "(4) hydroxy which may have a substituent(s)"), mercapto which may have a substituent(s) (which has the same meaning as the above-described "(5) mercapto which may have a substituent(s)"), amino which may have a substituent(s) (which has the same meaning as the above-described "(6) amino which may have a substituent(s)"), carbamoyl which may have a substituent(s) (which has the same meaning as the above-described "(7) carbamoyl which may have a substituent(s)"), sulfamoyl which may have a substituent(s) (which has the same meaning as the above-described "(8) sulfamoyl which may have a substituent(s)"), carboxy, alkoxycarbonyl (e.g., C1-6 alkoxycarbonyl such as methoxycarbonyl, ethoxycarbonyl, *tert*-butoxycarbonyl, *etc.),* sulfo, sulfino, phosphono, nitro, cyano, amidino, imino, dihydroboryl (-B(OH)₂), halogen (fluoro, chloro, bromo, iodo), alkylsulfinyl (e.g., C1-6 alkylsulfinyl such as methylsulfinyl, ethylsulfinyl, *etc.),* aromatic ring-sulfinyl (e.g., C6-10 aromatic ring-sulfinyl such as phenylsulfinyl, *etc.),* alkylsulfonyl (e.g., C1-8 alkylsulfonyl such as methylsulfonyl, ethylsulfonyl, *etc.),* aromatic ring-sulfonyl (e.g., C6-10 aromatic ring-sulfonyl such as phenylsulfonyl, etc.), oxo, thioxo, (C1-6 alkoxyimino)methyl (e.g., (methoxyimino)methyl, *etc.),* acyl (which has the same meaning as the above-described "(25) acyl"), formyl, alkyl substituted by hydroxy which may have a substituent(s) (which has the same meaning as the above-described "(27) alkyl substituted by hydroxy which may have a substituent(s)"), alkyl substituted by mercapto which may have a substituent(s) (which has the same meaning as the above-described "(28) alkyl substituted by mercapto which may have a substituent(s)"), alkyl substituted by amino which may have a substituent(s) (which has the same meaning as the above-described "(29) alkyl substituted by amino which may have a substituent(s)".), (alkyl which may have a substituent(s))oxycarbonyl (which has the same meaning as the above-described "(30) (alkyl which may have a substituent(s))oxycarbonyl"), and the 1 to 5 substituent(s) may exist wherever possible.

"A cyclic group which may have a substituent(s)" formed by two Rs together with an atom on ring X to which they bind has the same meaning as the above-described "a cyclic group which may have a substituent(s)" represented by ring 1, ring 2 and ring 3.

The "cyclic group" in the "cyclic group which may have a substituent(s)" formed by R¹ and R² together with a nitrogen atom to which they bind includes, for example, a monocyclic or polycyclic aromatic heterocyclic group which may be partially or fully saturated, contains two adjoining nitrogen atoms, and may contain 1 to 4 hetero atoms selected from a nitrogen atom(s), an oxygen atom(s) and a sulfur atom(s) which may be oxidized, *etc.*

The "monocyclic or polycyclic aromatic heterocyclic group which may be partially or fully saturated, contains two adjoining nitrogen atoms, and may contain 1 to 4 hetero atoms selected from a nitrogen atom(s), an oxygen atom(s) and a sulfur atom(s) which may be oxidized" includes, for example, triazoline, triazolidine, tetraazoline, tetrazolidine, dihyrdopyrazole (pyrazoline), tetrahydropyrazole (pyrazolidine), dihyrdopyridazine, tetrahydropyridazine, perhydropyridazine, dihydrotriazine, tetrahydrotriazine, perhydrotriazine, dihyrdooxadiazole, tetrahydrooxadiazole (oxadiazolidine), dihyrdooxadiadine, tetrahydrooxadiadine, dihyrdooxadiazepine, tetrahydrooxadiazepine, perhydrooxadiazepine, dihyrdothiadiazole, tetrahydrothiadiazole (thiadiazolidine), dihyrdothiadiadine, tetrahydrothiadiadine, dihyrdothiadiazepine, tetrahydrothiadiazepine, perhydrothiadiazepine, dihyrdoindazole, perhydroindazole, dihyrdophthalazine, tetrahydrophthalazine, perhydrophthalazine, tetrahydropyrrolopyridine, dihyrdocinnoline, tetrahydrocinnoline, perhydrocinnoline rings, *etc.* The cyclic group may have 1 to 5 substituent(s) at substitutable positions, and the substituent(s) have the same meanings as the substituent(s) in the "cyclic group which may have a substituent (s)" represented by ring 1, ring 2 and ring 3.

The cyclic group in the "cyclic group which may have a substituent(s)" formed by R² and R³ together with a nitrogen atom to which they bind includes, for example, a monocyclic or polycyclic aromatic heterocyclic group which may be partially or fully saturated, contains one nitrogen atom and may further contain 1 to 4 hetero atoms selected from a nitrogen atom(s), an oxygen atom(s) and a sulfur atom(s) which may be oxidized, *etc.*

The "monocyclic or polycyclic aromatic heterocyclic group which may be partially or fully saturated, contains one nitrogen atom and may further contain 1 to 4 hetero atoms selected from a nitrogen atom(s), an oxygen atom(s) and a sulfur atom(s) which may be oxidized" includes, for example, pyrrole, imidazole, triazole, tetrazole, pyrazole, azepine, diazepine, oxazine, oxadiazine, thiazine, thiadiazine, indole, isoindole, indazole, purine, pyrrolopyridine, benzazepine, benzodiazepine, benzotriazole, carbazole, β-carboline, phenothiazine, phenoxazine, perimidine, pyrazoloisoquinoline, pyrazolonaphthyridine, pyrroline, pyrrolidine, imidazoline, imidazolidine, triazoline, triazolidine, tetrazoline, tetrazolidine, dihydropyrazole (pyrazoline), tetrahydropyrazole (pyrazolidine), dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrotriazine, tetrahydrotriazine, perhydrotriazine, dihydroazepine, tetrahydroazepine, perhydroazepine, dihydrodiazepine, tetrahydrodiazepine, perhydrodiazepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, indoline, isoindoline, dihydroquinoline, tetrahydroquinoline, octahydroquinoline, perhydroquinoline, dihydroisoquinoline, tetrahydroisoquinoline, octahydroisoquinoline, perhydroisoquinoline, dihydrophthalazine, tetrahydrophthalazine, perhydrophthalazine, dihydronaphthyridine, dihydroquinoxaline, tetrahydroquinoxaline, perhydroquinoxaline, dihydroquinazoline, tetrahydroquinazoline, perhydroquinazoline, tetrahydropyrrolopyridine, dihydrocinnoline, tetrahydrocinnoline, perhydrocinnoline, dihydrobenzoxazine, dihydrobenzothiazine, pyrazinomorpholine, dihydrobenzoxazole, perhydrobenzoxazole, dihydrobenzothiazole, perhydrobenzothiazole, 4,5,6,7-tetrahydrothieno[3.2-c]pyridine, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, tetrahydrodibenzothiophene, tetrahydronaphthyridine, tetrahydro-β-carboline, dihydroazepinoindole, hexahydroazepinoindole, tetrahydropyrazoloisoquinoline, tetrahydropyrazolonaphthyridine, dihydroazepinoindazole, hexahydroazepinoindazole, dihydropyrazolopyridoazepine, hexahydropyrazolopyridoazepine, tetrahydropyrimidoindole, dihydrothiazinoindole, tetrahydrothiazinoindole, dihydrooxazinoindole, tetrahydrooxazinoindole, 2,3-dihydro-1H-benzo[de]isoquinoline, azaspiro[4.4]nonane, oxaazaspiro[4.4]nonane, oxaazaspiro[2.5]octane, dioxaspiro[4.4]nonane, azaspiro[4.5]decane, oxaazaspiro[4.5]decane, azaspiro[5.5]undecane, 1,4-dioxa-8-azaspiro[4.5]undecane, 1,3,8-triazaspiro[4.5]decane, azabicyclo[2.2.1]heptane, azabicyclo[3.1.1]heptane, azabicyclo[3.2.1]octane, azabicyclo[2.2.2]octane, diazabicyclo[2.2.2]octane, 2,5-diazabicyclo[2.2.1]heptane, 1,3,8-triazaspiro[4.5]decane, 2,7-diazaspiro[4.5]decane, 2,8-diazaspiro[4.5]decane, 1,4, 9-triazaspiro[5.5]undecane, 2,3,4,9-tetrahydrospiro[β-carboline-1,1'-cyclopentane], 3,9-diazaspiro[5.5]undecane rings, etc. The cyclic group may have 1 to 5 substituent(s) at substitutable positions, and the substituent(s) have the same meanings as the substituent(s) in the "cyclic group which may have a substituent(s)" represented by ring 1, ring 2 and ring 3.

Also, the group represented by 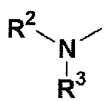 may represent 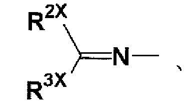 O=N- or S=N-(wherein R^{2X} and R^{3X} is each independently hydrogen atom, a hydrocarbon group which may have a substituent(s) (having the same meaning as the above-described "(1) hydrocarbon group which may have a substituent(s)), a carbocyclic group which may have a substituent(s) (having the same meaning as the above-described "(2) carbocyclic group which may have a substituent(s)) or a heterocyclic group which may have a substituent(s) (having the same meaning as the above-described "(3) heterocyclic group which may have a substituent(s)") by combining R² and R³, or R^{2X} and R^{3X}, together with a nitrogen atom to which they bind, may form a carbocyclic group which may have a substituent(s) (having the same meaning as the above-described "(2) carbocyclic group which may have a substituent(s) or a heterocyclic group which may have a substituent(s) (having the same meaning as the above-described "(3) heterocyclic group which may have a substituenl(s)")).

The "spacer having from 1 to 8 atoms of the principle chain" represented by J means a space formed by 1 to 8 continued atoms of a main chain. In this case, the "number of atoms as a principle chain" should be counted such that atoms as a main chain become minimum. The "spacer having from 1 to 8 atoms of the principle chain" includes, for example, a bivalent group formed by 1 to 8 continued atoms of a main chain comprising 1 to 8 selected from -O-, -S-, -S(O)-, -SO₂-, -CO-, a nitrogen atom which may have a substituent(s), and a bivalent C1-8 aliphatic hydrocarbon group which may have a substituent(s). Herein, the "nitrogen atom which may have a substituent(s)" is -NH-, =N- or one in which a hydrogen atom in "-NH-" is replaced with a hydrocarbon group which may have a substituent(s) (having the same meaning as the above-described (1) hydrocarbon group which may have a substituent(s)).

The "bivalent C1-8 aliphatic hydrocarbon group" in the "bivalent C1-8 aliphatic hydrocarbon group which may have a substituent(s)" includes, for example, C1-8 alkylene (e.g., -CH₂-, -(CH₂)₂-, -(CH₂)₃-, -(CH₂)₈-, *etc.),* C2-8 alkenylene (e.g., -CH=CH-, -CH₂-CH=CH-, -CH=CH-CH₂-, -CH=CH-(CH₂)₆-, *etc.),* C2-8 alkynylene (e.g., -C≡C-, -CH₂-C≡C-, -C≡C-CH₂-, -CH≡CH-(CH₂)₆-, *etc.*), and the like. Also, the "substituent(s)" in the "bivalent C1-8 aliphatic hydrocarbon group which may have a substituent(s)" has the same meaning as the substituent(s) in the above-described "(1) hydrocarbon group which may have a substituent(s)"

The monocyclic group represented by ring X is preferably a 5- to 7-membered monocyclic aromatic heterocyclic group which may be partially or fully saturated, contains one nitrogen atom and may further contain 1 to 3 hetero atoms selected from a nitrogen atom(s), an oxygen atom(s) and a sulfur atom(s) which may be oxidized.

Specifically, preferred as 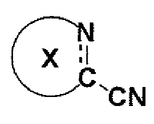 is 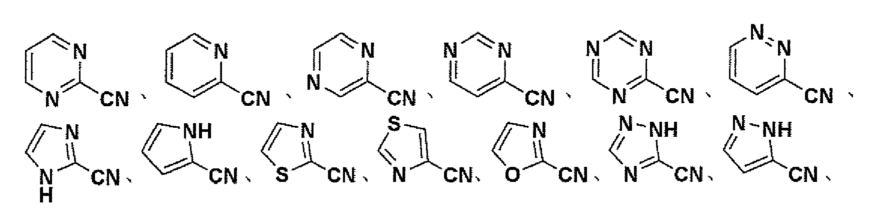 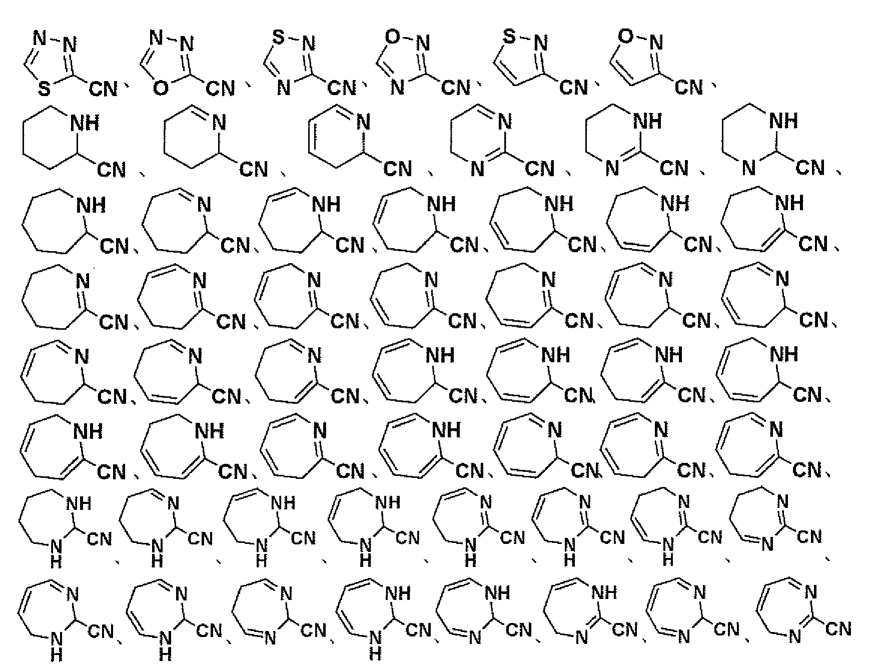 (wherein a hydrogen atom bound to a nitrogen atom may be replaced with J or R).

The carbocyclic group represented by ring A is preferably a C3-10 monocyclic or bicyclic aromatic carbocyclic group which may be partially or fully saturated, more preferably a C5-7 monocyclic aromatic carbocyclic ring which may be partially or fully saturated, and most preferably cyclopropane, cyclobutane, cyclopentane, cyclohexane, cycloheptane, cyclopentene, cyclohexene, cycloheptene, cyclopentadiene, cyclohexadiene, cycloheptadiene or benzene ring.

The heterocyclic group represented by ring A is preferably a 3- to 10-membered monocyclic or bicyclic aromatic heterocyclic group which may be partially or fully saturated and contains 1 to 3 hetero atoms selected from an oxygen atom(s), a nitrogen atom(s) and a sulfur atom(s) which may be oxidized, more preferably a 5- to 7-membered monocyclic aromatic heterocyclic group which may be partially or fully saturated and contains an oxygen atom(s), a nitrogen atom(s) and a sulfur atom(s) which may be oxidized, and most preferably pyridine, pyrimidine, pyrazine, tetrahydropyrimidine, dihyrdopyridazine, pyridazine, dihydropyrimidine, dihydropyrazine, dihydrotriazine, pyrazole, dihydropyrazole, pyrrole, imidazole, triazole, thiophene or furan ring.

Preferred as 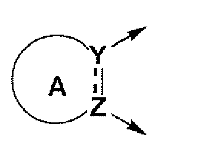 (wherein the rightward arrow represents a binding position to ring B) is 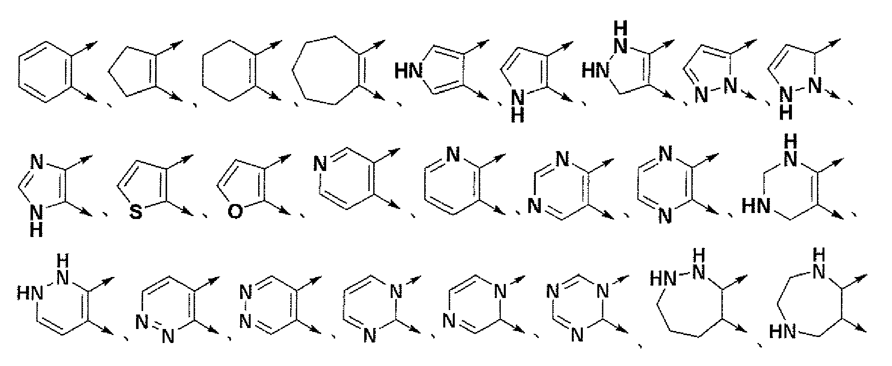 (wherein a hydrogen atom bound to a nitrogen atom may be replaced with J or R).

The nitrogen-containing heterocyclic group represented by ring B is preferable a 3- to 10-membered monocyclic or bicyclic aromatic heterocyclic group which may be partially or fully saturated, contains one nitrogen atom and further contains 1 or 2 hetero atoms selected from an oxygen atom(s), a nitrogen atom(s) and a sulfur atom(s) which may be oxidized, more preferably a 5- to 7-membered monocyclic aromatic heterocyclic group which may be partially or fully saturated, contains one nitrogen atom and further contains an oxygen atom(s), a nitrogen atom(s) and a sulfur atom(s) which may be oxidized, and most preferably pyrrole, imidazole, triazole, pyrazole, pyridine, pyrimidine, dihydrotriazine, pyrazine or dihyrdopyrimidine ring.

Preferred as 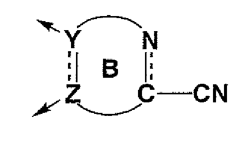 (wherein the leftward arrow represents a binding position to ring A) is 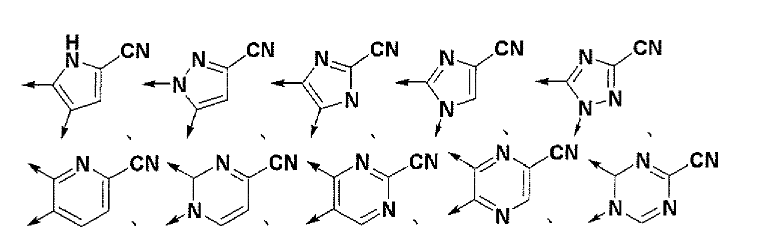 (wherein a hydrogen atom bound to a nitrogen atom may be replaced with J or R).

When ring X is a bicyclic heterocyclic group, a preferred ring is, for example, pyrazolo[1,5-a]pyrimidine, imidazole, 1,3-thiazole, pyrazolo[1,5-a]pyrimidine, pyrazolo[1,5-a][1,3,5]triazine, imidazo[1,2-a]pyrimidine, imidazo[1,5-a]pyrazine, pyrazolo[1,5-a]pyrimidine, 6,7-dihydro-5H-cyclopenta[d]pyrimidine, imidazo[1,5-a]pyrazine, thieno[3,2-d]pyrimidine, quinazoline, indole, isoindole, quinoxaline, indazole, quinolidine, quinoline, isoquinoline, phthalazine, naphthylidine, cinnoline or pteridine ring, *etc.*

Preferred as 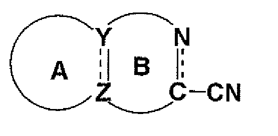 is concretely, for example, 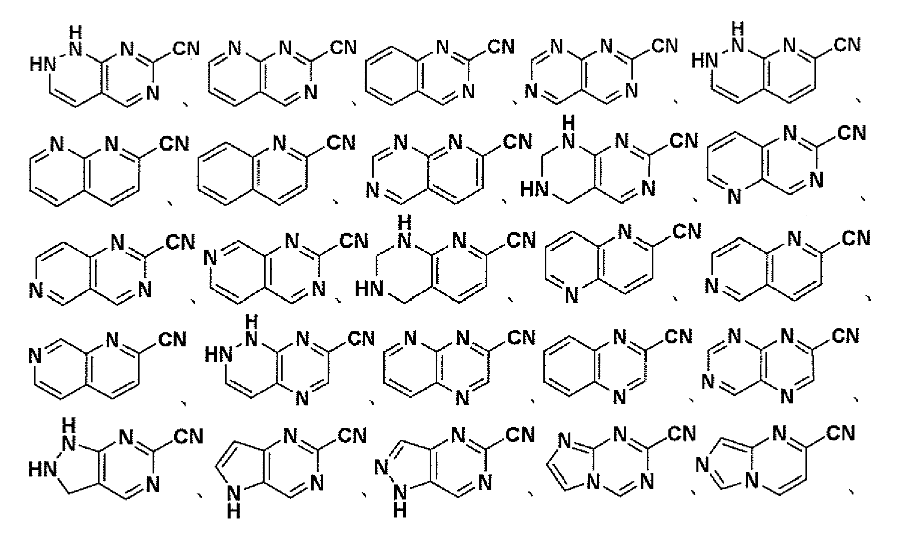 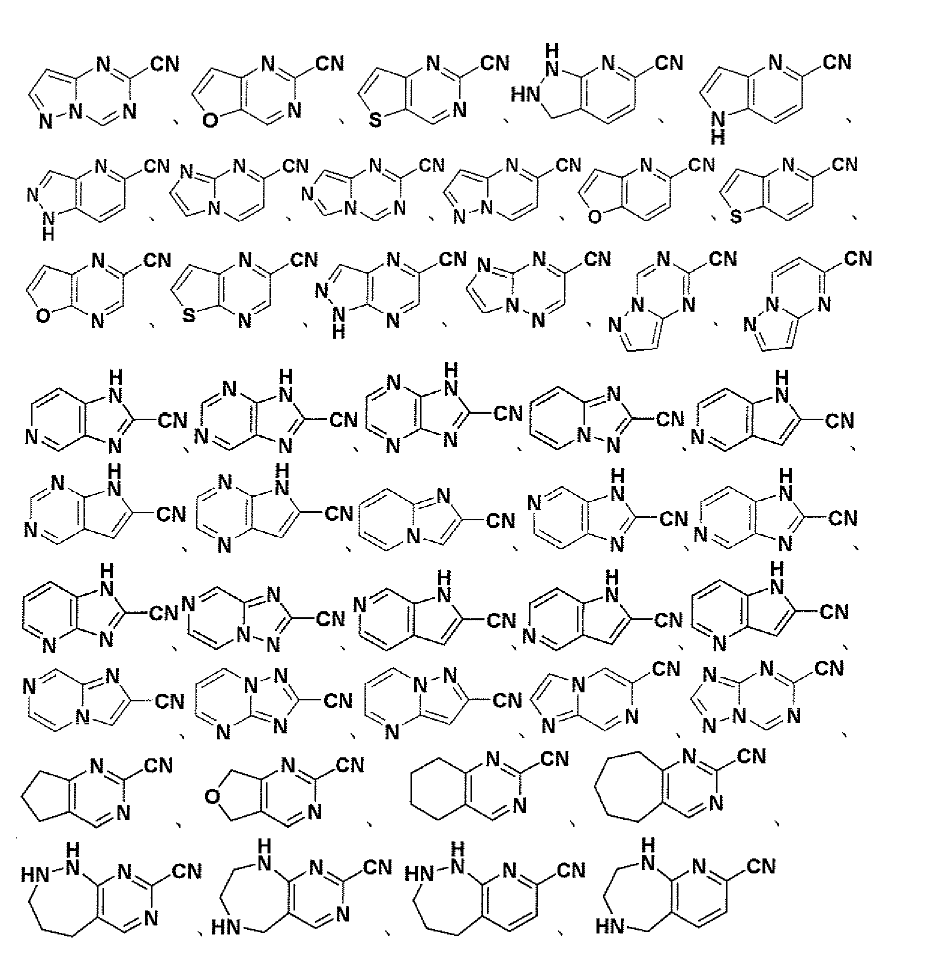 (wherein a hydrogen atom bound to a nitrogen atom may be replaced with J or R).

Also, other preferred embodiments as 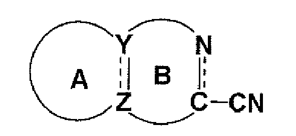 include, for example, 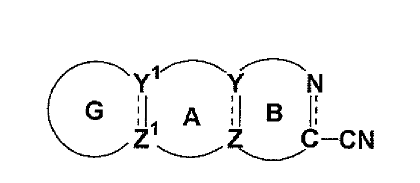 (wherein Y¹ and Z¹ is each independently a carbon atom or a nitrogen atom, ring G is a ring formed by two Rs), 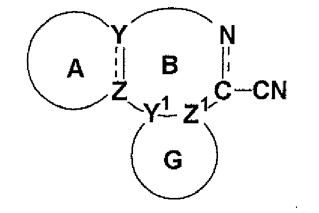 (wherein all symbols have the same meanings as described above), 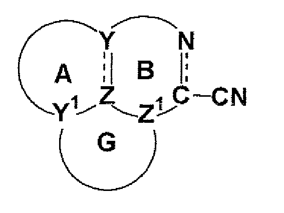 (wherein all symbols have the same meanings as described above), 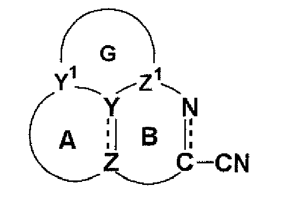 (wherein all symbols have the same meanings as described above), *etc*.

Preferred as 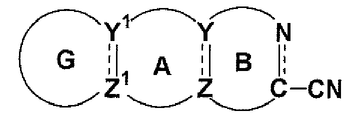 is concretely, for example, 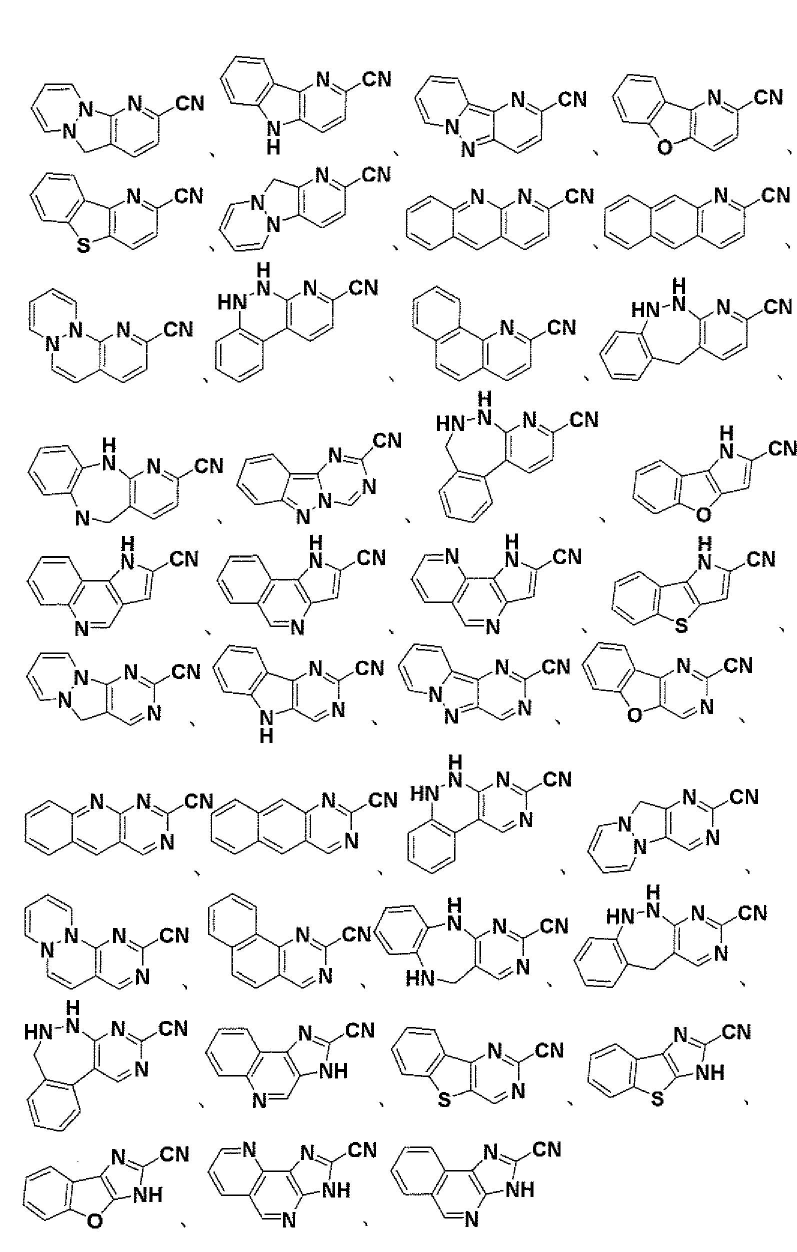 (wherein a hydrogen atom bound to a nitrogen atom may be replaced with J or R), *etc,*

Preferred as 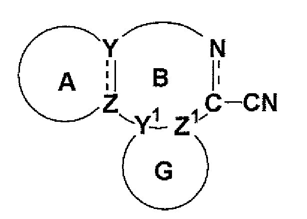 is concretely, for example, 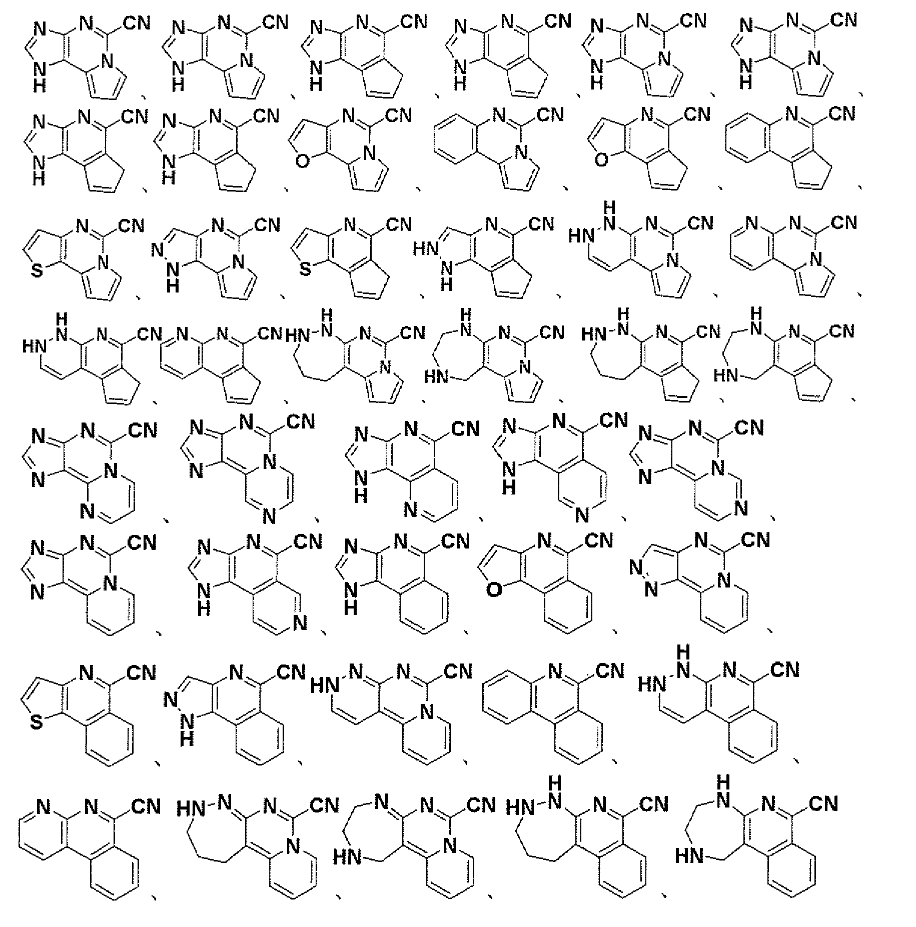 (wherein a hydrogen atom bound to a nitrogen atom may be replaced with J or R), *etc.*

Preferred as 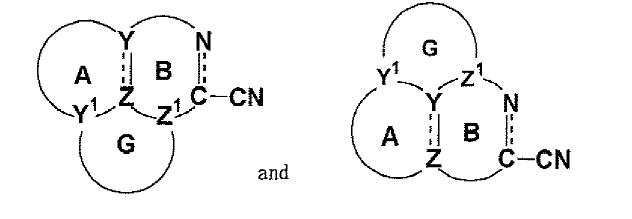 is concretely, for example, 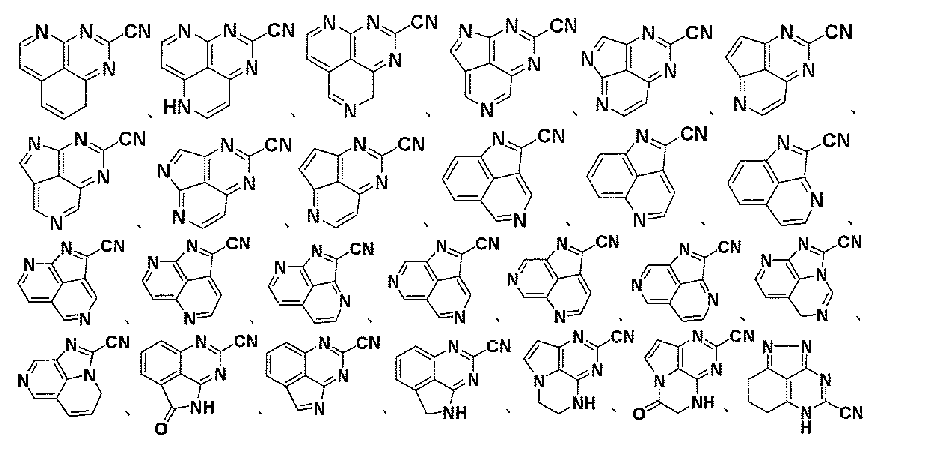 (wherein a hydrogen atom bound to a nitrogen atom may be replaced with J or R), *etc.*

Ring X is preferably, for example, a 5- to 7-membered monocyclic aromatic heterocyclic ring which may be partially or fully saturated, contains one nitrogen atom and may further contain 1 to 3 hetero atoms selected from a nitrogen atom(s), an oxygen atom(s) and a sulfur atom(s) which may be oxidized, *etc*., such as tetrahydropyrimidine, dihyrdopyridazine, pyridazine, dihydropyrimidine, dihydropyrazine, dihydrotriazine, dihydropyrazole, diazepane, pyrrole, pyrimidine, pyrazine, imidazole, pyrazole, triazole, tetrazole, thiazole, oxazole, thiadiazole, oxadiazole, azepane, azepine, dihydroazepine, tetrahydroazepine, diazepane, diazepine, dihydroazepine or tetrahydroazepine ring, and more preferably pyrimidine, pyrazine, pyridazine, thiazole or oxazole ring, *etc.*

R is preferably, for example, a hydrogen atom, C1-8 alkyl which may have a substituent(s), trifluoromethyl, hydroxy which may have a substituent(s) (e.g., hydroxy, C1-8 alkoxy which may have a substituent(s) (C1-8 alkoxy including, for example, methoxy, ethoxy, propoxy, butoxy, pentyloxy, hexyloxy, heptyloxy, octyloxy, *etc.*), C1-6 alkoxycarbonyl, cyano, a halogen atom, *etc.,* more preferably C1-6 alkyl which may have a substituent(s), trifluoromethyl, C1-6 alkoxy which may have a substituent(s), a halogen atom, *etc.,* and more preferably methyl, ethyl, propyl, butyl, isopropyl, isobutyl, neopentyl, methoxy, ethoxy, propoxy, butoxy, chloro, fluoro, bromo, trifluoromethyl, *etc*.

R¹ is preferably, for example, a hydrogen atom, C1-8 alkyl which may have a substituent(s), *etc.,* more preferably straight chain or branched C1-8 alkyl, *etc.,* and most preferably branched C3-8 alkyl such as isopropyl, isobutyl, sec-butyl, neopentyl and isopentyl, *etc*.

R² is preferably C1-8 alkyl which may have a substituent(s), C1-8 alkylcarbonyl which may have a substituent(s), C1-8 alkylsulfonyl which may have a substituent(s), carbamoyl which may have a substituent(s), a carbocyclic group which may have a substituent(s), a heterocyclic group which may have a substituent(s), 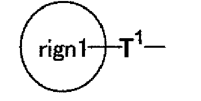 (wherein all symbols have the same meanings as described above), 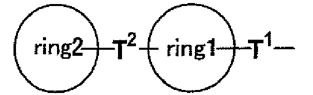 (wherein all symbols have the same meanings as described above), or 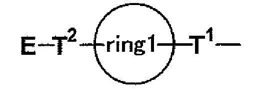 (wherein all symbols have the same meanings as described above), and R³ is preferably a hydrogen atom.

The ring represented by ring 1, ring 2 or ring 3 is preferably a C5-7 monocyclic aromatic carbocyclic group which may be partially or fully saturated, or a 5- to 7-membered monocyclic aromatic heterocyclic group which may be partially or fully saturated and contains 1 to 3 hetero atoms selected from an oxygen atom(s), a nitrogen atom(s) and a sulfur atom(s) which may be oxidized.

Ring 1 is more preferably, for example, a C5 or C6 monocyclic carbocyclic ring such as benzene, cyclohexane, cyclopentane, cyclohexene or cyclopentene ring, or a 5- or 6-membered monocyclic heterocyclic group such as pyridine, thiazole, pyrrolidine, imidazolidine, dihyrdopyridine, piperidine, piperazine, tetrahydropyran, tetrahydrothiazole (thiazolidine), morpholine or thiomorpholine ring, and most preferably benzene.

Ring 2 is more preferably, for example, a benzene ring or a 5- to 7-membered monocyclic aromatic heterocyclic ring which may be partially or fully saturated and contains 1 to 3 hetero atoms selected from an oxygen atom(s), a nitrogen atom(s) or a sulfur atom(s) which may be oxidized, and most preferably benzene, pyridine, pyrrolidine, imidazolidine, piperidine, piperazine, tetrahydropyran, morpholine or thiomorpholine ring.

T¹, T², T³ is preferably a bond or a spacer having 1 or 2 atoms of the principle chain of a combination of one or two selected from C1-3 alkylene (e.g., -CH₂-, -(CH₂)₂-, -(CH₂)₃-, *etc.)* which may have 1 or 2 substituent(s) (e.g., C1-4 alkyl such as methyl, ethyl, propyl and butyl, *etc.*), -O-, -SO₂-, -CO- and a nitrogen atom which may have a substituent(s) (specifically, e.g., -CO-, -CH₂-CO-, -C(CH₃)₂-CO-, -NH-CO-, - N(CH₃)-CO-, -CH₂-O-, *etc.).*

T¹ is preferably -SO₂-, -CO-, and more preferably -CO-.

T² is preferably a bond, C1-3 alkylene which may have a substituent(s) (e.g., C1-4 alkyl such as methyl, ethyl, propyl and butyl, *etc.* or -CH₂-O-, and more preferably -CH₂-.

E is preferably, for example, amino, amino which may have a substituent(s) (e.g., C1-4 alkyl, methoxyethyl, tetrahydropyranyl, *etc*.) such as dimethylamino, di(methoxyethyl)amino, N-methoxyethyl-N-methylamino and N-methyl-N-(tetrahydro-2H-pyran-4-yl)amino, or a 5- or 6-membered nitrogen-containing heterocyclic group (e.g., pyrrolidine, pyridine, piperidine, piperazine, morpholine ring, *etc*.) which may have a substituent(s) (e.g., C1-4 alkyl, methoxyethyl, tetrahydropyranyl, *etc.*).

Among the groups represented by 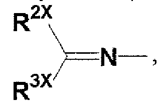 when R^{2X} and R^{3X} forms a ring together with a nitrogen atom to which they bind, 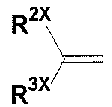 is preferably 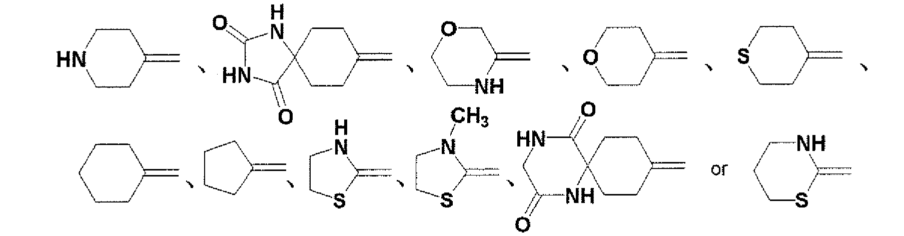 (wherein a hydrogen atom bound to a nitrogen atom may be replaced with J or R).

Among the groups represented by 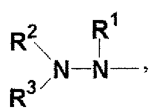 when R¹ and R² form a cyclic group together with a nitrogen atom to which they bind, that is, 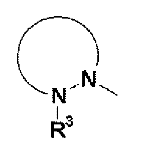 is preferably

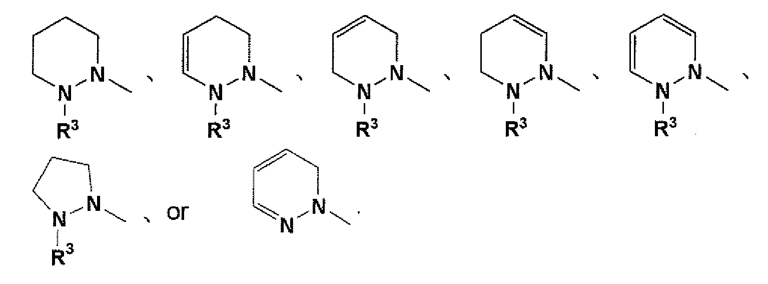

The cyclic group formed by R² and R³ together with a nitrogen atom to which they bind, that is, 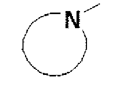 is preferably 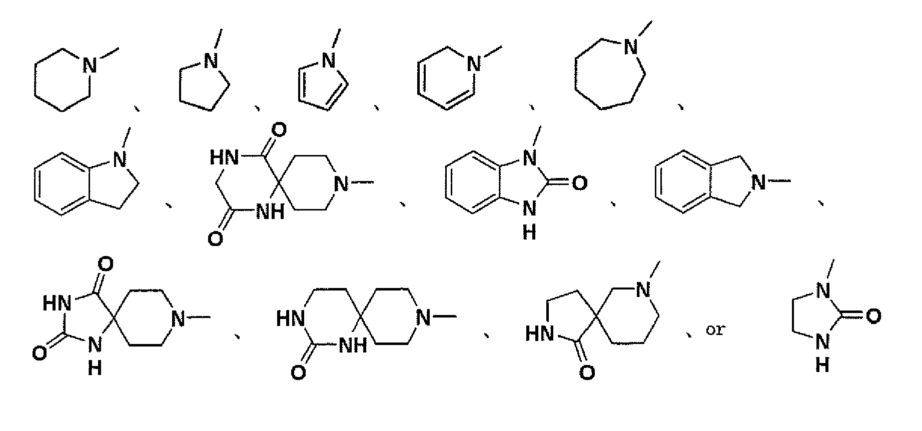 (wherein a hydrogen atom bound to a nitrogen atom may be replaced with J or R).

The substituents in the cyclic group which is formed by R¹ and R² together with a nitrogen atom to which they bind and the cyclic group formed by R² and R³ together with a nitrogen atom to which they bind are preferably 1 to 5 substituents selected from C1-8 alkyl, acyl, hydroxy which may have a substituent(s).

J is preferably a bond, C1-4 alkylene which may have 1 or 2 substituents, carbonyl or sulfonyl, more preferably a bond, methylene or carbonyl, and most preferably a bond.

Among the compounds represented by formula (I), preferred compounds are a compound represented by formula (I-1A): 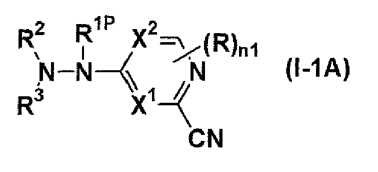 (wherein X¹ and X² is each independently a carbon atom or a nitrogen atom; R^{1P} is C1-8 alkyl which may have a substituent(s); and nl is 0 or an integer of 1-3),
a compound represented by formula (I-1B): 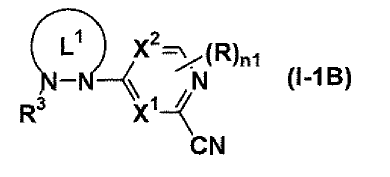 (wherein ring L¹ is a cyclic group which may have a substituent(s) formed by R¹ and R² together with a nitrogen atom to which they bind; and other symbols have the same meanings as described above),
a compound represented by formula (I-1C): 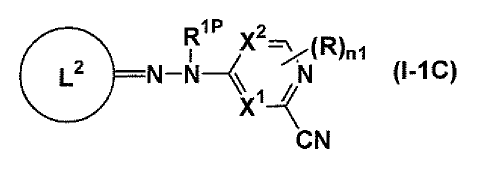 (wherein ring L² is a carbocyclic group which may have a substituent(s) or a heterocyclic group which may have a substituent(s) formed by R^{2X} and R^{3X} together with a carbon atom to which they bind; and other symbols have the same meanings as described above),
a compound represented by formula (I-1D): 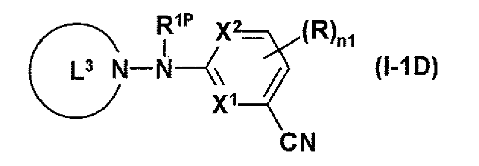 (wherein ring L³ is a cyclic group which may have a substituent(s) formed by R² and R³ together with a nitrogen atom to which they bind; and other symbols have the same meanings as described above),
a compound represented by formula (1-2A): 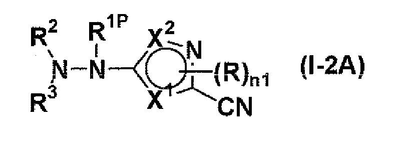 (wherein all symbols have the same meanings as described above),
a compound represented by formula (1-2B): 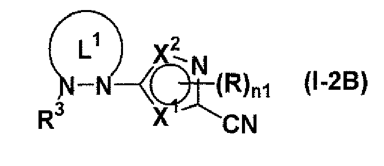 (wherein all symbols have the same meanings as described above),
a compound represented by formula (I-2C): 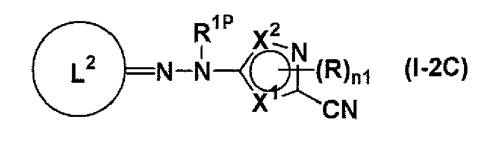 (wherein all symbols have the same meanings as described above),
a compound represented by formula (1-2D): 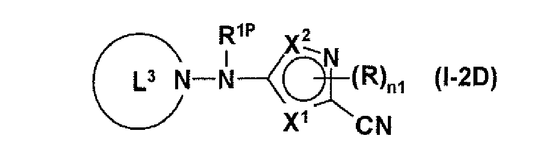 (wherein all symbols have the same meanings as described above),
a compound represented by formula (I-3A): 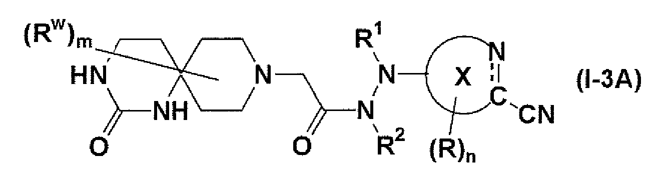 (wherein R^{w} is hydrogen atom or a substituent (having the same meaning as the substituent in the above-described "(2)carbocyclic group which may have a substituent(s)"; m is 0 or an integer of 1-5; and other symbols have the same meanings as described above, and wherein a hydrogen atom bound to a nitrogen atom may be replaced with R^{w}),
a compound represented by formula (I-3B): 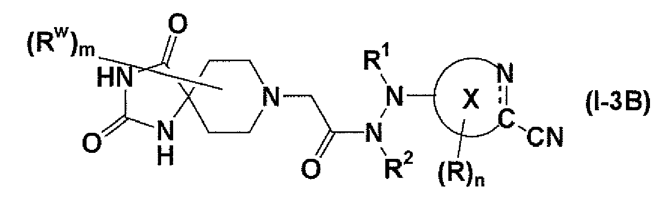 (wherein all symbols have the same meanings as described above, and wherein a hydrogen atom bound to a nitrogen atom may be replaced with R^{w}),
a compound represented by formula (I-3C): 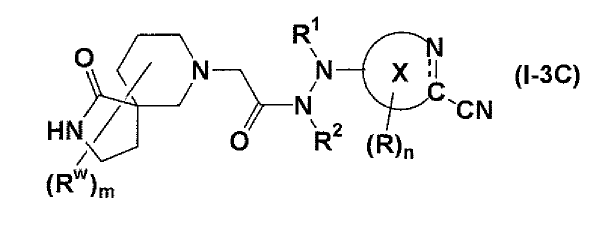 (wherein all symbols have the same meanings as described above, and wherein a hydrogen atom bound to a nitrogen atom may be replaced with R^{w}),
a compound represented by formula (I-3D): 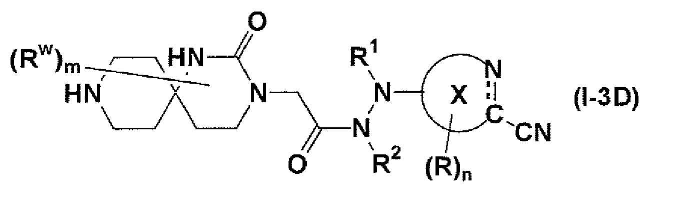 (wherein all symbols have the same meanings as described above, and wherein a hydrogen atom bound to a nitrogen atom may be replaced with R^{w}),
a compound represented by formula (I-3E): 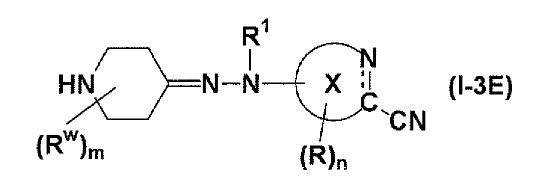 (wherein all symbols have the same meanings as described above, and wherein a hydrogen atom bound to a nitrogen atom may be replaced with R^{w}),
a compound represented by formula (I-3F): 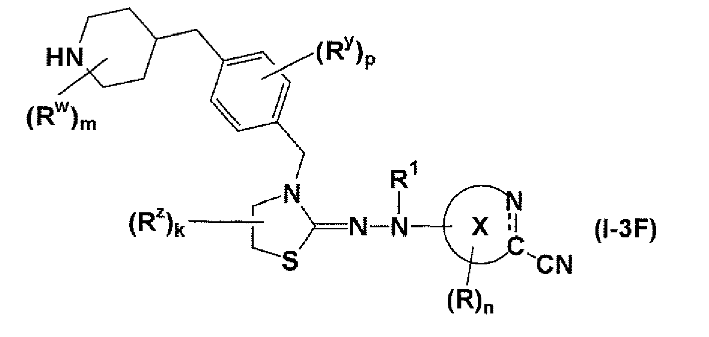 (wherein R^{y} and R^{z} is each independently a hydrogen atom or a substituent (having the same meaning as the substituent(s) in the above-described "(2) carbocyclic group which may have a substituent(s)"; p is 0 or an integer of 1-4; k is 0 or an integer of 1-4, and other symbols have the same meanings as described above, and wherein a hydrogen atom bound to a nitrogen atom may be replaced with R^{w}),
a compound represented by formula (1-3G): 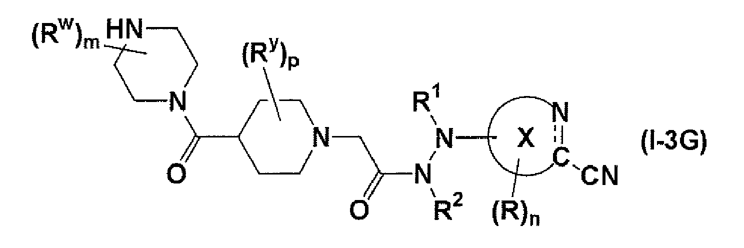 (wherein all symbols have the same meanings as described above, and wherein a hydrogen atom bound to a nitrogen atom may be replaced with R^{w}),
a compound represented by formula (I-3H): 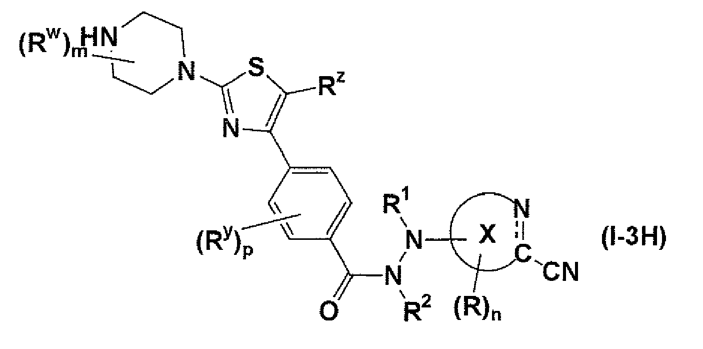 (wherein all symbols have the same meanings as described above, and wherein a hydrogen atom bound to a nitrogen atom may be replaced with R^{w}),
a compound represented by formula (I-3J): 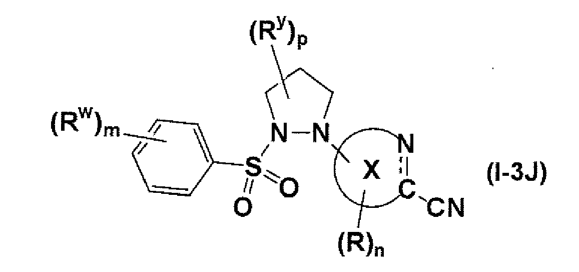 (wherein all symbols have the same meanings as described above),
a compound represented by formula (I-3K): 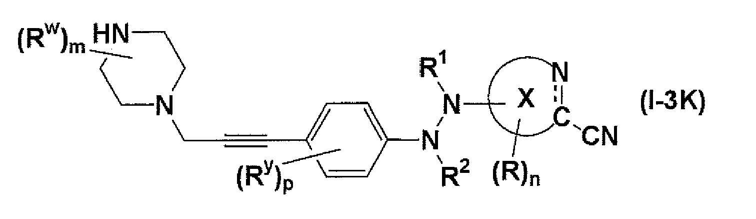 (wherein all symbols have the same meanings as described above, and wherein a hydrogen atom bound to a nitrogen atom may be replaced with R^{w}),
a compound represented by formula (I-4): 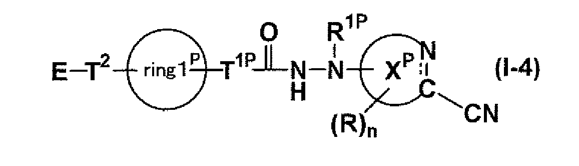 (wherein ring X^{P} is a 5- to 7-membered monocyclic heterocyclic group which may contain 1 to 3 hetero atoms selected from a nitrogen atom(s), an oxygen atom(s) and a sulfur atom(s) which may be oxidized, in addition to the nitrogen atom in the ring; T^{1P} is a bond or a spacer having 1 or 2 atoms of the principle chain; ring 1^{P} is a C5-7 monocyclic carbocyclic group or 5- to 7-membered monocyclic heterocyclic group which may have a substituent(s); and other symbols have the same meanings as described above),
a compound represented by formula (1-5); 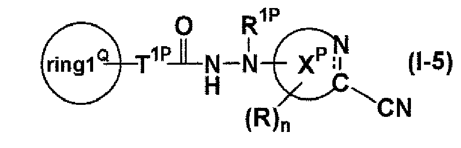 (wherein ring 1^{Q} is a nitrogen-containing heterocyclic group which may have a substituent(s); and other symbols have the same meanings as described above),
a compound represented by formula (I-6): 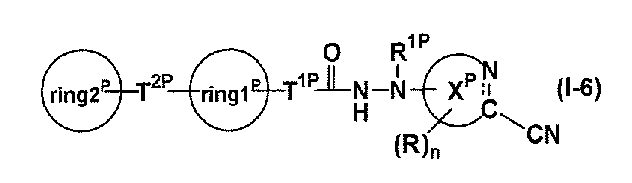 (wherein T^{2P} is a bond or a spacer having 1 or 2 atoms of the principle chain; ring 2^{P} is a C5-7 monocyclic carbocyclic ring or 5- to 7-membered heterocyclic group which may have a substituent(s); and other symbols have the same meanings as described above.), *etc.*

Furthermore, preferred is a compound represented by formula (I-1A) or a compound represented by formula (I-4). More preferred is a compound represented by formula (I-4-1): 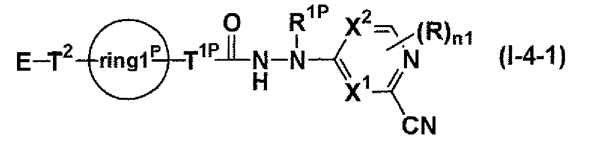 (wherein all symbols have the same meanings as described above).
"5- to 7-membered monocyclic heterocyclic group which may contain 1 to 3 hetero atoms selected from a nitrogen atom(s), an oxygen atom(s) and a sulfur atom(s) which may be oxidized, in addition to the nitrogen atom in the ring" represented by X^{P} is a 5- to 7-membered monocyclic aromatic heterocyclic group which may be partially or fully saturated, contains one nitrogen atom and may further contain 1 to 3 hetero atoms selected from a nitrogen atom(s), an oxygen atom(s) and a sulfur atom(s) which may be oxidized, and examples include tetrahydropyrimidine, dihyrdopyridazine, pyridazine, dihydropyrimidine, dihydropyrazine, dihydrotriazine, dihydropyrazole, diazepane, pyrrole, pyrimidine, pyrazine, imidazole, pyrazole, triazole, tetrazole, thiazole, oxazole, thiadiazole, oxadiazole, azepane, azepine, dihydroazepine, tetrahydroazepine, diazepane, diazepine, dihydroazepine and tetrahydroazepine rings, and more preferred are pyrimidine, pyrazine, pyridazine, thiazole and oxazole rings.

T^{1P} is preferably a bond, C1-2 alkylene (e.g., -CH₂-, -C(CH₃)₂-, *etc.)* which may have 1 or 2 substituents, -NH-, -N(CH₃)-, *etc.*

T^{2P} is preferably a bond, C1-2 alkylene (e.g., -CH₂-, -C(CH₃)₂-, *etc.*) which may have 1 or 2 substituents, -NH-, -N(CH₃)-, *etc.*

"C5-7 monocyclic carbocyclic group" represented by ring 1^{P} and ring 2^{P} is a C5-7 monocyclic aromatic carbocyclic group which may be partially or fully saturated, and "5- to 7-membered monocyclic heterocyclic group" is a 5- to 7-membered monocyclic aromatic heterocyclic group which may be partially or fully saturated and contains 1 to 3 hetero atoms selected from an oxygen atom(s), a nitrogen atom(s) and a sulfur atom(s) which may be oxidized. Ring 1^{P} and ring 2^{P} may be the same or different.

For example, ring 1^{P} is preferably a C5 or C6 monocyclic carbocyclic group such as benzene, cyclohexane, cyclopentane, cyclohexene or cyclopentene ring, or a 5-or 6-membered manocyclic heterocyclic group such as pyridine, thiazole, pyrrolidine, imidazolidine, dihyrdopyridine, piperidine, piperazine, tetrahydropyran, tetrahydrothiazole (thiazolidine), morpholine, or thiomorpholine ring, and is more preferably benzene, pyridine, pyrrolidine, piperidine, piperazine or morpholine ring.

For example, ring 2^{P} is preferably a C5 or C6 monocyclic carbocyclic group such as benzene, cyclohexane, cyclopentane, cyclohexene or cyclopentene ring, or a 5-or 6-membered monocyclic heterocyclic group, and is more preferably benzene, pyridine, imidazolidine, piperidine, piperazine, tetrahydropyran, morpholine or thiomorpholine ring.

The "nitrogen-containing heterocyclic group" in the "nitrogen-containing heterocyclic group which may have a substituent(s)" represented by ring 1^{Q} has the same meaning as the above-described "nitrogen-containing heterocyclic group" represented by ring B. For example, ring 1^{Q} is preferably piperazine, 2,8-diazabispiro[4.5]decane or 3,9-diazaspiro[5.5]undecane ring, *etc.*

Ring 1^{P}, ring 2^{P} and ring 1^{Q} may have 1 to 3 substituents at substitutable positions, and the substituents includes the above-described "substituent" in the "cyclic group which may have a substituent(s)" represented by ring 1, ring 2 and ring 3.

More specific embodiments include the following compounds, the compounds described in Examples and the like:
(1) N'-(2-cyanopyrimidin-4-yl)-N'-(2,2-dimethylpropyl)-1,3-dimethyl-2,5-dioxo-1,4,9-triazaspiro[5.5]undecane-9-carbahydrazide,
(2) N'-(2-cyanopyrimidin-4-yl)-N'-(2,2-dimethylpropyl)-4-[(4-methylpiperazin-1-yl)methyl]benzohydrazide,
(3) N'-(2-cyanopyrimidin-4-yl)-N'-(2,2-dimethylpropyl)-4-[1-(2-methoxyethyl)piperidin-4-yl]benzohydrazide,
(4) N'-(2-cyanopyrimidin-4-yl)-N'-(2,2-dimethylpropyl)-2-(2,4-dioxo-1,3,8-triazaspiro[4.5]dec-8-yl)acetohydrazide,
(5) 4-[2-(1-benzylpiperidin-4-ylidene)-1-(2,2-dimethylpropyl)hydrazino]pyrimidine-2-carbonitrile,
(6) N'-(3-chloro-6-cyanopyridin-2-yl)-N'-(2,2-dimethylpropyl)-1,3-dimethyl-2,5-dioxo-1,4,9-triazaspiro[5.5]undecane-9-carbohydrazide,
(7) N'-(3-chloro-6-cyanopyridin-2-yl)-N'-(2,2-dimethylpropyl)-4-[(4-methylpiperazin-1-yl)methyl]benzohydrazide,
(8) N'-(3-chloro-6-cyanopyridin-2-yl)-N'-(2,2-dimethylpropyl)-4-[1-(2-methoxyethyl)piperidin-4.-yI]benzohydrazide,
(9) N'-(6-cyano-3-(trifluoromethyl)pyridin-2-yl)-N'-(2,2-dimethylpropyl)-2-(2,4-dioxo-1,3,8-triazaspiro[4. 5]dec-8-yl)acetohydrazide,
(10) 6-[2-(1-benzylpiperidin-4-ylidene-1-(2,2-dimethylpropyl)hydrazino]-5-(trifluoromethyl)pyridine-2-carbonitrile,
(11) N'-[2-cyano-4-(2,2-dimethylpropoxy)pyrimidin-5-yl]-2,5-dioxo-3-(pyridin-4-ylmethyl)-1,4,9-triazaspiro[5.5]undecane-9-carbohydrazide,
(12) N'-[2-cyano-4-(2,2-dimethylpropoxy)pyrimidin-5-yl]-4-[(4-methylpiperazin-1-yl)methyl]benzohydrazide,
(13) N'-[2-cyano-4-(2,2-dimethylpropoxy)pyrimidin-5-yl]-4-[1-(2-methoxyethyl)piperidin-4-yl]benzohydrazide,
(14) N'-[2-cyano-4-(2,2-dimethylpropoxy)pyrimidin-5-yl]-2-(2,4-dioxo-1,3,8-triazaspiro[4.5]dec-8-yl)acetohydrazi de,
(15) N'-[2-cyano-4-(2,2-dimethylpropoxy)pyrimidin-5-yl]-2,4-dioxo-1,3,8-triazaspiro[4.5]decane-8-carbohydrazide,
(16) 5-[2-(1-benzylpiperidin-4-ylidene)hydrazino]-4-(2,2-dimethylpropoxy)pyrimidine-2-carbonitrile,
(17) 5-bromo-4-[1-(2,2-dimethylpropyl)-2-(4-methoxybenzyl)hydrazino]pyrimidine-2-carbonitrile,
(18) 5-bromo-4-[1-(2,2-dimethylpropyl)-2-(2,4-dioxo-1,3-diazaspiro[4.5]dec-8-ylidene)hydrazino]pyrimidine-2-carbonitrile,
(19) N'-(5-bromo-2-cyanopyrimidin-4-yl)-N'-(2,2-dimethylpropyl)-4-methylhenzenesulfonohydrazide,
(20) 5-bromo-4-{(2,2-dimethylpropyl)[1-methyl-2,5-dioxo-3-(pyridin-4-ylmethyl)-1,4,9-triazaspiro[5.5]undec-9-yl]amino}pyrimidine-2-carbonitrile,
(21) N'-(6-cyanopyradin-2-yl)-N'-(2,2-dimethylpropyl)-N, 1,3-trimethyl-2,5-dioxo-1,4,9-triazaspiro[5.5]undecane-9-carbohydrazide,
(22) N`-(5-cyanopyrazin-2-yl)-N'-(2,2-dimethylpropyl)-4-[2-(4-methylpiperazin-1-yl)-1, 3-thiazol-4-yl]benzohydrazide,
(23) N'-(6-cyanopyrazin-2-yl)-N'-(2,2-dimethylpropyl)-4-[1-(2-methoxyethyl)piperidin-4-yl]-N-methylbenzohydrazide,
(24) 6-[1-(2,2-dimethylpropyl)-2-(2,4-dioxo-1,3-diazaspiro[4.5]dec-8-ylidene)hydrazino]pyrazine-2-carbonitrile,
(25) benzyl (1-{[2-(6-cyanopyrazin-2-yl)-2-(2,2-dimethylpropyl) hydrazino]carbonyl}-3-methylbutyl)carbamate,
(26) 4-{2-[(1,3-dimethyl-2,5-dioxo-1,4,9-triazaspiro[5.5]undec-9-yl)carbonyl]-4,4-dimethylpyrazolidin-1-yl}pyrimidine-2-carbonitrile,
(27) 4-(4,4-dimethyl-2-{4-[(4-methylpiperazin-1-yl)methyl]benzoyl}pyrazolidin-1-yl)pyrimidine-2-carbonitrile,
(28) benzyl (1-{[2-(2-cyanopyrimidin-4-yl)-4,4-dimethylpyrazolidin-1-yl]carbonyl}-3-methylbutyl)carbamate,
(29) 4-(2-{[4-(1H-imidazol-1-yl)phenyl]acetyl}-4,4-dimethylpyrazolidin-1-yl)pyrimidine-2-carbonitrile,
(30) 4-[2-(4-methoxybenzyl)-4,4-dimethylpyrazolidin-1-yl]pyrimidine-2-carbonitrile,
(31) N'-(2-cyano-5-methylpyrimidin-4-yl)-2-(1,3-dimethyl-2-oxo-1,3,9-triazaspiro[5.5]undec-9-yl)-N'-(2,2-dimethylpropyl)acetohydrazide,
(32) N'-(3-bromo-6-cyanopyridin-2-yl)-2-(1,3-dimethyl-2-oxo-1,3,9-triazaspiro[5.5]undec-9-yl)-N'-(2,2-dimethylpropyl)acetohydrazide,
(33) N'-(6-cyanopyrazin-2-yl)-N'-(2,2-dimethylpropyl)-2-[1-methyl-2-oxo-3 - (pyridin-4-ylmethyl)-1,3,9-triazaspiro[5.5]undec-9-yl]acetohydrazide,
(34) N'-(4-cyano-1-methyl-1H-imidazol-2-yl)-N'-isobutyl-4-[1-(2-methoxyethyl)piperidin-4-yl]benzohydrazide,
(35) N'-(4-cyano-1-methyl-1H-imidazol-2-yl)-2-(2,4-dioxo-1,3,8-triazaspiro[4.5]dec-8-yl)-N'-isobutylacetohydrazide,
(36) N'-(4-cyano-1-methyl-1H-imidazol-2-yl)-N'-isobutyl-2-{4-[(4-methylpiperazin-1-yl)carbonyl]piperidin-1-yl}acetohydrazide,
(37) 2-[2-(2,4-dioxo-1,3-diazaspiro[4.5]dec-8-ylidene)-1-isobutylhydrazino]-1-methyl-1H-imidazole-4-carbonitrile,
(38) 2-[2-(1-benzylpiperidin-4-ylidene)-1-isobutylhydrazino]-1,3-thiazole-4-carbonitrile,
(39) 2-[(2,2-dimethylpropyl)(2,4-dioxo-1,3,8-triazaspiro[4.5]dec-8-yl)amino]-1,3-thiazole-4-carbonitrile.
(40) N'-[(2-cyanopyrimidin-4-yl)methyl]-N-isobutyl-4-[2-(4-methylpiperazin-1-yl)-1,3-thiazol-4-yl]benzohydrazide,
(41) N'-[(2-cyanopyrimidin-4-yl)methyl]-N'-isopropyl-2-[1-oxo-2-(pyridin-4-ylmethyl)-2,7-diazaspiro[4.5]dec-7-yl]acetohydrazide,
(42) N'-[(2-cyanopyrimidin-4-yl)methyl]-N'-isopropyl-1,3-dimethyl-2,5-dioxo-1,4,9-triazaspiro[5.5]undecane-9-carbohydrazide,
(43) N'-[(6-cyanopyrazin-2-yl)methyl]-N'-isopropyl-1,3-dimethyl-2,5-dioxo-1,4,9-triazaspiro[5.5]undecane-9-carbohydrazide,
(44) 4-{4,4-dimethyl-2-[(4-methylphenyl)sulfonyl]pyrazolidin-1-yl}pyrimidine-2-carbonitrile,
(45) 5-acetyl-6-(1-(2,2-dimethylpropyl)-2-{4-[3-(4-isopropylpiperazin-1-yl)prop-1-yn-1-yl]pheny}hydrazino)pyridine-2-carbonitrile,
(46) N'-[(2-cyanopyrimidin-5-yl)methyl]-N'-isobutyl-4-[(4-methylpiperazin-1-yl)methyl]benzohydrazide,
(47) N'-[(2-cyanopyrimidin-5-yl)methyl]-N'-(3,3-dimethylbutyl)-4-(1H-imidazol-1-yl)benzohydrazide,
(48) 2-cyano-N-(3,3-dimethylbutyl)-N'-{4-[(4-methylpiperazin-1-yl)methyl]benzoyl}pyrimidine-5-carbohydrazide,
(49) N'-[(2-cyanopyrimidin-5-yl)carbonyl]-N'-(3,3-dimethylbutyl)-2,4-dioxo-l,3,8-triazaspiro[4.5]decane-8-carbohydrazide,
(50) 2-cyano-4-(2,2-dimethylpropoxy)-N'-{4-[l-(2-methoxyethyl)piperidin-4-yl]benzoyl}pyrimidine-5-carbohydrazide,
(51) N'-(5-cyanopyrazolo[1,5-a]pyrimidin-7-yl)-N'-(2,2-dimethylpropyI)-2,4-dioxo-1,3,8-triazaspiro[4.5]decane-8-carbohydrazide,
(52) N'-(2-cyanopyrazolo[1,5-a][1,3,5]triazin-4-yl)-N'-(2,2-dimethylpropyl)-2,4-dioxo-1,3,8-triazaspiro[4.5]decane-8-carbohydrazide,
(53) N'-(7-cyanoimidazo[1,2-a]pyrimidin-5-yl)-N'-(2,2-dimethylpropyl)-2,4-dioxo-1,3,8-triazaspiro[4.5]decane-8-carbohydrazide,
(54) N'-(5-cyanopyrazolo[1,5-a]pyrimidin-7-yl)-N'-(2,2-dimethylpropyl)-1,3-dimethyl-2,5-dioxo-1,4,9-triazaspiro[5.5]undecane-9-carbohydrazide,
(55) N'-(2-cyanopyrazolo[1,5-a][1,3,5]triazin-4-yl)-N'-(2,2-dimethylpropyl)-1,3-dimethyl-2,5-dioxo-1,4,9-triazaspiro[5.5]undecane-9-carbohydrazide,
(56) N'-(7-cyanoimidazo[1,2-a]pyrimidin-5-yl)-N'-(2,2-dimethylpropyl)-1,3-dimethyl-2,5-dioxo-1,4,9-triazaspiro[5.5]undecane-9-carbohydrazide,
(57) N'-(5-cyanopyrazolo[1,5-a]pyrimidin-7-yl)-N'-(2,2-dimethylpropyl)-2-(2,4-dioxo-1,3,8-triazaspiro[4.5]dec-8-yl)acetohydrazide,
(58) N'-(2-cyanopyrazolo[1,5-a][1,3,5]triazin-4-yl)-N'-(2,2-dimethylpropyl)-2-(2,4-dioxo-1,3,8-triazaspiro[4.5]dec-8-yl)acetohydrazide,
(59) N'-(7-cyanoimidazo[1,2-a]pyrimidin-5-yl)-N'-(2,2-dimethylpropyl)-2-(2,4-dioxo-1,3,8-triazaspiro[4.5]dec-8-yl)acetohydrazide,
(60) N'-(5-cyanopyrazolo[1,5-a]pyrimidin-7-yl)-N'-(2,2-dimethylpropyl)-2-{4-[(4-methylpiperazin-1-yl)carbonyl]piperidin-1-yl}acetohydrazide,
(61) N'-(2-cyanopyrazolo[1,5-a][1,3,5]triazin-4-yl)-N'-(2,2-dimethylpropyl)-2-{4-[(4-methytpiperazin-1-yl)carbonyl]piperidin-1-yl}acetohydrazide,
(62) N'-(7-cyanoimidazo[1,2-a]pyrimidin-5-yl)-N'-(2,2-dimethylpropyl)-2-{4-[(4-methylpiperazin-1-yl)carbonyl]pipendin-1-yl} acetohydrazide,
(63) N'-(5-cyanopyrazolo[1,5-a]pyrimidin-7-yl)-N'-(2,2-dimethylpropyl)-4-[(4-methylpiperazin-1-yl)methyl]benzohydrazide,
(64) N'-(2-cyanopyrazolo[1,5-a][1,3,5]triazin-4-yl)-N'-(2,2-dimethylpropyl)-4-[(4-methylpiperazin-1-yl)methyl]benzohydrazide,
(65) N'-(7-cyanoimidazo[1,2-a]pyrimidin-5-yl)-N'-(2,2-dimethylpropyl)-4-[(4-methylpiperazin-1-yl)methyl]benzohydrazide,
(66) N'-(5-cyanopyrazalo[1,5-a]pyrimidin-7-yl)-N'-(2,2-dimethylpropyl)-4-[2-(4-methylpiperazin-1-yl)-1,3-thiazol-4-yl]benzohydrazide,
(67) N'-(2-cyanopyrazolo[1,5-a][1,3,5]triazin-4-yl)-N'-(2,2-dimethylpropyl)-4-[2-(4-methylpiperazin-1-yl)-1,3-thiazol-4-yl]benzohydrazide,
(68) N'-(7-cyanoimidazo[1,2-a]pyrimidin-5-yl)-N'-(2,2-dimethylpropyl)-4-[2-(4-methylpiperazin-1-yl)-1,3 -thiazol-4-yl]benzohydrazide,
(69) N'-(5-cyanopyrazolo[1,5-a]pyrimidin-7-yl)-N'-(2,2-dimethylpropyl)-4-[4-(2-methoxyethyl)piperazin-1-yl]benzohydrazide,
(70) N'-(2-cyanopyrazolo[1,5-a][1,3,5]triazin-4-yl)-N'-(2,2-dimethylpropyl)-4-[4-(2-methoxyethyl)piperazin-1-yl]benzahydrazide,
(71) N'-(7-cyanoimidazo[1,2-a]pyrimidin-5-yl)-N'-(2,2-dimethylpropyl)-4-[4-(2-methoxyethyl)piperazin-1-yl]benzohydrazide,
(72) N'-(5-cyanopyrazolo[1,5-a]pyrimidin-7-yl)-N'-(2,2-dimethylpropyl)-2-[1-oxo-2-(pyridin-4-ylmethyl)-2,7-diazaspiro[4.5]dec-7-yl]acetohydrazide,
(73) N'-(2-cyanopyrazolo[1,5-a][1,3,5]triazin-4-yl)-N'-(2,2-dimethylpropyl)-2-[1-oxo-2-(pyridin-4-ylmethyl)-2,7-diazaspiro[4.5]dec-7-yl]acetohydrazide,
(74) N'-(7-cyanoimidazo[1,2-a]pyrimidin-5-yl)-N'-(2,2-dimethylpropyl)-2-[1-oxo-2-(pyridin-4-ylmethyl)-2,7-diazaspiro[4.5]dec-7-yl]acetohydrazide,
(75) N'-(1-cyanoimidazo[1,5-a]pyrazin-3-yl)-N'-(2,2-dimethylpropyl)-2-[1-oxo-2-(pyridin-4-ylmethyl)-2,7-diazaspiro[4.5]dec-7-yl]acetohydrazide,
(76) 7-{(2,2-dimethylpropyl)[1-methyl-2,5-dioxo-3-(pyridin-4-ylmethyl)-1,4,9-triazaspiro[5.5]undec-9-yl]amino}pyrazolo[1,5-a]pyrimidine-5-carbonitrile,
(77) 4-{(2,2-dimethylpropyl)[1-methyl-2,5-dioxo-3-(pyridin-4-ylmethyl)-1,4,9-triazaspiro[5.5]undec-9-yl]amino}pyrazolo[1,5-a][1,3,5]triazine-2-carbonitrile,
(78) 5-{(2,2-dimethylpropyl)[1-methyl-2,5-dioxo-3-(pyridin-4-ylmethyl)-1,4,9-triazaspiro[5.5]undec-9-yl]amino}imidazo[1,2-a]pyrimidine-7-carbonitrile,
(79) 7-[1-(2,2-dimethylpropyl)-2-(2,4-dioxo-1,3-diazaspiro[4.5]dec-8-ylidene)hydrazino]pyrazolo[1,5-a]pyrimidine-5-carbonitrile,
(80) 4-[1-(2,2-dimethylpropyl)-2-(2,4-dioxo-1,3-diazaspiro[4,5]dec-8-ylidene)hydrazino]pyrazolo[1,5-a] [1,3,5]triazine-2-carbonitrile,
(81) 5-[1-(2,2-dimethylpropyl)-2-(2,4-dioxo-1,3-diazaspiro[4.5]dec-8-ylidene)hydrazino]imidazo[1,2-a]pyrimidine-7-carbonitrile,
(82) 5-[1-(2,2-dimethylpropyl)-2-(2,4-dioxo-1,3-diazaspiro[4.5]dec-8-ylidene)hydrazino]imidazo[1,2-c]pyrimidine-7-carbonitrile,
(83) 4-[1-(2,2-dimethylpropyl)-2-(4-methoxybenzyl)hydrazinol-7-isobutylpyrazolo [1,5-a] [1,3,5] triazine-2-carbonitrile,
(84) 7-[1-(2,2-dimethylpropyl)-2-(4-methoxybenzyl)hydrazino]-2-isobutylpyrazolo[1,5-a]pyrimidine-5-carbonitrile,
(85) benzyl (1-{[2-(5-cyanopyrazolo[1,5-a]pyrimidin-7-yl)-4,4-dimethylpyrazolidin-1-yl]carbonyl}-3-methylbutyl)carbamate,
(86) benzyl (1-{[2-(2-cyanopyrazolo [1,5-a] [1,3,5]triazin-4-yl)-4,4-dimethylpyrazolidin-1-yl]carbonyl}-3-methylbutyl) carbamate,
(87) benzyl (1-{[2-(7-cyanoimidazo[1,2-a]pyrimidin-5-yl)-4,4-dimethylpyrazolidin-1-yl]carbonyl}-3-methylbutyl)carbamate,
(88) 7-(1-(2,2-dimethylpropyl)-2-{4-[3-(4-isopropylpiperazin-1-yl)prop-1-yn-1-yl]phenyl}hydrazino)pyrazolo[1,5-a]pyrimidine-5-carbonitrile,
(89) 4-(1-(2,2-dimethylpropyl)-2-{4-[3-(4-isopropylpiperazin-1-yl)prop-1-yn-1-yl]phenyl}hydrazino)pyrazolo[1,5-a][1,3,5]triazine-2-carbonitrile,
(90) 5-(1-(2,2-dimethylpropyl)-2-{4-[3-(4-isopropylpiperazin-1-yl)prop-1-yn-1-yl]phenyl}hydrazino)imidazo[1,2-a]pyrimidine-7-carbonitrile,
(91) 7-(1-(2,2-dimethylpropyl)-2-{3-[4-(1-methylpiperidin-4-yl)benzyl]-1,3-thiazolidin-2-ylidene}hydrazino)pyrazolo[1,5-a]pyrimidine-5-carbonitrile,
(92) 4-(1-(2,2-dimethylpropyl)-2-{3-[4-(1-methylpiperidin-4-yl)benzyl]-1,3-thiazolidin-2-ylidene}hydrazino)pyrazolo[1,5-a][1,3,5]triazine-2-carbonitrile,
(93) 5-(1-(2,2-dimethylpropyl)-2-{3-[4-(1-methylpiperidin-4-yl)benzyl]-1,3-thiazolidin-2-ylidene}hydrazino)imidazo[1,2-a]pyrimidine-7-carbonitrile,
(94) 7-{1-(2,2-dimethylpropyl)-2-[(2,4-dioxo-1,3,8-triazaspiro[4.5]dec-8-yl)methyl]-2-methylhydrazino}pyrazolo[1,5-a]pyrimidine-5-carbonitrile,
(95) 2-(9-benzyl-1-methyl-2-oxo-1,3,9-triazaspiro[5.5]undec-3-yl)-N'-(2-cyanopyrazolo[1,5-a][1,3,5]triazin-4-yl)-N'-(2,2-dimethylpropyl)acetohydrazide,
(96) N'-(2-cyano-6,7-dihydro-5H-cyclopenta[d]pyrimidin-4-yl)-N'-(2,2-dimethylpropyl)-2,4-dioxo-1,3,8-triazaspiro[4.5]decane-8-carbohydrazide,
(97) N'-[2-cyano-8-(2,2-dimethylpropyl)pyrazolo[1,5-a][1,3,5]triazin-4-yl]-N'-(2,2-dimethylpropyl)-4-[(4-methylpiperazin-1-yl)methyl]benzohydrazide,
(98) N'-(1-cyanoimidazo[1,5-a]pyrazin-3-yl)-N'-(2,2-dimethylpropyl)-4-[(4-methylpiperazin-1-yl)methyl]benzohydrazide,
(99) N'-(2-cyanothieno[3,2-d]pyrimidin-4-yl)-N'-(2,2-dimethylpropyl)-2,4-dioxo-1,3,8-triazaspiro[4.5]decane-8-carbohydrazide,
(100) N'-(2-cyanoquinazolin-5-yl)-N'-(2,2-dimethylpropyl)-4-[(4-methylpiperazin-1-yl)methyl]benzohydrazide,
(101) N'-(2-cyanopyrazolo[1,5-a]pyrimidin-7-yl)-N'-(2,2-dimethylpropyl)-4-[(4-methylpiperazin-1-yl)methyl]benzohydrazide,
(102) 2-cyano-N-(2,2-dimethylpropyl)-N'-{4-[(4-methylpiperazin-1-yl)methyl]benzoyl} pyrazolo[1,5-a][1,3,5]triazine-4-carbohydrazide.

Furthermore, the compounds described in Examples, salts thereof solvates thereof, N-oxides thereof and prodrugs thereof are preferred. More preferred are
N'-[2-cyano-6-(trifluoromethyl-4-pyrimidinyl]-4-[(4-methyl-1-piperazinyl)methyl]-N'-neopentylbenzohydrazide (Compound 15-1),
N'-(2-cyano-5-methyl-4-pyrimidinyl)-4-[(4-methyl-1-piperazinyl)methyl]-N'-neopentylbenzohydrazide (Compound 15-3),
N'-[2-cyano-6-(trifluoromethyl-4-pyrimidinyl]-2-(dimethylamino)-N'-neopentylacetohydrazide (Compound 15-13),
N'-(5-bromo-2-cyano-4-pyrimidinyl)-4-[1-(2-methoxyethyl)-4-piperidinyl]-N'-neopentylbenzohydrazide (Compound 17-1),
N'-[2-cyano-6-(trifluoromethyl-4-pyrimidinyl]-4-[(dimethylamino)methyl]-N'-neopentylbenzohydrazide (Compound 17-21),
N'-[2-cyano-6-(trifluoromethyl-4-pyrimidinyl]-N'-neopentylnicotinohydrazide (Compound 17-26),
N'-[2-cyano-6-(trifluoromethyl-4-pyrimidinyl]-4-(4-morpholinylmethyl)-N'-neopentylbenzohydrazide (Compound I7-35),
N'-[2-cyano-6-(trifluoromethyl-4-pyrimidinyl]-4-[(dimethylamino)methyl]-3-fluoro-N`-neopentylbenzohydrazide (Compound 17-36),
N'-(2-cyanopyrazolo[1,5-a][1,3,5]triazin-4-yl)-4-[1-(2-methoxyethyl)-4-piperidinyl]-N'-neopentylbenzohydrazide (Compound 17-37),
N'-[2-cyano-6-(trifluoromethyl-4-pyrimidinyl]-2-methyl-2-[4-(1-methyl-4-piperidinyl)phenyl]-N'-neopentylpropanohydrazide (Compound 17-44),
N'-(2-cyano-6-methyl-4-pyrimidinyl)-4-[(dimethylamino)methyl]-N'-neopentylbenzohydrazide (Compound 17-48),
N'-[2-cyano-6-(trifluoromethyl-4-pyrimidinyl]-2-{4-[(dimethylamino)methyl]phenyl-N'-2-methyl-N'-neopentylpropanohydrazide (Compound 17-57),
N'-(2-cyano-6-methyl-4-pyrimidinyl)-4-[2-(dimethylamino)ethoxy]-N'-neopentylbenzohydrazide (Compound 17-69),
N'-(2-cyano-6-methyl-4-pyrimidinyl)-2-{4-[(dimethylamino)methyl]phenyl}-2-methyl-N'-neopentylpropanohydrazide (Compound 17-71),
N'-[2-cyano-6-(trifluoromethyl-4-pyrimidinyl]-N'-neopentyl-2-(1-pyrrolidinyl)acetohydrazide (Compound 17-74),
N'-[2-cyano-6-(trifluoromethyl-4-pyrimidinyl]-N'-(2,2-dimethylpropyl)-6-(4-methyl-1-piperazinyl)nicotinohydrazide (Compound 49-9),
N'-[2-cyano-6-(trifluoromethyl-4-pyrimidinyl]-N'-(2,2-dimethylpropyl)-4-{[(2-methoxyethyl)(methyl)amino]methyl}benzohydrazide (Compound 50-4),
N'-(2-cyano-6,7-dihydro-5H-cyclopenta[d]pyrimidin-4-yl)-4-[(dimethylamino)methyl]-N'-(2,2-dimethylprapyl)benzohydrazide (Compound 50-18),
N'-[2-cyano-6-(trifluoromethyl-4-pyrimidinyl]-3-(dimethylamino)-N'-(2,2-dimethylpropyl)propanohydrazide (Compound 50-19),
N'-(2-cyano-6,7-dihydro-5H-cyclopenta[d]pyrimidin-4-yl)-2-{4-[(dimethylamino)methyl]phenyl}-N'-(2,2-dimethylpropyl)-2-methylpropanohydrazide (Compound 50-22),
N'-(2-cyano-6-methyl-4-pyrimidinyl)-N'-(2,2-dimethylpropyl)-2-methyl-2-{4-[(4-methyl-1-piperazinyl)methyl]phenyl}propanohydrazide (Compound 50-25),
N'-[2-cyano-6-(trifluoromethyl-4-pyrimidinyl]-4'-[(dimethylamino)methyl]-N'-(2,2-dimethylpropyl)-4-biphenylcarbohydrazide (Compound 50-26),
N'-(2-cyano-6-methyl-4-pyrimidinyl)-4-[(dimethylamino)methyl]-N'-(2,2-dimethylpropyl)-3-methoxybenzohydrazide (Compound 50-30),
N-[2-cyano-6-(trifluoromethyl-4-pyrimidinyl]-N'-(2,2-dimethylpropyl)-2-(methylamino)acetohydrazide (Compound 51),
*tert-butyl* 2-(2-cyano-5,7-dihydrofuro[3,4-d]pyrimidio-4-yl)-2-(2,2-dimethylpropyl)hydrazinecarboxylate (Compound 55),
salts thereof, solvates thereof, N-oxides thereof, prodrugs thereof and the like.

In the present invention, isomers are included unless specified. For example, alkyl, alkoxy, alkylthio, alkenyl, alkynyl, alkylene, alkylidene, and alkenylidene include straight chain and branched ones. Furthermore, the present invention includes isomers in double bond, ring, fused ring (E, Z, cis, trans), isomers by the presence of asymmetric carbon (R-, S-form, α-, β-configuration, enantiomer, diastereomer), optical isomers having optical rotation (D, L, d, 1, +, - form), polars by chromatography separation (more polar compound, less polar compound), equilibrium compound, a compound of arbitrary ratios of those and racemic mixture. An optically active compound in the present invention includes not only 100% optically pure compound and may include (an)other optical isomer(s) less than 50%.

### Salt, N-oxide, solvate and prodrug:

The salt of the compound of formula (I) includes all of the salt which are pharmaceutically acceptable. With regard to the pharmaceutically acceptable salts, those which are low-toxic and soluble in water are preferred. Examples of appropriate salts are salt with alkaline metal (such as potassium, sodium and lithium), salt with alkaline earth metal (such as calcium and magnesium), ammonium salt (such as tetramethylammonium salt and tetrabutylammonium salt), salt with organic amine (such as triethylamine, methylamine, dimethylamine, cyclopentylamine, benzylamine, phenethylamine, piperidine, monoethanolamine, diethanolamine, tris(hydroxymethyl) methylamine, lysine, arginine and N-methyl-D-glucamine) and acid addition salt [such as inorganic acid salt (e.g., hydrochloride, hydrobromide, hydroiodide, sulfate, phosphate and nitrate, *etc.)* and organic acid salt (e.g., acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate, *etc.), etc*.].

An N-oxide of a compound of formula (I) means a compound of formula (I) which nitrogen is oxidized. An N-oxide of a compound of formula (I) may also be salt of alkaline (earth) metal, ammonium, organic amine, or acid addition salt.

The proper solvate of the compound of formula (I) includes, for example, hydarate, alcoholate (ethanolate, *etc.), etc.* The solvate is preferably non-toxic and water-soluble. The solvate of the compound of formula (I) also includes solvates of the above-mentioned alkaline (earth) metal salt thereof, (quaternary) ammonium salt thereof, organic amine salt thereof, acid addition salt thereof, and N-oxide thereof.

The compounds of formula (I) can be converted to salts thereof, N-oxide thereof, or solvate thereof by known methods.

The prodrugs of the compound of formula (I) mean the compounds converted into the compound of formula (I) by the reactions of enzymes, gastric acid and the like in an organism. As the prodrugs of the compound of formula (I), when the compound of formula (I) has amino group, the amino group of the compound is acylated, alkylated, or phosphorylated, (e.g., the amino group of the compound of formula (I) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated, *tert*-butylated and the like); when the compound of formula (I) has hydroxy group, the hydroxy group of the compound is acylated, alkylated, phosphorylated, borated (e.g., the hydroxy group of the compound of formula (I) is acetylated, palmitoylated, propanoylated, pivaloylated, succinylated, fumarylated, alanylated, dimethylaminomethylcarbonylated and the like); when the compound of formula (I) has carboxy group, the carboxy group of the compounds are ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonylethylesterified, methylamidated and the like); and the like. These compounds can be manufactured by the conventional methods per se. In addition, the prodrugs of the compounds of formula (I) may be hydrates or not. The prodrugs of the compound of formula (I) may be those ones which are converted to a compound of formula (I) in physiological conditions as described in Molecular Design, as Vol.7 of Development of pharmaceutical drugs, 1990. 163-198, Hirokawa Publishing. The compounds represented by formula (I) may be labeled with an isotope (e.g., ³H, ¹⁴C, ³⁵S, ¹²⁵I, *etc.)* or the like.

### Methods for the preparation of the compound of the present invention:

The compound represented by formula (I), the salt thereof, the solvate thereof, the N-oxide thereof or a prodrug thereof (hereinafter referred to as the compound of the present invention) can be prepared by methods which properly improved and combined known methods, such as methods described in Comprehensive Organic Transformations: A Guide to Functional Group Preparations, 2nd Edition (Richard C. Larock, John Wiley & Sons Inc, 1999), methods described below, or methods described in Examples. In each method described below, a starting material can be used as a salt thereof An example of the salt includes a pharmaceutically acceptable salt of the compound of formula (I) described above.

A compound represented by formula (I) can be prepared by cyanation of a compound represented by formula (II): 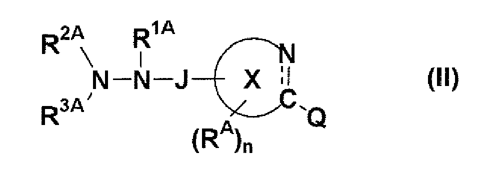 (wherein Q is a leaving group (e.g., halogen atom, methylsulfonyl, 4-methylphenylsulfonyl, *etc.),* R^{A}, R^{1A}, R^{2A} and R^{3A} have the same meanings as R, R¹, R² and R³, respectively, and carboxy, hydroxy, amino or mercapto in the groups represented by R^{A}, R^{1A}, R^{2A} and R^{3A} is protected if the protection is necessary; and other symbols have the same meanings as described above)
and if necessary, followed by removal of the protecting group.

The cyanation is well known. For example, it may be carried out by reacting a compound represented by formula (II) with cyanation reagent (e.g., potassium cyanide, sodium cyanide, tetrabutylammonium cyanide, tetraethylammonium cyanide, *etc.)* in an organic solvent (e.g., dimethylsulfoxide, dimethylformamide, dioxane or mixed solvent thereof and water), in the presence of tertiary amine (e.g., 1,4-diazabicyclo[2.2.2]octane (DABCO), trimethylamine, dimethylaminopyridine, *etc.)* at 0 to 150°C.

The reaction for removing the protective group for carboxy, hydroxy, amino or mercapto is known and its examples are as follows.
(1) a deprotection reaction by hydrolyzing reaction with an alkali;
(2) a deprotection reaction under an acidic condition;
(3) a deprotection reaction by hydrogenolysis;
(4) a deprotection reaction of silyl;
(5) a deprotection reaction using a metal; and
(6) a deprotection reaction using a metal complex.

Those methods will be specifically illustrated as follows.
(1) A deprotection reaction by hydrolyzing reaction with an alkali is carried out, for example, at 0 to 40°C using a hydroxide of alkaline metal (e.g., sodium hydroxide, potassium hydroxide and lithium hydroxide, *etc.),* a hydroxide of alkaline earth metal (e.g., barium hydroxide and calcium hydroxide, *etc.),* a carbonate (e.g., sodium carbonate and potassium carbonate, *etc.),* or an aqueous solution thereof or a mixture thereof in an organic solvent (e.g., methanol, tetrahydrofuran and dioxane *etc.).*
(2) A deprotection reaction under an acidic condition is carried out, for example, at 0 to 100°C in an organic acid (e.g., acetic acid, trifluoroacetic acid, methanesulfonic acid or p-toluenesulfonic acid, *etc.),* an inorganic acid (e.g., hydrochloric acid and sulfuric acid, *etc.)* or a mixture thereof (e.g., hydrogen bromide/acetic acid) in an organic solvent (e.g., dichloromethane, chloroform, dioxane, ethyl acetate and anisole *etc.)* in the presence or absence of 2,2,2-trifluoroethanol.
(3) A deprotection reaction by hydrogenolysis is carried out, for example, at 0 to 200°C under a hydrogen atmosphere of ordinary pressure or high pressure or in the presence of ammonium formate in the presence of a catalyst (e.g., palladium-carbon, palladium black, palladium hydroxide-carbon, platinum oxide and Raney nickel, *etc.)* in a solvent [e.g., an ether (e.g., tetrahydrofuran, dioxane, dimethoxyethane and diethyl ether, *etc.),* an alcohol (e.g., methanol and ethanol, *etc.),* a benzene (e.g., benzene and toluene, *etc.),* a ketone (e.g., acetone and methyl ethyl ketone, *etc.),* a nitrile (e.g., acetonitrile, *etc.),* an amide (e.g., dimethylformamide, *etc.),* water, ethyl acetate, acetic acid or a mixed solvent comprising two or more thereof].
(4) A deprotection reaction of silyl is carried out, for example, at 0 to 40°C using tetrabutylammonium fluoride in an organic solvent miscible with water (e.g., tetrahydrofuran and acetonitrile *etc.).*
(5) A deprotection reaction using metal is carried out, for example, at 0 to 40°C with or without ultrasonic wave in the presence of powdery zinc in an acidic solvent (e.g., acetic acid, a buffer of pH 4.2 to 7.2 and a mixed solution of a solution thereof with an organic solvent such as tetrahydrofuran, *etc.).*
(6) A deprotection reaction using a metal complex is carried out, for example, at 0 to 40°C using a metal complex [e.g., tetrakis(triphenylphosphine) palladium (0), bis(triphenylphosphine) palladium (II) dichloride, palladium (II) acetate and tris(triphenylphosphine) rhodium (I) chloride, etc.] in the presence or absence of a phosphine agent (e.g., triphenylphosphine, *etc.)* in the presence of a trap reagent (e.g., tributyltin hydride, triethylsilane, dimedone, morpholine, diethylamine, pyrrolidine, *etc.),* an organic acid (e.g., acetic acid, formic acid, 2-ethylhexanoic acid, *etc.)* and/or an organic acid salt (e.g., sodium 2-ethylhexanoate, potassium 2-ethylhexanoate, *etc.)* in an organic solvent (e.g., dichloromethane, dimethylformamide, tetrahydrofuran, ethyl acetate, acetonitrile, dioxane, ethanol, *etc.),* water or a mixed solvent thereof

Except for the above, the deprotection may also be carried out, for example, according to the methods described in T.W. Greene, Protective Groups in Organic Synthesis, Wiley, New York, 1999.

The protective group of carboxy includes, for example, methyl, ethyl, allyl, *tert-*butyl*,* trichloroethyl, benzyl (Bn) or phenacyl, p-methoxybenzyl, trityl, 2-chlorotrityl or polymer-supported group bound thereby, *etc,*

The protecting group of hydroxy includes, for example, methyl, trityl, methoxymethyl (MOM), 1-ethoxyethyl (EE), methoxyethoxymethyl (MEM), 2-tetrahydropyranyl (THP), trimethylsilyl (TMS), triethylsilyl (TES), tert-butyldimethylsilyl (TBDMS), *tert*-butyldiphenylsilyl (TBDPS), acetyl (Ac), pivaloyl, benzoyl, benzyl (Bn), p-methoxybenzyl, allyloxycarbonyl (Alloc), and 2,2,2-trichloroethoxycarbonyl (Troc), *etc.*

The protecting group of amino includes, for example, benzyloxycarbonyl, *tert*-butoxycarbonyl, allyloxycarbonyl (Alloc), 1-methyl-1-(4-biphenyl)ethoxycarbonyl (Bpoc), trifluoroacetyl, 9-fluorenylmethoxycarbonyl, benzyl (Bn), p-methoxybenzyl, benzyloxymethyl (BOM) or 2-(trimethylsilyl)ethoxymethyl (SEM), *etc.*

The protective group of mercapto includes, for example, benzyl, methoxybenzyl, methoxymethyl (MOM), 2-tetrahydropyranyl (THP), diphenylmethyl and acetyl (Ac), *etc.*

With regard to the protective group for carboxy, hydroxy, amino and mercapto, there is no particular limitation to the above ones so far as it is a group which is able to be easily and selectively removed. For example, a deprotection reaction may be carried out by a method described in "T. W. Greene, Protective Groups in Organic Synthesis, John Wiley & Sons Inc, 1999".

As persons skilled in the art can easily understand that the aimed compound of the present invention is able to be easily produced by using appropriate ones among those deprotection reactions.

The compound represented by formula (II) can be prepared, for example, by a method shown in the following reaction scheme 1.

In the scheme, Q¹ has the same meaning as Q, and other symbols have the same meanings as described above.

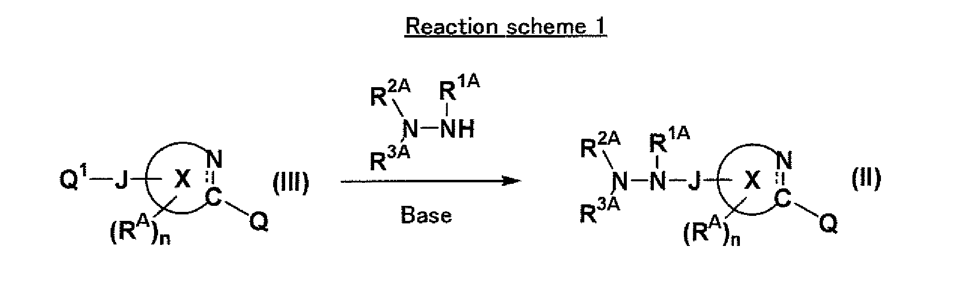

Among the compounds represented by formula (II), a compound in which Q is methylsulfonyl, that is, a compound represented by formula (II-1), can be prepared by a method shown in reaction scheme 2. In the reaction scheme 2, all symbols have the same meanings as described above.

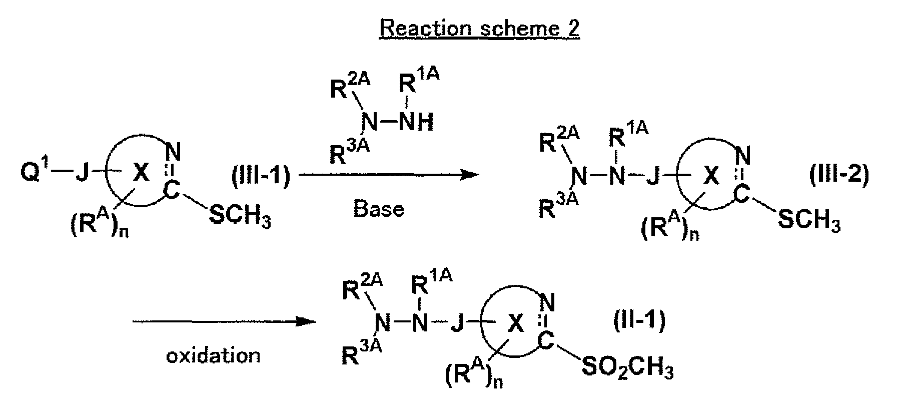

Also, among the compounds represented by formula (I), a compound in which ring X is a pyrimidine ring, for example, a compound represented by formula (IA) or formula (IA') shown below, can be prepared by a method shown in reaction scheme 3.

In the reaction scheme 3, R¹⁰¹ is a leaving group such as a halogen atom or methylsulfonyl; R¹⁰² is a protecting group of amino (e.g., , *tert*-butoxycarbonyl, benzyloxycarbonyl, *etc.*); R' and R" each independently has the same meaning as R; and other symbols have the same meanings as described above.

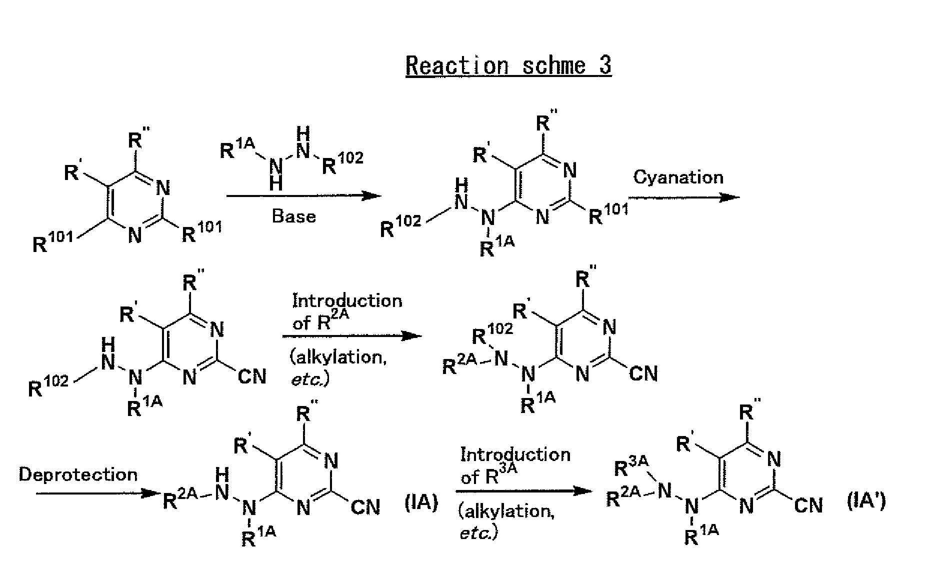

Among the compounds represented by formula (I), a compound in which R³ is alkyl which may have a substituent(s), that is, a compound represented by formula (Ia): 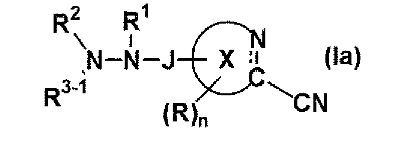 (wherein R³⁻¹ is alkyl which may have a substituent(s), and other symbols have the same meanings as described above), can be prepared by alkylation of a compound represented by formula (IV): 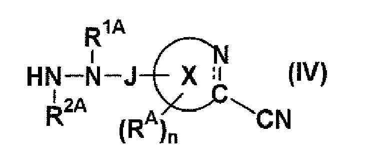 (wherein all symbols have the same meanings as described above) with a compound represented by formula (V):

**R^{3A-1}-Q** **(V)**

(wherein R^{3A-1} has the same meaning as R³⁻¹, and carboxy, hydroxy, amino or mercapto in the group represented by R^{3A-1} is protected if the protection is necessary; and other symbols have the same meanings as described above),
and if necessary, followed by removal of the protecting group.

The alkylation is known and, for example, is carried out by a reaction at -20 to 100°C in an organic solvent (dimethylformamide, dimethylacetamide, diethyl ether, tetrahydrofuran, dimethoxyethane, *etc*.), in the presence of a base (e.g., sodium hydroxide, potassium hydroxide, butyllithium, phenyllithium, lithium hexamethyldisilazide, potassium hexamethyldisilazide, *etc.)* and in the presence or absence of iodide (e.g., , potassium iodide, sodium iodide, *etc.).*

The removal of the protecting group can be carried out in the same manner as described above.

Among the compounds represented by formula (I), a compound represented by formula (Ib): 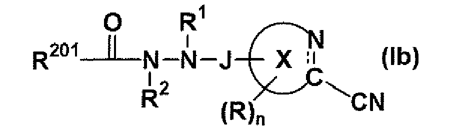 (wherein 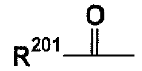 is a group wherein the group adjacent to the nitrogen atom described is carbonyl among R³; and other symbols have the same meanings as described above) can be prepared by hydrazidation of a compound represented by formula (IV) and a compound represented by formula (VI): 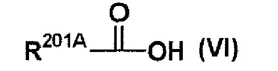 (wherein R^{201A} has the same meaning as R²⁰¹, and carboxy, hydroxy, amino or mercapto in the groups represented by R^{201A} is protected if the protection is necessary),
and if necessary, followed by removal of the protecting group.

The hydrazidation is known and its examples are as follows.
- (1): a method using acid halide;
- (2): a method using mixed acid anhydride;
- (3): a method using a condensing agent; *etc*.

Those methods will be specifically illustrated as follows.
(1) The method using acid halide can be carried out, for example, by reacting carboxylic acid with an acid halidation agent (oxazalyl chloride, thionyl chloride, *etc.)* at -20°C to the refluxing temperature in an organic solvent (chloroform, dichloromethane, diethyl ether, tetrahydrofuran, *etc*.) or without a solvent, and reacting the resulting acid halide with amine at 0 to 40°C in the presence of a base (pyridine, triethylamine, dimethylaniline , dimethylaminopyridine, diisopropylethylamine, *etc.)* in an organic solvent (chloroform, dichloromethane, diethyl ether, tetrahydrofuran, *etc*.). Furthermore, the reaction can also be carried out by reacting the resulting acid halide with amine at 0 to 40°C in an organic solvent (dioxane, tetrahydrofuran, *etc.*) using an aqueous alkaline solution (aqueous sodium bicarbonate solution, aqueous sodium hydroxide solution, *etc.*)*.*
(2) The method using mixed acid anhydride can be carried out, for example, by reacting carboxylic acid with acid halide (pivaloyl chloride, tosyl chloride, mesyl chloride, *etc.)* or an acid derivative (ethyl chloroformate, isobutyl chloroformate, *etc*.) in an organic solvent (chloroform, dichloromethane, diethyl ether, tetrahydrofuran, *etc*.) or without a solvent, in the presence of a base (pyridine, triethylamine, dimethylaniline , dimethylaminopyridine, diisopropylethylamine, *etc.)* at 0 to 40°C, and the resulting mixed acid anhydride with amine at 0 to 40°C in an organic solvent (chloroform, dichloromethane, diethyl ether, tetrahydrofuran, *etc*.).
(3) The method using a condensing agent can be carried out, for example, by reacting carboxylic acid with amine at 0 to 40°C in an organic solvent (chloroform, dichloromethane, dimethylformamide, diethyl ether, tetrahydrofuran, *etc*.) or without a solvent in the presence or absence of a base (pyridine, triethylamine, dimethylaniline, dimethylaminopyridine, N-methylmorpholine, *etc.)* using a condensing agent (1,3-dicyclohexylcarbodiimide (DCC), 1-ethyl-3 -[3 - (dimethylamino)propyl]carbodiimide (EDC), 1,1'-carbonyldiimidazole (CDI), 2-chloro-1-methylpyridinium iodide, 1-propanephosphonic acid cyclic anhydride (PPA), bis(2-oxo-3-oxazolidinyl)phoshinic chloride, *etc.)* and using or without using 1-hydraxybenzotriazole (HOBt) or 1-hydroxy-7-azabenzotriazole (HOAt).

These reactions (1), (2) and (3) are preferably carried out under anhydrous conditions in inert gas (argon, nitrogen, *etc*.) atmosphere.

The removal of the protecting group can be carried out in the same manner as described above.

Among the compounds represented by formula (Ib), a compound represented by formula (Ic): 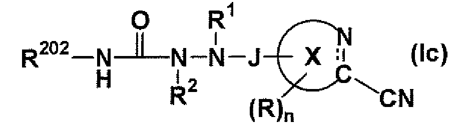 (wherein 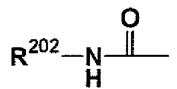 is a group in which the group adjacent to the nitrogen atom described is 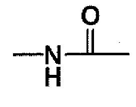 in R³, and other symbols have the same meanings as described above) can be prepared by the following reaction of a compound represented by formula (IV) with a compound represented by formula (Vll):

**R^{202A}-N=C=O** **(VII)**

(wherein R^{202A} has the same meaning as R²⁰², and carboxy, hydroxy, amino or mercapto in the group represented by R^{202A} is protected if the protection is necessary)
and if necessary, followed by removal of the protecting group.

This reaction is known and, for example, is carried out in an organic solvent (toluene, chloroform, tetrahydrofuran, *etc.)* at room temperature to the refluxing temperature.

The removal of the protecting group can be carried out in the same manner as described above.

Also, the compound represented by formula (Ic) can be prepared by the following reaction of a compound represented by formula (IV) with a compound represented by formula (VIII):

**R^{202A}-NH₂** **(VIII)**

(wherein R^{202A} has the same meaning as described above)
and if necessary, followed by removal of the protecting group.

The reaction is known and, for example, is carried out at 0 to 40°C in an organic solvent (tetrahydrofuran, N,N-dimethylformamide, *etc.*), in the presence of triphosgene, using a base (triethylamine, *etc.).* Moreover, for example, the reaction is also carried out at 0 to 80°C in an organic solvent (methylene chloride, N,N-dimethylformamide, *etc.),* in the presence of 1,1'-carbonylbis-1H-imidazole (CDI) using or without using a base (triethylamine, N-methylmorpholine, *etc.).*

The removal of the protecting group can be carried out in the same manner as described above.

All reactions in each reaction scheme is well known. All starting materials and reagents in the reaction schemes are known compounds, or can be prepared easily by known methods.

In each reaction of the present specification, reaction products may be purified by conventional techniques, for example, distillation under atmospheric or reduced pressure, high performance liquid chromatography, thin layer chromatography or column chromatography using silica gel or magnesium silicate, washing or recrystallization, *etc*. Purification may be carried out after each reaction, or after a series of reactions.

### Pharmacological activities of the compounds of the present invention:

The following methods can be used as pharmacological test besides the methods described in "Biological Examples."
(1) Measurement of cathepsin B inhibitory activity
   Cathepsin B inhibitory activity can be measured in the way prescribed hereinafter.
   10 µL of synthesized substrate (carbobenzoxy-L-arginyl-L-arginine-4-methyl-chromanyl-7-amide or carbobenzoxy-L-phenylalanyl-L-arginine-4-methyl-chromanyl-7-amide) solution of several concentrations, 10 µl of cysteine protease inhibitor solution of several concentrations, 70 µl of cathepsin B enzyme reaction buffer (mixture of 400 mmol/L of acetic acid, 4 mmol/L af EDTA, 8 mmol/L of DDT to adjust to pH 5.5) and 10 µl of cathepsin B enzyme solution are mixed and the increase of fluorescence intensity is measured (λex (excitation wavelength)=355 nm, λem (fluorescence wavelength)=460 nm) when reacted at 37°C.
(2) Measurement of cathepsin S inhibitory activity
   Cathepsin S inhibitory activity can be measured in the way prescribed hereinafter.
   10 µl of synthesized substrate (carbobenzoxy-L-leucyl-L-leucyl-L-arguinine-4-methyl-chromanyl-7-amide) solution and 5 µl of cysteine protease inhibitor solution of several concentrations, 75 µl of cathepsin S enzyme reaction buffer (100 mmol/L of sodium phosphate, 2 mmol/L of EDTA, 2 mmol/L of DTT are mixed to adjust to pH 6.5) and 10 µl of cathepsin S enzyme solution are mixed and the increase of fluorescence intensity is measured (λex(excitation wavelength) =355 nm, λem (fluorescence wavelength)=460 nm) when reacted at 37°C.
(3) Measurement of cathepsin L inhibitory activity
   Cathepsin L inhibitory activity can be measured in the way prescribed hereinafter.
   5 µl of Synthesized substrate (carbobenzoxy-L-phenylalanyl-L-arguine-4-methylchromanyl-7-amide or L-prolyl-L-phenylalanyl-L-arginine-4-methylchromanyl-7-amide) solution and 5 µl of cysteine protease inhibitor solution of several concentrations, 80 µl of cathepsin L enzyme reaction buffer (400 mmol/L of acetic acid, 4 mmol/L of EDTA, 8 mmol/L of DTT are mixed to adjust to pH 5.5) and 10 µl of cathepsin L enzyme solution are mixed and the increase of fluorescence intensity is measured (λex (excitation wavelength)=355 nm, λem (fluorescence wavelength)=460 nm) when reacted at 37°C.
(4) Measurement of calpain inhibitory activity
   The activity is measured according to the method described in Calcium-depending protease, Seibutsukagaku-Jikkenhou (Biochemistry Experimental Method) Tanpakubunkaikouso (Protease) I, 57 (1993).
(5) Measurement of caspase-1 inhibitory activity
   Caspase-1 inhibitory activity can be measured in the way prescribed hereinafter.
   50 µl of caspase-1 enzyme reaction solution (20 mmol/L of 4-(2-hydroxyethyl)-1-piperazinethanesulfonate-sodium hydroxide buffer (pH 7.4), 10 mmol/L of potassium chloride, 1.5 mmol/L of magnesium chloride, 0.1 mmol/L EDTA, 10% glycerol) and 50 µl of cysteine protease inhibitor solution of several concentrations, 50 µl of caspase-1 enzyme solution and 100 µl of synthesized substrate (acetyl-L-tyrosinyl-L-valinyl-L-alanyl-L-aspartic acid-4-methylchromanyl-7-amide) solution of several concentrations are reacted at 37°C and the fluorescence intensity is measured (λex (excitation wavelength)=355 nm, λem (fluorescence wavelength)=460 nm).
(6) Investigation in bone resorption inhibitory activity using mouse calvaria cultivation system
   Mouse neonatal calvaria is cultured in D-minimum essential medium containing cysteine protease inhibitor (mixture of Penicillin G potassium (final concentration 100 U/ml), streptomycin sulfate (final concentration 0.1 mg/ml), bovine serum albumin (final concentration 0.1 %), glutamine (final concentration 0.3 mg/ml) in D-minimal essential medium) with incitant (parathyroid hormone (PTH) or arotinoid) at 37°C and the calcium concentration in the culture medium is measured.
(7) Bone resorption pit formation test using rabbit osteoclast cells
   Osteoclast cells collected from rabbit bones are sowed over slices of bovine cortical bone, dentine or teeth of toothed whale and are cultured at 37°C in α-minimal essential medium containing final concentration 5% of fetal bovine serum and various concentrations of cysteine protease inhibitor. The pits form on the slices by the osteoclast cells are observed and at the same time type-I collagen C-terminal telopeptide (CTx) concentration in culture medium can be measured.
(8) Investigation of immune reaction inhibitory effect using antigen-sensitized mouse spleen cells
   Spleen cells are collected from mice sensitized by ovalbumin (OVA) several times. Inhibitory effect of cysteine protease inhibitors against immune response induced by OVA stimulus can be investigated, using cytokine concentration and immunoglobulin concentration in culture solution as indicators.
(9) Investigation in inhibitory effect against bone resorption using the rat PTH hypercalcemia model
   The effect of cysteine protease inhibitor (compulsory oral administration, intraperitoneal administration) on bone resorption which is promoted by intravenous administration of parathyroid hormone (PTH) solution (30 µg/ml) can be investigated in rats, using calcium concentration in blood as an indicator.
(10) Studies on bone resorption inhibitory effect using TPTx rat PTHrP-induced hypercalcemia model
   The effect of cysteine protease inhibitor (compulsory oral administration, intraperitoneal administration) on bone resorption, promoted by subcutaneous administration of parathyroid hormone related peptide (PTHrP) to a fasting rat (thyroparathyroidectomized; TPTx) can be investigated, using calcium concentration in blood as an indicator.
(11) Measurement of cathepsin H inhibitory activity
   Using Arg-MCA (4-methyl-chromanyl-7-amide) as synthetic substrate, according to the method described in FEBS Lett. 280(2), 307-310/1991, Methods Enzymol. 80, 535-561/1981, the activity can be measured.
(12) Measurement of cathepsin C inhibitory activity
   Using Gly-Phe-CHN₂ as synthetic substrate, according to the method described in J. Immnol. 150, 4733-4742,1993, the activity can be measured.

### Toxicity:

The toxicity of the compound of the present invention was very low, so the compound is safe enough for pharmaceutical use.

### Application to pharmaceuticals:

The compound of the present invention has an inhibitory activity against cysteine proteases (cathepsins such as K, L, S, B, F, H, C, V, O, W, Z, *etc.,* caspases such as caspase-1, calpains such as calpain, *etc.),* and therefore it is useful as an agent for the prophylaxis and/or treatment of inflammatory diseases (periodontitis, arthritis, inflammatory bowel diseases, infectious diseases, pancreatitis, hepatitis, glomerulonephritis, endocarditis, myocarditis, ulcerative colitis, *etc*.), diseases induced by apoptosis (graft versus host diseases, rejection in transplantation, acquired immunodeficiency syndrome (AIDS), AIDS-related complex (ARC), adult T cell leukemia, hairy cells leukemia, spondylopathy, disorders of respiratory apparatus, arthritis, HIV or HTLV-1 related diseases (uveitis *etc.),* virus related diseases (hepatitis C *etc.),* cancer, collagenosis (systemic lupus erythematosus, rheumatoid arthritis, *etc*.), ulcerative colitis, Sjoegren syndrome, primary biliary cirrhosis, spontaneous thrombocytopenic purpura, autoimmune hemolytic anemia, myasthenia gravis, autoimmune diseases (insulin dependent (type I) diabetes, *etc*.), diseases accompanied by thrombocytopenia (myelodysplastic syndrome, cyclic thrombocytopenia, aplastic anemia, spontaneous thrombocytopenia, disseminated intravascular coagulation (DIC), *etc.),* viral hepatitis (A, B, C, F, *etc.)* or hepatitis medicamentosa and cirrhosis, *etc*., dementia such as Alzheimer's disease, Alzheimer-type senile dementia, cerebrovascular injury, neurodegenerative disease, adult acute respiratory distress syndrome, infectious diseases, prostatomegaly, hysteromyoma, bronchial asthma, arteriosclerosis, hyperlipidemia, all kinds of lusus naturae, nephritis, senile cataract, chronic fatigue syndrome, myodystrophy, peripheral nerve injury, *etc.),* diseases induced by immune response disorder (graft versus host diseases, rejection in transplantation, allergic diseases (asthmatic bronchitis, atopic dermatitis, allergic rhinitis, hay fever, diseases by house dust, hypersensitive pneumonia, food allergy, *etc.*), psoriasis, rheumatoid arthritis, *etc.),* autoimmune diseases (insulin dependent (type I) diabetes, systemic lupus erythematosus, Hashimoto's diseases, multiple sclerosis, *etc.),* diseases induced by decomposition of proteins which compose a body (myodystrophy, cataract, periodontitis, hepatocyte injury by bile acid (cholestatic cirrhosis *etc.), etc.,* decomposition of alveolus elastica (emphysema *etc.*)*,* ischemic diseases (brain ischemia, brain disorder by ischemic reperfusion, cardiac infarction, ischemic liver damage, *etc*.), *etc.*)*,* shock (septic shock, systemic inflammatory responsive syndrome, endotoxin shock, acidosis, *etc.*)*,* circulatory disorder (arteriosclerosis, restenosis after PTCA (percutaneous transluminal coronary angioplasty), *etc*.), disorder of blood coagulation system (thrombocytopenic purpura, hemolytic uremic syndrome, *etc.),* malignant tumor, acquired immune deficiency syndrome (AIDS, AIDS-related complex (ARC), *etc.),* parasitic diseases (malaria *etc*.), neurodegenerative disease (Alzheimer-type senile dementia, Huntington's chorea, Parkinson's disease, multiple sclerosis, traumatic encephalopathy, traumatic spondylopathy, *etc*.), lung disorder (fibroid lungs, *etc.),* bone diseases (osteoporosis, bone fracture, rheumatoid arthritis, arthritis, osteoarthritis, hypercalcaemia, osteometastasis of cancer, periodontitis, bone Paget's disease, *etc*.), endacrinesthenia (hyperthyroidism *etc.), etc.* As the compound of formula (I) has an inhibitory activity against cystein protease, it is also useful as a bone resorption inhibitor.

Cysteine protease which the compound of the present invention inhibits is all preferable, for example, cathepsin K, cathepsin L, cathepsin S, cathepsin B, cathepsin H, cathepsin F, cathepsin Y, cathepsin C, calpain, caspase-1, *etc.* Among them, cathepsin K is most preferred. Of course, cysteine proteases other than them are included in the scope of the present invention and naturally so are those cysteine proteases to be discovered in the future.

The compound of the present invention may also be administered as a concomitant agent in combination with other agents for
1) supplement and/or reinforcement of preventive and/or treating effect(s) of the compound,
2) improvement in kinetics and absorption of the compound of and reduction of dose and/or
3) reduction of side effect of the compound.

A concomitant agent of the compound of the present invention with other agents may be administered in a mode of compounded agent in which both components are compounded in a single preparation or in a mode of separate preparations. When administration is conducted using separate preparations, a simultaneous administration and administrations with time difference is included. In the case of administrations with time difference, the compound of the present invention may be firstly administered and then other drug may be administered, or the other drug may be firstly administered and then the compound of the present invention may be administered. Each of the methods for the administration may be the same or different.

There is no particular limitation for the diseases for which the above-mentioned concomitant agent achieves the preventive and/or the treating effect but any disease will be acceptable so far as it supplements and/or enforces the preventive and/or treating effect of the compound of the present invention.

For example, examples of the other drug for supplement and/or reinforcing the preventive and/or treating effect of the compound of the present invention to bone diseases include, for example, bisphosphonate formulations, Vitamin D and its derivatives, Vitamin K and its derivatives, calcitonin formulations, α-calcitonin gene-related peptide formulations, female hormone formulations, selective estrogen receptor modulators (SERM), ipriflavone formulations, calcium formulations, anabolic steroid formulations, parathyroid hormone (PTH) formulations, PTHrP derivatives, caspase-1 inhibitors, farnesoid X receptor agonists, Bone Morphogenetic Protein (BMP) formulations, anti-RANKL (receptor activator of NF-kappa B ligand) antibody, GSK inhibitors (GSK:glycogen synthase kinase), metalloprotease inhibitors, prostaglandin derivatives, strontium formulations, anti TNF-α antibody, anti-IL-6 antibody, HMG-CoA reductase inhibitors, steroidal drugs, and antiinflammatory drugs, *etc.* Nonsteroidal antiinflammatory agent, local anesthetic agent and/or muscle-relaxing drug can also be used together for the purpose of redress of pain accompanied by bone fracture and osteoporosis.

Bisphosphonate formulations include, for example, minodronic acid (1-hydroxy-2-(imidazo[1,2-a]pyridin-3-yl)ethylidenebisphosphonic acid, salt thereof, or hydrate thereof), alendronate (4-amino-1-hydroxybutylidene-1,1-bisphosphonic acid (alendronic acid), or sodium salt thereof, or trihydrate thereof), incadronate (cycloheptylaminomethylene-1,1-diphosphoric acid (incadronic acid), or disodium salt thereof, hydrate thereof), clodronate (1,1-dichloromethylene-1,1-diphosphoric acid (clodronic acid), or disodium salt thereof), tiludronate ((4-chlorophenyl)thiomethylene-1,1-diphosphoric acid (tiludronic acid), or disodium salt thereof), etidronate (1-hydroxy-1,1-diphosphoric acid (etidronic acid), or disodium salt thereof,), ibandronate (1-hydroxy-3-(N-methyl-N-pentylamino)propylidene-1,1-bisphosphonic acid), risedronate (1-hydroxy-2-pyridin-3-ylethylidenediphosphoric acid (risedronic acid), or sodium salt thereof, sester hydrate thereof), piridronate ([2-(2-pyridinyl)ethylidene]-1,1-bisphosphonic acid), pamidronate (3-amino-1-hydroxypropylidene-1,1-bisphosphonic acid (pamidronic acid), or disodium salt thereof, or pentahydrate thereof), zoledronate (1-hydroxy-2-(1H-imidazol-1-yl)ethylidene-1,1-bisphosphonic acid, or hydrate thereof), olpadronate (3-(dimethylamino)-1-hydroxypropylidene-1,1-bisphosphonic acid), neridronate (6-amino-1-hydroxyhexylidene-1,1-bisphosphonic acid), *etc.*

Vitamin D include, for example, Vitamin D₂ (ergocalciferol), Vitamin D₃ (cholecalciferol), *etc*.

Vitamin D derivatives include, for example, alfacalcidol, falecalcitriol, calcitriol, 1α,25-dihydroxycholecalciferol, dihydrotaxisterol, ST-630, KDR, ST-630, ED-71, rocaltrol (Ro44-7190), tacalcitol, maxacalcitol, *etc.*

Vitamin K and its derivatives include, for example, vitamin K₁ (phytonadione), vitamin K₂ (menatetrenone), etc.

Calcitonin formulations include, for example, calcitonin salmon (STH-32, SMC20-51), calcitonin chicken (MCI-536), secalciferol, elcatonin, TJN-135, *etc.*

Female hormone formulations include, for example, estrogen preparations, progesterone preparations, *etc.* Estrogen preparations include, for example, estrogen, estradiol, estradiol benzoate, estradiol cypionate, estradiol dipropionate, estradiol enannthate, estradiol hexahydrobenzoate, estradiol phenylpropionate, estradiol undecanoate, estradiol valerate, estrone, ethinylestradiol, mestranol, estriol, chlormadinone acetate, norethisterone. Progesterone preparations include, for example, progesterone, hydroxyprogesterone caproate, medroxyprogesterone acetate, trimegestone, *etc*.

Selective estrogen receptor modulators (SERM) include, for example, tamoxifen, lasofoxifene tartrate, raloxifene hydrochloride, bazedoxifene acetate, PSK-3471, *etc.*

Ipriflavone formulations include, for example, ipriflavone, *etc.*

Calcium formulations include, for example, calcium chloride, calcium gluconate, calcium glycerophosphate, calcium lactate, calcium L-aspartate, calcium hydrogen phosphate, *etc*.

Anabolic steroid formulations include, for example, nandrolone decanoate, nandrolone phenylpropionate, nandrolone cyclohexylpropionate, metenolone enanthate, mestanolone, stanozolol, oxymetholone, *etc*.

Parathyroid hormone (PTH) formulations include, for example, PTH, teriparatide acetate, MBRI-93.02, Ostabolin-C, *etc.*

PTHrP derivatives include, for example, RS-66271, hPTHrP, *etc*.

Caspase-1 inhibitors include, for example, pralnacasan, nitroflubiprofen, *etc.*

Farnesoid X receptor agonists include, for example, SR-45023A, *etc.*

Anti-RANKL antibody includes, for example, AMG162, *etc.*

Metalloprotease inhibitors include, for example, minocycline hydrochloride, *etc.*

Prostaglandin derivatives include, for example, EP2 agonist, EP4 agonist, EP4 antagonist, for example, ONO-4819, nitroflurbiprofen, *etc.*

Strontium formulations include, for example, strontium ranelate, *etc*.

Anti TNF-α antibody include, for example, infliximab, etanercept, *etc.*

Anti-IL-6 antibody include, for example, MRA, *etc.*

HMG-CoA reductase inhibitors include, for example, pravastatin, simvastatin, lovastatin, fluvastatin, atorvastatin, pitavastatin, rosuvastatin, *etc*.

Steroidal drugs include, for example, KB-889 (OD14, tibolone), Hipros (TZP-4238), *etc.*

Antiinflammatory drugs include, for example, sasapyrine, sodium salicylate, aspirin, aspirin dialuminate formulation, diflunisal, indomethacin, suprofen, ufenamate, dimethylisopropyl azulen, bufexamac, felbinac, diclofenac, tolmetin sodium, Clinoril, fenbufen, napmetone, proglumetacin, indomethacin farnesil, acemetacin, proglumetacin maleate, amfenac sodium, mofezolac, etodolac, ibuprofen, ibuprofen piconol, naproxen, flurbiprofen, flurbiprofen axethyl, ketoprofen, fenoprofen calcium, tiaprofenen, oxaprozin, pranoprofen, loxoprofen sodium, aluminoprofen, zaltoprofen, mefenamic acid, aluminum mefenamate, tolfenamic acid, floctafenine, ketophenylbutazone, oxyfenbutazone, piroxicam, tenoxicam, anpiroxicam, napageln cream, epirizole, tiaramide hydrochloride, tinoridine hydrochloride, emorfazone, sulpyrine, Migrenin, Saridon, Sedes G, Amipylo N, Sorbon, pyrine system antipyretics, acetaminophen, phenacetin, dimethothiazine mesylate, simetride formulation, or antipyrine system antipyretics, *etc*.

There is no limitation for the ratio by weight of the compound of the present invention to other agents.

With regard to other agents, two or more agents may be administered in combination.

Such other agents which supplement and/or reinforce the preventive and/or treating effect of the compound of the present invention include not only those which have been found on the basis of the above-mentioned mechanism but also those which will be found in future.

The pharmaceutical composition comprising the compound of the present invention, a combination of the compound of the present invention and other drug as an active ingredient is generally administered systemically or topically and orally or parenterally when it is used for the above objects.

The dosages are determined depending on age, body weight, symptom, therapeutic effect, administration route, duration of the treatment and the like. Generally, 1 mg to 1000 mg per adult is orally administered once to several times per day, or 0.1 mg to 100 mg per adult is parenterally administered (preferably by intravenous administration) once to several times per day, or continuously administered from vein for 1 to 24 hours per day.

Since the dose changes depending on various conditions as described above, there are cases in which doses lower than or greater than the above ranges may be used.

The pharmaceutical composition comprising the compound of the present invention, a concomitant agent of the compound of the present invention and other drug as an active ingredient may be administered in the form of solid compositions, liquid compositions and other compositions for oral administration, and injections, external preparations, suppositories, and the like for parenteral administration.

Solid compositions for oral administration include tablets, pills, capsules, dispersible powders, granules and the like. Capsules include hard capsules and soft capsules.

In such solid compositions, one or more of the active compound(s) may be admixed with vehicles (such as lactose, mannitol, glucose, microcrystalline cellulose or starch), binders (such as hydroxypropyl cellulose, polyvinylpyrrolidone or magnesium metasilicate aluminate), disintegrants (such as cellulose calcium glycolate), lubricants (such as magnesium stearate), stabilizing agents, and solution adjuvants (such as glutamic acid or aspartic acid) and prepared according to methods well known in normal pharmaceutical practice. The solid forms may, if desired, be coated with coating agents (such as sugar, gelatin, hydroxypropyl cellulose or hydroxypropylmethyl cellulose phthalate), or be coated with two or more films. And further, coating may include containment within capsules of absorbable materials such as gelatin.

Liquid compositions for oral administration include pharmaceutically acceptable solutions, suspensions, emulsions, syrups and elixirs. In such forms, one or more of the active compound(s) may be dissolved, suspended or emulized into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

Injections for parenteral administration in the present invention include solutions, suspensions and emulsions, and also solid injections which are to be dissolved or suspended in solvents upon use. Such an injection is prepared by dissolving, suspending or emulsifying one or more active substances in a solvent and then put to use. Examples of the solvent include distilled water for injection, physiological saline, plant oil, alcohols such as propylene glycol, polyethylene glycol and ethanol, and combinations thereof Further, the injection may contain a stabilizer, a solubilizing auxiliary agent such as glutamic acid, aspartic acid and POLYSORBATE 80 (registered trade mark) *etc.),* suspending agent, emulsifying agent, soothing agent, buffering agent, preservative agent and the like. The injection may be sterilized in the final step of the preparation process or the whole preparation process may be operated under sterile conditions. Alternatively, the sterile product, for example a sterile freeze-dried product may be prepared, and upon use, the product may be dissolved in sterilized or aseptic distilled water for injection or other sterilized or aseptic solvents.

Other compositions for parenteral administration include liquids for external use, ointments, endemic liniments, inhalants, spray compositions, suppositories for intrarectal administration, and pessaries for intravaginal administration and the like containing one or more active compound(s) which can be prepared by known methods.

Spray compositions may contain stabilizing agents such as sodium hydrogen sulfate, buffering agents to give isotonicity, isotonic solutions such as sodium chloride, sodium citrate or citric acid, in addition to inert diluents. For preparation of such spray compositions, for example, the method described in the United States Patent Nos 2,868,691 and 3.095.355.

### Effect of the invention:

The compounds of the present invention have the inhibitory activity against cathepsin K, so they are useful for preventing and/or treating cathepsin K-related diseases.

## Claims

1. A compound represented by formula (I): 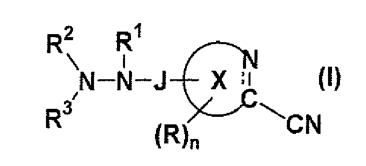 wherein ring X is a heterocyclic group which may contain 1 to 4 hetero atoms selected from a nitrogen atom(s), an oxygen atom(s) and a sulfur atom(s) which may be oxidized, in addition to the nitrogen atom in the ring; 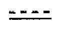 is a single bond or a double bond; J is a bond or a spacer having from 1 to 8 atoms of the principle chain; R is hydrogen atom or a substituent, and if R is plural, Rs are the same or different, and two Rs may form, together with the atom on the ring to which they bind, a cyclic group which may have a substituent(s); R¹, R² and R³ is each independently a hydrogen atom or a substituent; n is 0 or an integer of 1 to 10, and wherein R¹ and R² may form, together with the nitrogen atom to which they bind, a cyclic group which may have a substituent(s), and R² and R³ may form, together with the nitrogen atom to which they bind, a cyclic group which may have a substituent(s),
a salt thereof, a solvate thereof, or an N-oxide thereof, or a prodrug thereof.

2. The compound according to claim 1, wherein the ring X is a 5- to 7-membered monocyclic heterocyclic group which may contain 1 to 3 hetero atoms selected from a nitrogen atom(s), an oxygen atom(s) and a sulfur atom(s) which may be oxidized, in addition to the nitrogen atom in the ring.

3. The compound according to claim 1, wherein the ring X is 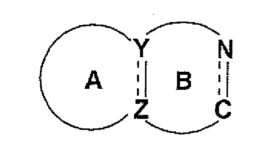 wherein ring A is a carbocyclic group or a heterocyclic group; ring B is a nitrogen-containing heterocyclic group; Y and Z is each independently a carbon atom or a nitrogen atom; and other symbols have the same meanings as described in claim 1.

4. The compound according to claim 2, wherein the ring X is a ring selected from tetrahydropyrimidine, dihyrdopyridazine, pyridazine, dihydropyrimidine, dihydropyrazine, dihydrotriazine, dihydropyrazole, pyrrole, pyrimidine, pyrazine, imidazole, pyrazole, triazole, tetrazole, thiazole, oxazole, thiadiazole, oxadiazole, azepane, azepine, dihydroazepine, tetrahydroazepine, diazepane, diazepine, dihydrodiazepine and tetrahydrodiazepine.

5. The compound according to claim 1, wherein J is a bond.

6. The compound according to claim 1, wherein R¹ is C1-8 alkyl which may have a substituent(s).

7. The compound according to claim 1, wherein R² is C1-8 alkyl which may have a substituent(s), C1-8 alkylcarbonyl which may have a substituent(s), C1-8 alkylsulfonyl which may have a substituent(s), carbamoyl which may have a substituent(s), a carbocyclic group which may have a substituent(s), a heterocyclic group which may have a substituent(s), 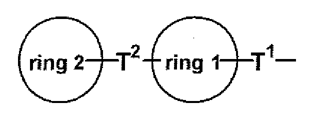 wherein T¹ and T² is each independently a bond or a spacer having from 1 to 3 atoms of the principle chain, and ring 1 and ring 2 is each independently a cyclic group which may have a substituent(s), or 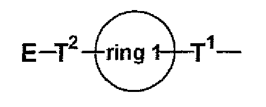 wherein E is a substituent containing a nitrogen atom having basicity, and other symbols have the same meanings as above, and
R³ is hydrogen atom.

8. The compound according to claim 1, which is represented by formula (I-4): 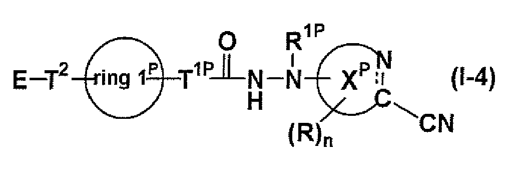 wherein ring X^{P} is a 5- to 7-membered monocyclic heterocyclic group which may contain 1 to 3 hetero atoms selected from a nitrogen atom(s), an oxygen atom(s) and a sulfur atom(s) which may be oxidized, in addition to the nitrogen atom in the ring; R^{1P} is C1-8 alkyl which may have a substituent(s); T^{1P} is a bond or a spacer having 1 or 2 atoms of the principle chain; ring1^{P} is a C5 to 7 membered monocyclic carbocyclic group which may have a substituent(s) or a 5- to 7-membered monocyclic heterocyclic group which may have a substituent(s); and other symbols have the same meanings as described in claim 1 or 7.

9. The compound according to claim 1, which is
N'-[2-cyano-6-(trifluoromethyl)-4-pyrimidinyl]-4-[(4-methyl-1-piperazinyl)methyl]-N'-neopentylbenzohydrazide,
N'-(2-cyano-5-methyl-4-pyrimidinyl)-4-[(4-methyl-1-piperazinyl)methyl]-N'-neopentylbenzohydrazide,
N'-[2-cyano-6-(trifluoromethyl)-4-pyrimidinyl]-2-(dimethylamino)-N'-neopentylacetohydrazide,
N'-(5-bromo-2-cyano-4-pyrimidinyl)-4-[1-(2-methoxyethyl)-4-piperidinyl]-N'-neopentylbenzahydrazide,
N'-[2-cyano-6-(trifluoromethyl)-4-pyrimidnyl]-4-[(dimethylamino)methyl]-N'-neopentylbenzohydrazide,
N'-[2-cyano-6-(trifluoromethyl)-4-pyrimidinyl]-N'-neopentylnicotinohydrazide,
N'-[2-cyano-6-(trifluoromethyl)-4-pyrimidinyl]-4-(4-morpholinylmethyl)-N'-neopentylbenzohydrazide,
N'-[2-cyano-6-(trifluoromethyl)-4-pyrimidinyl]-4-[(dimethylamino)methyl]-3 -fluoro-N'-neopentylbenzohydrazide,
N'-(2-cyanopyrazolo[1,5-a][1,3,5]triazin-4-yl)-4-[1-(2-methoxyethyl)-4-piperidinyl]-N'-neopentylbenzohydrazide,
N'-[2-cyano-6-(trifluoromethyl)-4-pyrimidinyl]-2-methyl-2-[4-(1-methyl-4-piperidinyl)phenyl]-N'-neopentylpropanohydrazide,
N'-(2-cyano-6-methyl-4-pyrimidinyl)-4-[(dimethylamino)methyl]-N'-neopentylbenzohydrazide,
N'-[2-cyano-6-(trifluoromethyl)-4-pyrimidinyl]-2-(4-[(dimethylamino)methyl]phenyl)-2-methyl-N'-neopentylpropanohydrazide,
N'-(2-cyano-6-methyl-4-pyrimidinyl)-4-[2-(dimethylamino)ethoxy]-N'-neopentylbenzohydrazide,
N'-(2-cyano-6-methyl-4-pyrimidinyl)-2-{4-[(dimethylamino)methyl]phenyl}-2-methyl-N'-neopentylpropanohydrazide,
N'-[2-cyano-6-(trifluoromethyl)-4-pyrimidinyl]-N'-neopentyl-2-(1-pyrrolidinyl)acetohydrazide,
N'-[2-cyano-6-(trifluoromethyl)-4-pyrimidinyl]-N'-(2,2-dimethylpropyl)-6-(4-methyl-1-piperazinyl)nicotinohydrazide,
N'-[2-cyano-6-(trifluoromethyl)-4-pyrimidinyl]-N'-(2,2-dimethylpropyl)-4-{[(2-methoxyethyl)(methyl)amino]methyl}benzohydrazide,
N'-(2-cyano-6,7-dihydro-5H-cyclopenta[d]pyrimidin-4-yl)-4-[(dimethylamino)methyl]-N'-(2,2-dimethylpropyl)benzohydrazide,
N'-[2-cyano-6-(trifluoromethyl)-4-pyrimidinyl]-3-(dimethylamino)-N'-(2,2-dimethylpropyl)propanohydrazide,
N'-(2-cyano-6,7-dihydro-5H-cyclopenta[d]pyrimidin-4-yl)-2-{4-[(dimethylamino)methyl]phenyl}-N'-(2,2-dimethylpropyl)-2-methylpropanohydrazide,
N'-(2-cyano-6-methyl-4-pyrimidinyl)-N'-(2,2-dimethylpropyl)-2-methyl-2-{4-[(4-methyl-1-pyrimidinyl)methyl]phenyl}propanohydrazide,
N'-[2-cyano-6-(trifluoromethyl)-4-pyrimidinyl]-4'-[(dimethylamino)methyl]-N'-(2,2-dimethylpropyl)-4-biphenylcarbohydrazide,
N'-(2-cyano-6-methyl-4-pyrimidinyl)-4-[(dimethylamino)methyl]-N'-(2,2-dimethylpropyl)-3-methoxybenzohydrazide,
N'-[2-cyano-6-(trifluoromethyl)-4-pyrimidinyl]-N'-(2,2-dimethylpropyl)-2-(methylamino)acetohydrazide, or
*tert*-butyl 2-(2-cyano-5,7-dihydrofuro[3,4-d]pyrimidin-4-yl)-2-(2,2-dimethylpropyl)hydrazinecarboxylate.

10. A pharmaceutical composition comprising the compound of formula (I) according to claim 1, a salt thereof, a solvate thereof, or an N-oxide thereof, or a prodrug thereof as an active ingredient.

11. The pharmaceutical composition according to claim 10, which is an agent for prevention and/or treatment for a cysteine protease-related disease.

12. The pharmaceutical composition according to claim 11, wherein a cysteine protease-related disease is a cathepsin K-related disease.

13. The pharmaceutical composition according to claim 12, wherein a cathepsin K-related disease is a bone disease.

14. The pharmaceutical composition according to claim 13, wherein a bone disease is at least one selected from osteoporosis, bone fracture, arthritis, rheumatoid arthritis, osteoarthritis, hypercalcaemia, osteometastasis of cancer, osteosarcoma, periodontitis, and bone Paget's disease.

15. The pharmaceutical composition according to claim 10, which is a bone resorption inhibitor.

16. A drug comprising the compound of formula (I) according to claim 1, a salt thereof, a solvate thereof, or an N-oxide thereof, or a prodrug thereof, combined with at least one selected from bisphosphonate formulations, vitamin D and its derivatives, vitamin K and its derivatives, calcitonin formulations, α-calcitonin gene-related peptide formulations, female hormone formulations, selective estrogen receptor modulators, ipriflavone formulations, calcium formulations, anabolic steroid formulations, parathyroid hormone formulations, PTHrP derivatives, caspase-1 inhibitors, farnesoid X receptor agonists, Bone Morphogenetic Protein formulations, anti-RANKL antibody, GSK inhibitor, metalloprotease inhibitors, prostaglandin derivatives, strontium formulations, anti TNF-α antibody, anti-IL-6 antibody, HMG-CoA reductase inhibitors, steroidal drugs, and antiinflammatory drugs.

17. A method for the prophylaxis and/or treatment of a cysteine protease-related disease in a mammal **characterized by** administering to a mammal an effective amount of the compound of formula (I) according to claim 1, a salt thereof, a solvate thereof, or an N-oxide thereof, or a prodrug thereof.

18. Use of the compound of formula (I) according to claim 1, a salt thereof, a solvate thereof, or an N-oxide thereof, or a prodrug thereof, for the manufacture of a pharmaceutical preparation for the prophylaxis and/or treatment of a cysteine protease-related disease.
